(19) Europäisches Patentamt
European Patent Office
Office européen des brevets

(11) EP 4 149 478 B1

(12) EUROPEAN PATENT SPECIFICATION

(45) Date of publication and mention of the grant of the patent:
**01.07.2026 Bulletin 2026/27**

(21) Application number: **21734482.9**

(22) Date of filing: **13.05.2021**

(51) International Patent Classification (IPC):
***A61K 31/549*** (2006.01) ***A61P 25/24*** (2006.01)

(52) Cooperative Patent Classification (CPC):
**A61K 31/549; A61P 25/24**

(86) International application number:
**PCT/IB2021/000313**

(87) International publication number:
**WO 2021/229294 (18.11.2021 Gazette 2021/46)**

(54) **TREATMENT FOR DEPRESSION WITH A POSITIVE ALLOSTERIC MODULATOR OF AMPA**

BEHANDLUNG VON DEPRESSION MIT EINEM POSITIVEN ALLOSTERISCHEN AMPA-MODULATOR

TRAITEMENT DE LA DÉPRESSION PAR UN MODULATEUR ALLOSTÉRIQUE POSITIF DE L' AMPA

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(30) Priority: **15.05.2020 US 202063025405 P**

(43) Date of publication of application:
**22.03.2023 Bulletin 2023/12**

(73) Proprietor: **Takeda Pharmaceutical Company Limited**
**Osaka-shi, Osaka 541-0045 (JP)**

(72) Inventors:
- **PATHI JAGANNATHAM, Naga Venkatesha, Murthy**
  **Cambridge, MA 02139 (US)**
- **ASGHARNEJAD, Mahnaz**
  **Cambridge, MA 02139 (US)**
- **XU, Lin**
  **Cambridge, MA 02139 (US)**

(74) Representative: **Mewburn Ellis LLP**
**Aurora Building**
**Counterslip**
**Bristol BS1 6BX (GB)**

(56) References cited:
**WO-A1-2012/020848**

- **NISENBAUM ERIC S. ET AL: "Positive Allosteric Modulation of AMPA Receptors: A Novel Potential Antidepressant Therapy", GLUTAMATE-BASED THERAPIES FOR PSYCHIATRIC DISORDERS, 1 January 2010 (2010-01-01), Basel, pages 39 - 56, XP055837283, ISBN: 978-3-0346-0241-9, Retrieved from the Internet <URL:http://dx.doi.org/10.1007/978-3-0346-0241-9_3> DOI: 10.1007/978-3-0346-0241-9_3**
- **FARLEY S ET AL: "Antidepressant-like effects of an AMPA receptor potentiator under a chronic mild stress paradigm", INTERNATIONAL JOURNAL OF NEUROPSYCHOPHARMACOLOGY, CAMBRIDGE UNIV. PRESS, CAMBRIDGE, vol. 13, no. 9, 1 October 2010 (2010-10-01), pages 1207 - 1218, XP002753248, ISSN: 1461-1457, [retrieved on 20100111], DOI: 10.1017/S1461145709991076**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- ASGHARNEJAD MAHNAZ ET AL: "Pharmacokinetic and Pharmacodynamic Properties of the Investigational AMPA Receptor Positive Allosteric Modulator TAK-653 After Single and Multiple Rising Doses in Healthy Volunteers", NEUROPSYCHOPHARMACOLOGY, vol. 43, 1 December 2018 (2018-12-01), pages S157, XP055836901
- KUNUGI AKIYOSHI ET AL: "TAK-137, an AMPA-R potentiator with little agonistic effect, has a wide therapeutic window", NEUROPSYCHOPHARMACOLOGY, ELSEVIER SCIENCE PUBLISHING, NEW YORK, NY, US, vol. 44, no. 5, 12 September 2018 (2018-09-12), pages 961 - 970, XP036724697, ISSN: 0893-133X, [retrieved on 20180912], DOI: 10.1038/S41386-018-0213-7

## Description

### Technical Field

The present invention relates to 9-[4-(cyclohexyloxy)phenyl]-7-methyl-3,4-dihydropyrazino[2,1-c][1,2,4]thiadiazine 2,2-dioxide ("COMPOUND A"), or a pharmaceutically acceptable salt thereof, for use in a method of treating depression.

### Background

Major depressive disorder ("MDD") is characterized by a pervasive depressed mood or loss of interest or pleasure (anhedonia) in almost all activities in discrete episodes of at least 2 weeks duration involving clear-cut changes in affect, cognition, and neurovegetative functions and interepisode remissions or decreases of symptom severity. MDD has been identified as the second leading cause of years lived with disability, designating it as a major public health priority. Currently available antidepressant drugs have limitations, including a low treatment response rate (*e.g.*, treatment-resistant depression ("TRD")) and a time lag of several weeks before a therapeutic effect is observed. Thus, there is a significant unmet medical need for novel antidepressant treatments with faster onset of action and improved efficacy.

### Summary

9-[4-(Cyclohexyloxy)phenyl]-7-methyl-3,4-dihydropyrazino[2,1-c][1,2,4]thiadiazine 2,2-dioxide ("COMPOUND A" or "CMPD A") or a salt thereof is a potent and selective positive allosteric modulator (PAM) of alpha-amino-3-hydroxy-5-methyl-4-isoxazole-propionic acid (AMPA) receptors. This application discloses COMPOUND A, or a pharmaceutically acceptable salt thereof, for use in methods for treating depression and methods for treating and/or preventing a relapse in depression in a patient, comprising: administering to the patient a first dose comprising 9-[4-(cyclohexyloxy)phenyl]-7-methyl-3,4-dihydropyrazino[2,1-c][1,2,4]thiadiazine 2,2-dioxide ("COMPOUND A" or "CMPD A") or a salt thereof; and administering to the patient at least one daily maintenance dose comprising COMPOUND A or a salt thereof.

In some embodiments, COMPOUND A or a salt thereof is COMPOUND A.

In some embodiments, the first dose comprises COMPOUND A. In some embodiments, the first dose comprises a salt of COMPOUND A. In some embodiments, the first dose comprises a pharmaceutically acceptable salt of COMPOUND A.

In some embodiments, the at least one daily maintenance dose comprises COMPOUND A. In some embodiments, the at least one daily maintenance dose comprises a salt of COMPOUND A. In some embodiments, the at least one daily maintenance dose comprises a pharmaceutically acceptable salt of COMPOUND A.

In one aspect, is a compound which is 9-[4-(cyclohexyloxy)phenyl]-7-methyl-3,4-dihydropyrazino[2,1-c][1,2,4]thiadiazine 2,2-dioxide ("COMPOUND A"), or a pharmaceutically acceptable salt thereof, for use in a method for treating depression in a patient, comprising: administering to the patient a first dose comprising 9-[4-(cyclohexyloxy)phenyl]-7-methyl-3,4-dihydropyrazino[2,1-c][1,2,4]thiadiazine 2,2-dioxide ("COMPOUND A" or "CMPD A") or a pharmaceutically acceptable salt thereof; and administering to the patient at least one daily maintenance dose comprising COMPOUND A or a pharmaceutically acceptable salt thereof, wherein the maintenance dose is less than the first dose.

In some embodiments, the depression is disruptive mood dysregulation disorder, major depressive disorder, persistent depressive disorder dysthymia, premenstrual dysphoric disorder, substance/medication-induced depressive disorder, depressive disorder due to another medical condition, other specified depressive disorder, unspecified depressive disorder, moderate depression, severe depression, and/or specifiers for depressive disorders. In some embodiments, the depression is major depressive disorder. In some embodiments, the depression is treatment-resistant depression.

In some embodiments, the method further treats cognitive impairment associated with the depression. In some embodiments, the method further treats cognitive impairment associated with the depression, as measured by the Brief Assessment of Cognition. In some embodiments, the method further treats cognitive impairment involving decline in speed of processing, executive function, attention, or verbal learning and memory associated with the depression. In some embodiments, the method further treats cognitive impairment associated with the depression involving decline in speed of processing. In some embodiments, the method further treats cognitive impairment associated with the depression involving decline in executive function. In some embodiments, the method further treats cognitive impairment associated with the depression involving decline in attention. In some embodiments, the method further treats cognitive impairment associated with the depression involving decline in verbal learning. In some embodiments, the method further treats cognitive impairment associated with the depression involving decline in memory.

In some embodiments, the patient has already received at least one prior therapy for depression. In some embodiments, the patient has failed to respond to at least one prior therapy for depression. In some embodiments, the

patient is a responder to at least one prior therapy for depression.

In some embodiments, the prior therapy for depression comprises an administration of: ketamine or a salt thereof; (S)-ketamine (esketamine) or a salt thereof; (R)-ketamine or a salt thereof; norketamine or a salt thereof; 2 diastereomeric hydroxyketamine or a salt thereof; 6 diastereomeric hydroxynorketamine (HNK) or a salt thereof; (2S,6S)-HNK or a salt thereof; (2R,6R)-HNK or a salt thereof; dehydronorketamine or a salt thereof; methoxetamine or a salt thereof; or a combination thereof. In some embodiments, the prior therapy comprises an administration of ketamine or a salt thereof; (S)-ketamine (esketamine) or a salt thereof; methoxetamine or a salt thereof; or a combination thereof.

In some embodiments, the maintenance dose is administered not less than about 12 hours after the first dose.

In some embodiments, the first dose comprises between about 5 mg and about 9 mg of COMPOUND A.

In some embodiments, the maintenance dose comprises between about 0.5 mg and about 4 mg once per day of COMPOUND A.

In some embodiments, the first dose is effective to achieve a mean $C_{max}$ in the patient of at least about 6 ng/mL. In some embodiments, the first dose is effective to achieve a mean $C_{max}$ in the patient of not more than 100 ng/mL. In some embodiments, the first dose is effective to achieve a mean $C_{max}$ in the patient of not more than about 100 ng/mL. In some embodiments, the first dose is effective to achieve a mean $C_{max}$ in the patient of about 6 ng/mL to about 100 ng/mL.

In some embodiments, the at least one daily maintenance dose is effective to achieve a mean $C_{max}$ at steady-state in the patient of no greater than about 130 ng/mL.

In some embodiments, the first dose is effective to achieve a mean $C_{max}$ in the patient of 6 ng/mL to about 100 ng/mL. In some embodiments, the first dose is effective to achieve a mean $C_{max}$ in the patient of about 75% to about 130% of 42 ng/mL. In some embodiments, the at least one daily maintenance dose is effective to achieve a mean $C_{max}$ at steady-state in the patient of about 75% to about 130% of 78 ng/mL.

In some embodiments, the mean $AUC_{\tau}$ at steady-state in the patient is no greater than about 2600 ng·h/mL. In some embodiments, the mean $AUC_{\tau}$ at steady-state in the patient is about 700 ng·h/mL to about 2600 ng·h/mL. In some embodiments, the mean $AUC_{\tau}$ at steady-state in the patient is about 75% to about 130% of 1487 ng·h/mL. In some embodiments, the mean $AUC_{\infty}$ in the patient is about 75% to about 130% of 2328 ng·h/mL.

In some embodiments, the first dose is orally administered. In some embodiments, the at least one daily maintenance dose is orally administered.

In some embodiments, the first dose comprises about 6 mg of COMPOUND A.

In some embodiments, the maintenance dose comprises about 3 mg once per day of COMPOUND A.

In some embodiments, the first dose is effective to achieve a mean $C_{max}$ in the patient of about 40 to about 100 ng/mL; and the at least one daily maintenance dose is effective to achieve a mean $C_{max}$ at steady-state in the patient of no greater than 130 ng/mL, and a mean $AUC_{\tau}$ at steady-state in the patient of no greater than 2600 ng·h/mL.

In some embodiments, the first dose is effective to achieve a mean $C_{max}$ in the patient of about 75% to about 130% of about 42 ng/mL; and the at least one daily maintenance dose is effective to achieve a mean $C_{max}$ at steady-state in the patient of about 75% to about 130% of 78 ng/mL, and a mean $AUC_{\tau}$ at steady-state in the patient of about 75% to about 130% of 1487 ng·h/mL, after the at least one daily maintenance dose. In some embodiments, the first dose is effective to achieve a mean $C_{max}$ in the patient of about 75% to about 130% of about 42 ng/mL and a mean $AUC_{\infty}$ in the patient of about 75% to about 130% of 2328 ng·h/mL; and the at least one daily maintenance dose is effective to achieve a mean $C_{max}$ at steady-state in the patient of about 75% to about 130% of 78 ng/mL, and a mean $AUC_{\tau}$ at steady-state in the patient of about 75% to about 130% of 1487 ng·h/mL, after the at least one daily maintenance dose.

In one embodiment, the method is for treating and/or preventing relapse of depression in a patient, the method comprising: administering to the patient a first dose comprising 9-[4-(cyclohexyloxy)phenyl]-7-methyl-3,4-dihydropyrazino[2,1-c][1,2,4]thiadiazine 2,2-dioxide ("COMPOUND A" or "CMPD A") or a pharmaceutically acceptable salt thereof; and administering to the patient at least one daily maintenance dose comprising COMPOUND A or a pharmaceutically acceptable salt thereof, wherein the maintenance dose is less than the first dose.

In some embodiments, the depression is major depressive disorder. In some embodiments, the depression is treatment-resistant depression. In some embodiments, the method treats and/or prevents cognitive impairment. In some embodiments, the method treats and/or prevents cognitive impairment associated with the depression, as measured by the Brief Assessment of Cognition. In some embodiments, the method treats and/or prevents cognitive impairment involving decline in speed of processing, executive function, attention, or verbal learning and memory. In some embodiments, the method treats and/or prevents cognitive impairment associated with the depression. In some embodiments, the method treats and/or prevents cognitive impairment involving decline in speed of processing, executive function, attention, or verbal learning and memory associated with the depression.

In some embodiments, the patient has already received at least one prior therapy for depression. In some embodiments, the patient has failed to respond to at least one prior therapy for depression. In some embodiments, the patient is a responder to at least one prior therapy for depression.

In some embodiments, the prior therapy for depression comprises an administration of: ketamine or a salt

thereof; (S)-ketamine (esketamine) or a salt thereof; (R)-ketamine or a salt thereof; norketamine or a salt thereof; 2 diastereomeric hydroxyketamine or a salt thereof; 6 diastereomeric hydroxynorketamine (HNK) or a salt thereof; (2S,6S)-HNK or a salt thereof; (2R,6R)-HNK or a salt thereof; dehydronorketamine or a salt thereof; methoxetamine or a salt thereof; or a combination thereof. In some embodiments, the prior therapy comprises an administration of ketamine or a salt thereof; (S)-ketamine (esketamine) or a salt thereof; methoxetamine or a salt thereof; or a combination thereof.

In some embodiments, the maintenance dose is administered not less than about 12 hours after the first dose.

In some embodiments, the first dose comprises between about 5 mg and 9 mg of COMPOUND A.

In some embodiments, the maintenance dose comprises between about 0.5 mg and about 4 mg once per day of COMPOUND A.

In some embodiments, the first dose is effective to achieve a mean $C_{max}$ in the patient of not less than about 6 ng/mL. In some embodiments, the first dose is effective to achieve a mean $C_{max}$ in the patient of not more than 100 ng/mL.

In some embodiments, the maintenance dose is effective to achieve a mean $C_{max}$ at steady-state in the patient of no greater than about 130 ng/mL.

In some embodiments, the first dose is effective to achieve a mean $C_{max}$ in the patient of about 6 ng/mL to about 100 ng/mL. In some embodiments, the first dose is effective to achieve a mean $C_{max}$ in the patient of about 75% to about 130% of 42 ng/mL. In some embodiments, the maintenance dose is effective to achieve a mean $C_{max}$ at steady-state in the patient is about 75% to about 130% of 78 ng/mL.

In some embodiments, the mean $AUC_{\tau}$ at steady-state in the patient is no greater than about 2600 ng·h/mL. In some embodiments, the mean $AUC_{\tau}$ at steady-state in the patient is about 700 ng·h/mL to about 2600 ng·h/mL. In some embodiments, the mean $AUC_{\tau}$ at steady-state in the patient is about 75% to about 130% of 1487 ng·h/mL. In some embodiments, the mean $AUC_{\infty}$ in the patient is about 75% to about 130% of 2328 ng·h/mL.

In some embodiments, the first dose is orally administered. In some embodiments, the at least one daily maintenance dose is orally administered.

In some embodiments, the first dose comprises about 6 mg of COMPOUND A or a pharmaceutically acceptable salt thereof. In some embodiments, the first dose comprises about 6 mg of COMPOUND A.

In some embodiments, the maintenance dose comprises about 3 mg once per day of COMPOUND A or a pharmaceutically acceptable salt thereof. In some embodiments, the maintenance dose comprises about 3 mg once per day of COMPOUND A.

In some embodiments, the first dose comprises about 6 mg of COMPOUND A or a pharmaceutically acceptable salt thereof, and the at least one daily maintenance dose comprises about 3 mg once per day of COMPOUND A or a pharmaceutically acceptable salt thereof. In some embodiments, the first dose comprises about 6 mg of COMPOUND A, and the at least one daily maintenance dose comprises about 3 mg once per day of COMPOUND A.

In some embodiments, the first dose is effective to achieve a mean $C_{max}$ in the patient of about 40 ng/mL to about 100 ng/mL; and the at least one daily maintenance dose is effective to achieve a mean $C_{max}$ at steady-state in the patient of no greater than 130 ng/mL, and a mean $AUC_{\tau}$ at steady-state in the patient of no greater than 2600 ng·h/mL. In some embodiments, the first dose is effective to achieve a mean $C_{max}$ in the patient of about 40 ng/mL to about 100 ng/mL; and the at least one daily maintenance dose is effective to achieve a mean $C_{max}$ at steady-state in the patient of no greater than about 130 ng/mL, and a mean $AUC_{\tau}$ at steady-state in the patient of no greater than about 2600 ng·h/mL.

In some embodiments, the first dose is effective to achieve a mean $C_{max}$ in the patient of about 75% to about 130% of about 42 ng/mL; and the at least one daily maintenance dose is effective to achieve a mean $C_{max}$ at steady-state in the patient of about 75% to about 130% of 78 ng/mL, and a mean $AUC_{\tau}$ at steady-state in the patient of about 75% to about 130% of 1487 ng·h/mL, after the at least one daily maintenance dose. In some embodiments, the first dose is effective to achieve a mean $C_{max}$ in the patient of about 75% to about 130% of 42 ng/mL; and the at least one daily maintenance dose is effective to achieve a mean $C_{max}$ at steady-state in the patient of about 75% to about 130% of 78 ng/mL, and a mean $AUC_{\tau}$ at steady-state in the patient of about 75% to about 130% of 1487 ng·h/mL, after the at least one daily maintenance dose. In some embodiments, the first dose is effective to achieve a mean $C_{max}$ in the patient of about 75% to about 130% of about 42 ng/mL and a mean $AUC_{\infty}$ in the patient of about 75% to about 130% of 2328 ng·h/mL.; and the at least one daily maintenance dose is effective to achieve a mean $C_{max}$ at steady-state in the patient of about 75% to about 130% of 78 ng/mL, and a mean $AUC_{\tau}$ at steady-state in the patient of about 75% to about 130% of 1487 ng·h/mL, after the at least one daily maintenance dose. In some embodiments, the first dose is effective to achieve a mean $C_{max}$ in the patient of about 75% to about 130% of 42 ng/mL and a mean $AUC_{\infty}$ in the patient of about 75% to about 130% of 2328 ng·h/mL.; and the at least one daily maintenance dose is effective to achieve a mean $C_{max}$ at steady-state in the patient of about 75% to about 130% of 78 ng/mL, and a mean $AUC_{\tau}$ at steady-state in the patient of about 75% to about 130% of 1487 ng·h/mL, after the at least one daily maintenance dose.

It should be understood that references herein to methods of treatment and/or prevention (e.g., methods of treating and/or preventing relapse of depression) using 9-[4-(Cyclohexyloxy)phenyl]-7-methyl-3,4-dihydropyrazino[2,1-c][1,2,4]thiadiazine 2,2-dioxide ("COMPOUND A") or a pharmaceutically acceptable salt thereof, or a pharmaceutical

composition comprising the same, should be interpreted as references to:

- COMPOUND A or a pharmaceutically acceptable salt thereof, or a pharmaceutical composition comprising the same, for use in methods of treating and/or preventing relapse of, e.g., depression.

BRIEF DESCRIPTION OF THE DRAWINGS

FIG. 1A is a study schematic for Part 1 single rising dose ("SRD") Cohorts 1 to 6.

FIG. 1B is a study schematic for Part 2 SRD/multiple rising dose ("MRD") Cohorts 1 to 5.

FIG. 2 shows linear (upper) and semilog (lower) mean plasma concentration-time profiles of COMPOUND A following single oral administration of COMPOUND A.

FIG. 3 shows linear (upper) and semilog (lower) mean concentration-time plots of COMPOUND A on Day 18 following oral administration of COMPOUND A once a day ("QD") for 13 days.

**FIG.** 4 shows a schematic illustration of the final population pharmacokinetics ("PK") model.

FIG. 5 shows a summary of simulated scenarios of the planned Phase 2 clinical study. Solid lines represent predicted median concentrations. Dashed lines represent [5% - 95%] prediction intervals.

FIG. 6 shows a study schematic for the randomized, double-blind, placebo-controlled, 3-period crossover study followed by 1 open-label comparator period, for evaluating the central nervous system ("CNS") pharmacodynamics ("PD") activity of COMPOUND A in healthy subjects using transcranial magnetic stimulation ("TMS").

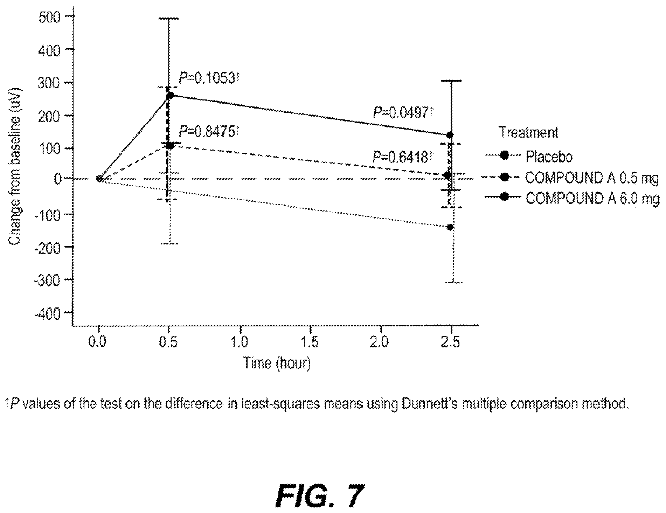

FIG. 7 shows a plot of the change in peak-to-peak amplitude of the motor evoked potential ("MEP"), relative to baseline, versus time. Peak-to-peak amplitudes of MEP were obtained using single-pulse TMS for placebo, 0.5 mg COMPOUND A, and 6.0 mg COMPOUND A.

FIG. 8 is a study schematic for a planned Phase 1b randomized, double-blind, placebo-controlled study to assess the safety and pharmacokinetics of adjunctive COMPOUND A in adult subjects with major depressive disorder.

**Example Embodiments:**

Non-limiting embodiments/clauses of the disclosure include:

The method described herein is for treating depression in a patient, the method comprising:

administering to the patient a first dose comprising 9-[4-(cyclohexyloxy)phenyl]-7-methyl-3,4-dihydropyrazino [2,1-c][1,2,4]thiadiazine 2,2-dioxide ("COMPOUND A") or a pharmaceutically acceptable salt thereof; and administering to the patient at least one daily maintenance dose comprising COMPOUND A or pharmaceutically acceptable salt thereof, wherein the at least one daily maintenance dose is less than the first dose.

In some embodiments, the depression is major depressive disorder.

In some embodiments, the depression is treatment-resistant depression.

In some embodiments, treating depression comprises treating cognitive impairment associated with the depression.

In some embodiments, the patient has already received at least one prior therapy for depression.

In some embodiments, the patient has failed to respond to at least one prior therapy for depression.

In some embodiments, the patient is a responder to at least one prior therapy for depression.

In some embodiments, the at least one prior therapy for depression comprises an administration of: ketamine or a salt thereof; (S)-ketamine (esketamine) or a salt thereof; (R)-ketamine or a salt thereof; norketamine or a salt thereof; 2 diastereomeric hydroxyketamine or a salt thereof; 6 diastereomeric hydroxynorketamine (HNK) or a salt thereof; (2S,6S)-HNK or a salt thereof; (2R,6R)-HNK or a salt thereof; dehydronorketamine or a salt thereof; methoxetamine or a salt thereof; or combinations thereof.

In some embodiments, the at least one prior therapy comprises an administration of ketamine or a salt thereof; (S)-ketamine (esketamine) or a salt thereof; methoxetamine or a salt thereof; or combinations thereof.

In some embodiments, the at least one daily maintenance dose is administered not less than about 12 hours after the first dose.

In some embodiments, the first dose comprises between about 5 mg and about 9 mg of COMPOUND A or a pharmaceutically acceptable salt thereof.

In some embodiments, the at least one daily maintenance dose comprises between about 0.5 mg and about 4 mg once per day of COMPOUND A or a pharmaceutically acceptable salt thereof.

In some embodiments, the first dose is effective to achieve a mean $C_{max}$ in the patient of at least about 6 ng/mL.

In some embodiments, the first dose is effective to achieve a mean $C_{max}$ in the patient of not more than about 100 ng/mL.

In some embodiments, the at least one daily maintenance dose is effective to achieve a mean $C_{max}$ at steady-state in the patient of no greater than about 130 ng/mL.

In some embodiments, the first dose is effective to achieve a mean $C_{max}$ in the patient of about 6 ng/mL to about 100 ng/mL.

In some embodiments, the first dose is effective to achieve a mean $C_{max}$ in the patient of about 75% to about 130% of 42 ng/mL.

In some embodiments, the at least one daily maintenance dose is effective to achieve a mean $C_{max}$ at steady-state in the patient of about 75% to about 130% of 78 ng/mL.

In some embodiments, the mean $AUC_{\tau}$ at steady-state in the patient is no greater than about 2600 ng·h/mL.

In some embodiments, the mean $AUC_{\tau}$ at steady-state in the patient is about 700 ng·h/mL to about 2600 ng·h/mL.

In some embodiments, the mean $AUC_{\tau}$ at steady-state in the patient is about 75% to about 130% of 1487 ng·h/mL.

In some embodiments, the mean $AUC_{\infty}$ in the patient is about 75% to about 130% of 2328 ng·h/mL.

In some embodiments, the first dose is orally administered.

In some embodiments, the at least one daily maintenance dose is orally administered.

In some embodiments, the first dose comprises about 6 mg of COMPOUND A or a pharmaceutically acceptable salt thereof.

In some embodiments, the maintenance dose comprises about 3 mg once per day of COMPOUND A or a pharmaceutically acceptable salt thereof.

In some embodiments:

the first dose is effective to achieve a mean $C_{max}$ in the patient of about 40 to about 100 ng/mL; and
the at least one daily maintenance dose is effective to achieve a mean $C_{max}$ at steady-state in the patient of no greater than about 130 ng/mL, and a mean $AUC_{\tau}$ at steady-state in the patient of no greater than about 2600

ng·h/mL, after the at least one daily maintenance dose.

In some embodiments:

the first dose is effective to achieve a mean $C_{max}$ in the patient of about 75% to about 130% of about 42 ng/mL; and the at least one daily maintenance dose is effective to achieve a mean $C_{max}$ at steady-state in the patient of about 75% to about 130% of 78 ng/mL, and a mean $AUC_{\tau}$ at steady-state in the patient of about 75% to about 130% of 1487 ng·h/mL, after the at least one daily maintenance dose.

In some embodiments:

the first dose is effective to achieve a mean $C_{max}$ in the patient of about 75% to about 130% of 42 ng/mL, and a mean $AUC_{\infty}$ in the patient of about 75% to about 130% of 2328 ng·h/mL; and the at least one daily maintenance dose is effective to achieve a mean $C_{max}$ at steady-state in the patient of about 75% to about 130% of 78 ng/mL, and a mean $AUC_{\tau}$ at steady-state in the patient of about 75% to about 130% of 1487 ng·h/mL, after the at least one daily maintenance dose.

Also described herein is a method for preventing relapse of depression in a patient, comprising:

administering to the patient a first dose comprising 9-[4-(cyclohexyloxy)phenyl]-7-methyl-3,4-dihydropyrazino [2,1-c][1,2,4]thiadiazine 2,2-dioxide ("COMPOUND A") or a pharmaceutically acceptable salt thereof; and administering to the patient at least one daily maintenance dose comprising COMPOUND A or a salt thereof, wherein the at least one daily maintenance dose is less than the first dose.

In some embodiments, the depression is major depressive disorder.

In some embodiments, the depression is treatment-resistant depression.

In some embodiments, the method further comprises treating and/or preventing cognitive impairment associated with the depression in the patient.

In some embodiments, the patient has already received at least one prior therapy for depression.

In some embodiments, the patient has failed to respond to at least one prior therapy for depression.

In some embodiments, the patient is a responder to at least one prior therapy for depression.

In some embodiments, the at least one prior therapy for depression comprises an administration of: ketamine or a salt thereof; (S)-ketamine (esketamine) or a salt thereof; (R)-ketamine or a salt thereof; norketamine or a salt thereof; 2 diastereomeric hydroxyketamine or a salt thereof; 6 diastereomeric hydroxynorketamine (HNK) or a salt thereof; (2S,6S)-HNK or a salt thereof; (2R,6R)-HNK or a salt thereof; dehydronorketamine or a salt thereof; methoxetamine or a salt thereof; or combinations thereof.

In some embodiments, the at least one prior therapy comprises an administration of ketamine or a salt thereof; (S)-ketamine (esketamine) or a salt thereof; methoxetamine or a salt thereof; or a combination thereof.

In some embodiments, the at least one daily maintenance dose is administered not less than about 12 hours after the first dose.

In some embodiments, the first dose comprises between about 5 mg and about 9 mg of COMPOUND A or a pharmaceutically acceptable salt thereof.

In some embodiments, the at least one daily maintenance dose comprises between about 0.5 mg and about 4 mg once per day of COMPOUND A or a pharmaceutically acceptable salt thereof.

In some embodiments, the first dose is effective to achieve a mean $C_{max}$ in the patient of not less than about 6 ng/mL.

In some embodiments, the first dose is effective to achieve a mean $C_{max}$ in the patient of not more than about 100

ng/mL.

In some embodiments, the at least one daily maintenance dose is effective to achieve a mean $C_{max}$ at steady-state in the patient of no greater than about 130 ng/mL.

In some embodiments, the first dose is effective to achieve a mean $C_{max}$ in the patient of about 6 ng/mL to about 100 ng/mL.

In some embodiments, the first dose is effective to achieve a mean $C_{max}$ in the patient of about 75% to about 130% of 42 ng/mL.

In some embodiments, the mean $C_{max}$ at steady-state in the patient is about 75% to about 130% of 78 ng/mL.

In some embodiments, the mean $AUC_{\tau}$ at steady-state in the patient is no greater than about 2600 ng·h/mL.

In some embodiments, the mean $AUC_{\tau}$ at steady-state in the patient is about 700 ng·h/mL to about 2600 ng·h/mL.

In some embodiments, the mean $AUC_{\tau}$ at steady-state in the patient is about 75% to about 130% of 1487 ng·h/mL.

In some embodiments, the mean $AUC_{\infty}$ in the patient is about 75% to about 130% of 2328 ng·h/mL.

In some embodiments, the first dose is orally administered.

In some embodiments, the at least one daily maintenance dose is orally administered.

In some embodiments, the first dose comprises about 6 mg of COMPOUND A or a pharmaceutically acceptable salt thereof.

In some embodiments, the maintenance dose comprises about 3 mg once per day of COMPOUND A or a pharmaceutically acceptable salt thereof.

In some embodiments:

the first dose is effective to achieve a mean $C_{max}$ in the patient of about 40 to about 100 ng/mL; and
the at least one daily maintenance dose is effective to achieve a mean $C_{max}$ at steady-state in the patient of no greater than about 130 ng/mL, and a mean $AUC_{\tau}$ at steady-state in the patient of no greater than about 2600 ng·h/mL.

In some embodiments:

the first dose is effective to achieve a mean $C_{max}$ in the patient of about 75% to about 130% of about 42 ng/mL; and
the at least one daily maintenance dose is effective to achieve a mean $C_{max}$ at steady-state in the patient of about 75% to about 130% of 78 ng/mL, and a mean $AUC_{\tau}$ at steady-state in the patient of about 75% to about 130% of 1487 ng·h/mL, after the at least one daily maintenance dose.

In some embodiments:

the first dose is effective to achieve a mean $C_{max}$ in the patient of about 75% to about 130% of 42 ng/mL, and a mean $AUC_{\infty}$ in the patient of about 75% to about 130% of 2328 ng·h/mL; and
the at least one daily maintenance dose is effective to achieve a mean $C_{max}$ at steady-state in the patient of about 75% to about 130% of 78 ng/mL, and a mean $AUC_{\tau}$ at steady-state in the patient of about 75% to about 130% of 1487 ng·h/mL, after the at least one daily maintenance dose.

In some embodiments, the first dose comprises COMPOUND A.

In some embodiments, the at least one daily maintenance dose comprises COMPOUND A.

In some embodiments, the patient is 18 to 55 years old.

In some embodiments, the patient is 20 to 49 years old.

In some embodiments, treating depression comprises improving symptoms of depression, as measured by the Montgomery Åsberg Depression Rating Scale (MADRS).

In some embodiments, treating depression comprises reducing MDD severity, as measured by the Clinical Global Impression-Severity Scale (CGI-S).

In some embodiments, treating depression comprises treating MDD, as measured by the Clinical Global Impression-Severity Scale (CGI-S).

In some embodiments, treating depression comprises improving quality of life as measured by 5 Level EuroQol-5 Dimension.

In some embodiments, the patient is on stable pharmacological treatment for depression.

In some embodiments, the patient is on stable pharmacological treatment for depression, defined as ≤50% change in dose during the 6 weeks prior to randomization.

In some embodiments, the first dose comprises about 6 mg of COMPOUND A or a pharmaceutically acceptable salt thereof, and the at least one daily maintenance dose comprises about 3 mg of COMPOUND A or a pharmaceutically acceptable salt thereof.

In some embodiments, the first dose comprises about 6 mg of COMPOUND A, and the at least one daily maintenance dose comprises about 3 mg of COMPOUND A.

In some embodiments, the first dose comprises about 6 mg of COMPOUND A or a pharmaceutically acceptable salt thereof.

In some embodiments, the maintenance dose comprises about 3 mg once per day of COMPOUND A or a pharmaceutically acceptable salt thereof.

Also described herein is a pharmaceutical composition for use in the treatment of depression comprising:

a first dose comprising 9-[4-(cyclohexyloxy)phenyl]-7-methyl-3,4-dihydropyrazino[2,1-c][1,2,4]thiadiazine 2,2-dioxide ("COMPOUND A") or a pharmaceutically acceptable salt thereof; and
at least one daily maintenance dose comprising COMPOUND A or pharmaceutically acceptable salt thereof, wherein the at least one daily maintenance dose is less than the first dose.

Also described herein is a pharmaceutical composition for use in the treatment of depression comprising 9-[4-(cyclohexyloxy)phenyl]-7-methyl-3,4-dihydropyrazino[2,1-c][1,2,4]thiadiazine 2,2-dioxide ("COMPOUND A") or a pharmaceutically acceptable salt thereof, administered in a first dose and at least one daily maintenance, wherein the at least one daily maintenance dose is less than the first dose.

Also described herein is a pharmaceutical composition for use in the prevention of relapse of depression comprising:

a first dose comprising 9-[4-(cyclohexyloxy)phenyl]-7-methyl-3,4-dihydropyrazino[2,1-c][1,2,4]thiadiazine 2,2-dioxide ("COMPOUND A") or a pharmaceutically acceptable salt thereof; and
at least one daily maintenance dose comprising COMPOUND A or pharmaceutically acceptable salt thereof, wherein the at least one daily maintenance dose is less than the first dose.

Also described herein is a pharmaceutical composition for use in the prevention of relapse of depression comprising 9-[4-(cyclohexyloxy)phenyl]-7-methyl-3,4-dihydropyrazino[2,1-c][1,2,4]thiadiazine 2,2-dioxide ("COMPOUND A") or a pharmaceutically acceptable salt thereof, administered in a first dose and at least one daily maintenance, wherein the at least one daily maintenance dose is less than the first dose.

*Definitions:*

Unless defined otherwise, all technical and scientific terms used herein have the meaning commonly understood by a person skilled in the art to which this disclosure belongs. The following references provide one of skill with a general definition of many of the terms used in this disclosure: Singleton et al., Dictionary of Microbiology and Molecular Biology (2nd ed. 1994); The Cambridge Dictionary of Science and Technology (Walker ed., 1988); The Glossary of Genetics, 5th Ed., R. Rieger et al. (eds.), Springer Verlag (1991); and Hale & Marham, The Harper Collins Dictionary of Biology (1991); The American Psychiatric Association's fifth edition of the Diagnostic and Statistical Manual of Mental Disorders (DSM-5); FDA Guidance for Industry: Bioequivalence Studies with Pharmacokinetic Endpoints for Drugs Submitted under an ANDA (2013). As used herein, the following terms have the meanings ascribed to them below, unless specified otherwise. The terminology used herein is for the purpose of describing particular embodiments only and is not intended to be limiting of the disclosure.

As used herein, the singular forms "a", "an" and "the" are intended to include the plural forms as well, unless the context clearly indicates otherwise. "A," "one or more," and "at least one" are used interchangeably herein.

As used herein, the term "about" or "approximately" means within an acceptable error range for the particular value as determined by one of ordinary skill in the art, which will depend in part on how the value is measured or determined, *i.e.,* the limitations of the measurement system. For example, "about" can mean within 3 or more than 3 standard deviations, per the practice in the art. Alternatively, "about" can mean a range of up to 20%, *e.g.,* up to 10%, *e.g.,* up to 5%, *e.g.,* up to 1% of a given value. Alternatively, particularly with respect to biological systems or processes, the term can mean within an order of magnitude, *e.g.,* within 5-fold, *e.g.,* within 2-fold, of a value.

As used herein, the term "administration" of an agent to a subject includes any route of introducing or delivering the agent to a subject to perform its intended function. Administration can be carried out by any suitable non-oral route, including, but not limited to, intravenously, intramuscularly, intraperitoneally, subcutaneously, and other suitable routes as described herein. Administration includes self-administration and the administration by another.

As used herein, the term "effective amount" or "therapeutically effective amount" refers to a quantity of COMPOUND A or a pharmaceutically acceptable salt thereof sufficient to achieve a desired effect or a desired therapeutic effect. In the context of therapeutic applications, the amount of COMPOUND A or a pharmaceutically acceptable salt thereof administered to the subject can depend on the type and severity of the depression or symptom and on the characteristics of the individual, such as general health, age, sex, body weight, and tolerance to drugs. The skilled artisan will be able to determine appropriate dosages depending on these and other factors.

In some embodiments, an amount expressed in terms of "mg of [X]," where [X] is an active, refers to the total amount in milligrams of [X] calculated based on the free base of [X]. When [X] is a pharmaceutically acceptable salt, an equivalent amount of one or more pharmaceutically acceptable salts of [X] based on the weight of free base therein may be present.

As used herein, the term "modulate" means to positively or negatively alter. Exemplary modulations include an about 1%, about 2%, about 5%, about 10%, about 25%, about 50%, about 75%, or about 100% change.

As used herein, the term "increase" refers to altering positively by at least about 5%, including, but not limited to, alter positively by about 5%, by about 10%, by about 25%, by about 30%, by about 50%, by about 75%, or by about 100%.

As used herein, the term "reduce" refers to altering negatively by at least about 5% including, but not limited to, alter negatively by about 5%, by about 10%, by about 25%, by about 30%, by about 50%, by about 75%, or by about 100%.

As used herein, a "responder" to a prior therapy for depression refers to a patient who showed an improvement in his or her symptoms of depression from baseline (before administration of the therapy) to a point in time during or after therapy. For example, the responders are selected using clinical judgement or depression rating scales (*e.g.,* the Montgomery-Åsberg Depression Rating Scale ("MADRS"), the Hamilton Depression Rating Scale ("HAM-D")). For instance, a "ketamine responder" to a prior therapy for depression refers to a subject who shows, for example, more than about any of 0%, 10%, 20%, 30%, 40%, 50, 60%, 70%, 80%, 90%, or 100% improvement in his or her symptoms of depression from pre-ketamine-like compound administration baseline to Day 1, during or after one or more administrations of ketamine-like compound, based on the MADRS or HAM-D or clinical observation by the treating physician.

As used herein, a patient who "failed to respond to at least one prior therapy for depression" refers to a subject who either failed to respond to at least one prior therapy for depression at all or responded to at least one prior therapy for depression but then subsequently relapsed. For instance, a patient who "failed to respond to at least one prior therapy for depression" refers to a subject who showed, for example, less than about any of 10%, 20%, 30%, 40%, 50%, 60%, 70%, 80%, 90%, or 100% improvement in his or her symptoms of depression from baseline (before administration of the therapy) to a point in time during or after the therapy. For example, the patients who failed to respond to at least one prior therapy for depression are selected using clinical judgement or depression rating scales (for *e.g.,* MADRS, HAM-D). For instance, a patient who "failed to respond to at least one prior therapy for depression" refers to a subject who shows, for example, less than about any of 10%, 20%, 30%, 40%, 50%, 60%, 70%, 80%, 90%, or 100% improvement in his or her symptoms of depression from pre-ketamine-like compound administration baseline to Day 1, during or after one or more

administrations of ketamine-like compound, based on the MADRS or HAM-D or clinical observation by the treating physician. "Patient" and "subject" are used interchangeably herein.

DETAILED DESCRIPTION

Again, references herein to methods of treatment by therapy or surgery or in vivo diagnosis methods in this description are to be interpreted as references to compounds, pharmaceutical compositions and medicaments for use in those methods.

The present disclosure relates to a method for treating depression in a patient, comprising: administering to the patient a first dose comprising 9-[4-(cyclohexyloxy)phenyl]-7-methyl-3,4-dihydropyrazino[2,1-c][1,2,4]thiadiazine 2,2-dioxide ("COMPOUND A" or "CMPD A") or a pharmaceutically acceptable salt thereof; and administering to the patient at least one daily maintenance dose comprising COMPOUND A or a pharmaceutically acceptable salt thereof.

9-[4-(Cyclohexyloxy)phenyl]-7-methyl-3,4-dihydropyrazino[2,1-c][1,2,4]thiadiazine 2,2-dioxide ("COMPOUND A" or "CMPD A") or a salt thereof is a potent and selective positive allosteric modulator ("PAM") of alpha-amino-3-hydroxy-5-methyl-4-isoxazole-propionic acid ("AMPA") receptors. Synthesis and characterization of COMPOUND A or a salt thereof may proceed according to the procedures disclosed in U.S. Patent No. 8,575,154.

O O S N N N O $CH_3$

(COMPOUND A)

Alpha-amino-3-hydroxy-5-methyl-4-isoxazolepropionic acid ("AMPA") receptors are ubiquitously expressed throughout the central nervous system ("CNS") and play a central role in a multitude of higher neurophysiological processes, including attention, learning, memory, and other cognitive functions. Therefore, AMPA receptors are attractive as potential therapeutic targets for rapid onset of antidepressant activity in patients not responding to classical antidepressants. 9-[4-(Cyclohexyloxy)phenyl]-7-methyl-3,4-dihydropyrazino[2,1-c][1,2,4]thiadiazine 2,2-dioxide ("COMPOUND A" or "CMPD A") or a salt thereof is a potent and selective positive allosteric modulator (PAM) of alpha-amino-3-hydroxy-5-methyl-4-isoxazole-propionic acid (AMPA) receptors.

In some embodiments, a salt of COMPOUND A (*e.g.,* a pharmaceutically acceptable salt of COMPOUND A) or a prior therapy for depression therefor, a pharmaceutically acceptable salt, and the like are used. In some embodiments, a salt of COMPOUND A or a prior therapy for depression therefor, salts with inorganic bases, salts with organic bases, salts with inorganic acids, salts with organic acids, salts with basic or acidic amino acids and the like are used. In some embodiments, the salts with inorganic bases include alkali metal salts such as sodium salt, potassium salt and the like, alkaline earth metal salts such as calcium salt, magnesium salt and the like, aluminum salt, and ammonium salt. In some embodiments, the salts with organic bases include salts with trimethylamine, triethylamine, pyridine, picoline, 2,6-lutidine, ethanolamine, diethanolamine, triethanolamine, cyclohexylamine, dicyclohexylamine, N,N'-dibenzylethylenediamine, and the like. In some embodiments, the salts with inorganic acids include salts with hydrochloric acid, hydroiodic acid, hydrobromic acid, nitric acid, sulfuric acid, phosphoric acid, and the like. In some embodiments, the salts with organic acids include salts with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, p-toluenesulfonic acid, and the like. In some embodiments, the salts with basic amino acids include salts with arginine, lysine, ornithine, and the like. In some embodiments, the salts with acidic amino acids include salts with aspartic acid, glutamic acid, and the like. In some embodiments, when the compound has an acidic functional group, inorganic salts such as alkali metal salts (e.g., sodium salt, potassium salt, etc.), alkaline earth metal salts (e.g., calcium salt, magnesium salt, barium salt, etc.) and the like, ammonium salts, and the like are used. In some embodiments, when the compound has a basic functional group, salts with inorganic acids such as hydrochloric acid, hydroiodic acid, hydrobromic acid, nitric acid, sulfuric acid, phosphoric acid and the like, and salts with organic acids such as acetic acid, phthalic acid, fumaric acid, oxalic acid, tartaric acid, maleic acid, citric acid, succinic acid, methanesulfonic acid, p-toluenesulfonic acid, and the like are used.

### *Methods of Treating Depression*

The present disclosure relates to a method of treating depression in a patient as described herein.

The method may be a method of treating disruptive mood dysregulation disorder, major depressive disorder, persistent depressive disorder dysthymia, premenstrual dysphoric disorder, substance/medication-induced depressive disorder, depressive disorder due to another medical condition, other specified depressive disorder, unspecified depressive disorder, moderate depression, severe depression and/or specifiers for depressive disorders in a patient. In some embodiments, the depression is major depressive disorder ("MDD"). In some embodiments, the depression is treatment-resistant depression ("TRD"). In some embodiments, the TRD is MDD with a minimum of two prior antidepressant treatment failures and confirmation of prior adequate dose and duration. In some embodiments, the method further treats cognitive impairment associated with depression. In some embodiments, the method treats and/or prevents cognitive impairment involving decline in speed of processing, executive function, attention, or verbal learning and memory associated with the depression. In some embodiments, the present disclosure relates to a method for treating and/or preventing relapse in depression.

In some embodiments, the patient has already received at least one prior therapy for depression. In some embodiments, the patient has failed to respond to at least one prior therapy for depression. In some embodiments, the patient is a responder to at least one prior therapy for depression.

In some embodiments, the depression is diagnosed and/or the patient subpopulation is selected by at least one diagnostic and/or prognostic test. In some embodiments, the diagnostic test and/or the prognostic tests include genomic tests, combinatorial pharmacogenetic tests, proteomic/metabolomic tests, systematic biological pathway tests, computerized adaptive diagnostic tests, machine learning tests, EEG tests, magnetic resonance imaging (MRI) tests, positron emission tomography (PET) tests, magnetoencephalography (MEG) tests, TMS tests and/or dexamethasone suppression tests. In some embodiments, the genomic tests include providing the patient's genotype comprising CYP1A2, CYP2C19, CYP2D6, SLC6A4, HTR2A, CYP2B6, CYP2C9, CYP3A4, CYP2E1, CYP1A1, CYP1A1*lA, CYP1A1*2, CYP1A1*3, CYP1A1*4, CYP1A2*lA, CYP1A2*3, CYP2C19*1A, CYP2C19*1B, CYP2C19*2A, CYP2D6*1A, CYP2D6*2, CYP2D6*2N, CYP2D6*3, CYP2D6*4, CYP2D6*5, CYP2D6*6, CYP2D6*7, CYP2D6*8, CYP2D6*10, CYP2D6*12, CYP 2D6*17, SLC6A4, UGT1A4, UGT2B15, SLC6A4, HTR2A, HLA-A*3101, HLA-B*1502, IL-1, IL-6, and/or TNF. In some embodiments, the proteomic/metabolomic tests include providing the patient's proteomic/metabolomic type comprising BDNF, IL-1, IL-6, and/or TNF. In some embodiments, the MRI tests are performed by fMRI, sMRI, and/or DTI. In some embodiments, TMS tests are performed by TMS and/or rTMS. In some embodiments, the machine learning tests analyze data generated from genomic tests, proteomic/metabolomic tests, systematic biological pathway tests, computerized adaptive diagnostic tests, EEG tests, MRI tests, PET tests, MEG tests, TSM tests, dexamethasone suppression tests, sensing devices, and/or mobile devices.

In some embodiments, the prior therapy for depression comprises an administration of ketamine or ketamine-like compounds (*e.g.*, a ketamine-like compound). In some embodiments, the prior therapy for depression comprises an administration of: ketamine or a salt thereof; (S)-ketamine (esketamine) or a salt thereof; (R)-ketamine or a salt thereof; norketamine or a salt thereof; 2 diastereomeric hydroxyketamine or a salt thereof; 6 diastereomeric hydroxynorketamine (HNK) or a salt thereof; (2S,6S)-HNK or a salt thereof; (2R,6R)-HNK or a salt thereof; dehydronorketamine or a salt thereof; methoxetamine or a salt thereof; or combinations thereof. In some embodiments, the prior therapy for depression comprises an administration of ketamine or a salt thereof; (S)-ketamine (esketamine) or a salt thereof; methoxetamine or a salt thereof; or combinations thereof. In some embodiments, the prior therapy for depression comprises an administration of ketamine or a salt thereof; (S)-ketamine (esketamine) or a salt thereof; or combinations thereof.

In some embodiments, the prior therapy for depression comprises an administration of: benzodiazepine (chlordiazepoxide or a salt thereof, diazepam or a salt thereof, potassium clorazepate or a salt thereof, lorazepam or a salt thereof, clonazepam or a salt thereof, alprazolam or a salt thereof, etc.); L-type calcium channel inhibitor (pregabalin or a salt thereof, etc.); tricyclic or tetracyclic antidepressant (imipramine or a salt thereof, amitriptyline or a salt thereof, desipramine or a salt thereof, clomipramine or a salt thereof, etc.); selective serotonin reuptake inhibitor (fluvoxamine or a salt thereof, fluoxetine or a salt thereof, citalopram or a salt thereof, sertraline or a salt thereof, paroxetine or a salt thereof, escitalopram or a salt thereof, etc.); serotonin-noradrenaline reuptake inhibitor (venlafaxine or a salt thereof, duloxetine or a salt thereof, desvenlafaxine or a salt thereof ,etc.); noradrenaline reuptake inhibitor (reboxetine or a salt thereof, etc.); noradrenaline-dopamine reuptake inhibitor (bupropion or a salt thereof etc.); mirtazapine or a salt thereof; trazodone or a salt thereof; nefazodone or a salt thereof; bupropion or a salt thereof; setiptiline or a salt thereof; 5-HT1A agonist (buspirone or a salt thereof, tandospirone or a salt thereof, osemozotan or a salt thereof, etc.); 5-HT3 antagonist (cyamemazine or a salt thereof, etc.); heart non-selective inhibitor (propranolol or a salt thereof, oxprenolol or a salt thereof, etc.); histamine H1 antagonist (hydroxyzine or a salt thereof, etc.); therapeutic drug for schizophrenia (chlorpromazine or a salt thereof, haloperidol or a salt thereof, sulpiride or a salt thereof, clozapine or a salt thereof, trifluoperazine or a salt thereof, fluphenazine or a salt thereof, olanzapine or a salt thereof, quetiapine or a salt thereof, risperidone or a salt thereof, aripiprazole or a salt thereof, etc.); CRF antagonist; other antianxiety drug (meprobamate or a

salt thereof, etc.); psilocybin or a salt thereof; lysergic acid diethylamide or a salt thereof; mescaline or a salt thereof; N,N-dimethyltryptamine or a salt thereof; brexanolone or a salt thereof; zuranolone or a salt thereof; rapastinel or a salt thereof; dextromethorphan or a salt thereof; dextromethadone or a salt thereof; pimavanserin or a salt thereof; seltorexant or a salt thereof; L-4-chlorokynurenine or a salt thereof; 2-[(4R)-5-[2-chloro-3-(trifluoromethyl)benzoyl]-4-methyl-1H,4H,5H,6H,7H-[1,2,3]triazolo[4,5-c]pyridin-1-yl]-5-fluoropyrimidine or a salt thereof; 3β-methoxypregnenolone or a salt thereof; aticaprant or a salt thereof; cariprazine or a salt thereof; N-(4-chloropyridin-3-yl)-4-[(2,2-difluoro-1,3-benzodioxol-5-yl)methyl]piperazine-1-carboxamide or a salt thereof; chlorokynurenine or a salt thereof; sirukumab a salt thereof; or combinations thereof.

In some embodiments, the patient is on stable pharmacological treatment for depression. In some embodiments, the patient is on stable pharmacological treatment for depression, defined as ≤50% change in dose during the 6 weeks prior to randomization.

In some embodiments, the patient is 18 to 65 years of age. In some embodiments, the patient is 18 to 55 years of age. In some embodiments, the patient is 20 to 49 years of age.

In some embodiments, the methods of the present disclosure relate to treating depression and methods of treating and/or preventing relapse of depression in a patient (*e.g.,* human patient) while reducing incidence and/or severity of at least one potential adverse event of COMPOUND A or a pharmaceutically acceptable salt thereof. In some embodiments, the methods of the present disclosure also relate to reducing incidence and/or severity of at least one potential adverse event of COMPOUND A or a pharmaceutically acceptable salt thereof in the treatment of depression and treatment and/or the prevention of relapse of depression in a patient, comprising: administering to the patient a first dose comprising COMPOUND A or a pharmaceutically acceptable salt thereof; and administering to the patient at least one daily maintenance dose comprising COMPOUND A or a pharmaceutically acceptable salt thereof, wherein the maintenance dose is less than the first dose. In some embodiments, the at least one potential adverse event is neurotoxicity. In some embodiments, the at least one potential adverse event is chosen from convulsions, tonic convulsions, convulsive seizures, seizures, catalepsy, partial seizures, epilepsy, myoclonic jerks, decreased activity, weakness, uncoordinated movements, salivation, sedation, nystagmus, muscle twitches, tremors, ataxia, emesis, yawning, and combinations thereof.

In some embodiments, treating depression comprises improving symptoms of depression, as measured by the Montgomery Åsberg Depression Rating Scale (MADRS). In some embodiments, treating depression comprises reducing MDD severity, as measured by the Clinical Global Impression-Severity Scale (CGI-S). In some embodiments, treating depression comprises treating MDD, as measured by the Clinical Global Impression-Severity Scale (CGI-S). In some embodiments, treating depression comprises improving quality of life as measured by 5 Level EuroQol-5 Dimension.

In some embodiments, COMPOUND A or a pharmaceutically acceptable salt thereof is used in combination with or in adjunctive combination with a therapeutically effective amount of one or more other active ingredients. In some embodiments, COMPOUND A or a pharmaceutically acceptable salt thereof (*e.g.*, COMPOUND A) is used in combination with or in adjunctive combination with a therapeutically effective amount of one or more other prior therapies for depression. In some embodiments, the administration time of COMPOUND A in combination with or in adjunctive combination with one or more other active ingredients is not restricted, and COMPOUND A or a pharmaceutical composition thereof and one or more other active ingredients or a pharmaceutical composition thereof are administered to an administration subject simultaneously (*e.g.*, a human patient), or are administered at different times.

***First Dose***

In some embodiments, the first dose is effective to achieve a mean $C_{max}$ of COMPOUND A in the patient of at least about 1 ng/mL, at least about 2 ng/mL, at least about 3 ng/mL, at least about 4 ng/mL, at least about 5 ng/mL, at least about 6 ng/mL, at least about 7 ng/mL, at least about 8 ng/mL, at least about 9 ng/mL, at least about 10 ng/mL, at least about 11 ng/mL, at least about 12 ng/mL, at least about 13 ng/mL, at least about 14 ng/mL, at least about 15 ng/mL, at least about 16 ng/mL, at least about 17 ng/mL, at least about 18 ng/mL, at least about 19 ng/mL, at least about 20 ng/mL, at least about 21 ng/mL, at least about 22 ng/mL, at least about 23 ng/mL, at least about 24 ng/mL, at least about 25 ng/mL, at least about 26 ng/mL, at least about 27 ng/mL, at least about 28 ng/mL, at least about 29 ng/mL, at least about 30 ng/mL, at least about 31 ng/mL, at least about 32 ng/mL, at least about 33 ng/mL, at least about 34 ng/mL, at least about 35 ng/mL, at least about 36 ng/mL, at least about 37 ng/mL, at least about 38 ng/mL, at least about 39 ng/mL, at least about 40 ng/mL, at least about 41 ng/mL, at least about 42 ng/mL, at least about 43 ng/mL, at least about 44 ng/mL, at least about 45 ng/mL, at least about 46 ng/mL, at least about 47 ng/mL, at least about 48 ng/mL, at least about 49 ng/mL, at least about 50 ng/mL, at least about 51 ng/mL, at least about 52 ng/mL, at least about 53 ng/mL, at least about 54 ng/mL, at least about 55 ng/mL, at least about 60 ng/mL, at least about 65 ng/mL, at least about 70 ng/mL, at least about 75 ng/mL, at least about 80 ng/mL, at least about 85 ng/mL, at least about 90 ng/mL, at least about 95 ng/mL, at least about 100 ng/mL, at least about 105 ng/mL, at least about 110 ng/mL, at least about 115 ng/mL, at least about 120 ng/mL, at least about 125 ng/mL, at least about 130 ng/mL, at least about 135 ng/mL, at least about 140 ng/mL, at least about 145 ng/mL, at least about 150 ng/mL, at least about 160

ng/mL, at least about 165 ng/mL, at least about 170 ng/mL, at least about 175 ng/mL, at least about 180 ng/mL, at least about185 ng/mL, at least about 190 ng/mL, at least about 195 ng/mL, at least about 200 ng/mL, or any range or value therein between.

In some embodiments, the first dose is effective to achieve a mean $C_{max}$ of COMPOUND A in the patient of not more than about 200 ng/mL, not more than about 195 ng/mL, not more than about 190 ng/mL, not more than about 185 ng/mL, not more than about 180 ng/mL, not more than about 175 ng/mL, not more than about 170 ng/mL, not more than about 165 ng/mL, not more than about 160 ng/mL, not more than about 155 ng/mL, not more than about 150 ng/mL, not more than about 145 ng/mL, not more than about 140 ng/mL, not more than about 135 ng/mL, not more than about 130 ng/mL, not more than about 125 ng/mL, not more than about 120 ng/mL, not more than about 115 ng/mL, not more than about 110 ng/mL, not more than about 105 ng/mL, not more than about 100 ng/mL, not more than about 95 ng/mL, not more than about 90 ng/mL, not more than about 85 ng/mL, not more than about 80 ng/mL, not more than about 75 ng/mL, not more than about 70 ng/mL, not more than about 65 ng/mL, not more than about 60 ng/mL, not more than about 59 ng/mL, not more than about 58 ng/mL, not more than about 57 ng/mL, not more than about 56 ng/mL, not more than about 55 ng/mL, not more than about 54 ng/mL, not more than about 53 ng/mL, not more than about 52 ng/mL, not more than about 51 ng/mL, not more than about 50 ng/mL, not more than about 49 ng/mL, not more than about 48 ng/mL, not more than about 47 ng/mL, not more than about 46 ng/mL, not more than about 45 ng/mL, not more than about 44 ng/mL, not more than about 43 ng/mL, not more than about 42 ng/mL, not more than about 41 ng/mL, not more than about 40 ng/mL, not more than about 39 ng/mL, not more than about 38 ng/mL, not more than about 37 ng/mL, not more than about 36 ng/mL, not more than about 35 ng/mL, not more than about 34 ng/mL, not more than about 33 ng/mL, not more than about 32 ng/mL, not more than about 31 ng/mL, not more than about 30 ng/mL, not more than about 29 ng/mL, not more than about 28 ng/mL, not more than about 27 ng/mL, not more than about 26 ng/mL, not more than about 25 ng/mL, not more than about 20 ng/mL, not more than about 15 ng/mL, not more than about 10 ng/mL, not more than about 5 ng/mL, or any range or value therein between.

In some embodiments, the first dose is effective to achieve a mean $C_{max}$ of COMPOUND A in the patient of between about 1 ng/mL and about 200 ng/mL, between about 1 ng/mL and about 190 ng/mL, between about 1 ng/mL and about 180 ng/mL, between about 1 ng/mL and about 170 ng/mL, between about 1 ng/mL and about 160 ng/mL, between about 1 ng/mL and about 150 ng/mL, between about 1 ng/mL and about 140 ng/mL, between about 1 ng/mL and about 130 ng/mL, between about 1 ng/mL and about 125 ng/mL, between about 1 ng/mL and about 120 ng/mL, between about 1 ng/mL and about 115 ng/mL, between about 1 ng/mL and about 110 ng/mL, between about 1 ng/mL and about 105 ng/mL, between about 1 ng/mL and about 100 ng/mL, between about 1 ng/mL and about 95 ng/mL, between about 1 ng/mL and about 90 ng/mL, between about 1 ng/mL and about 85 ng/mL, between about 1 ng/mL and about 80 ng/mL, between about 1 ng/mL and about 75 ng/mL, between about 1 ng/mL and about 70 ng/mL, between about 1 ng/mL and about 65 ng/mL, between about 1 ng/mL and about 60 ng/mL, between about 1 ng/mL and about 55 ng/mL, between about 1 ng/mL and about 50 ng/mL, or any range or value therein.

In some embodiments, the first dose is effective to achieve a mean $C_{max}$ of COMPOUND A in the patient of between about 1 ng/mL and about 200 ng/mL, between about 2 ng/mL and about 200 ng/mL, between about 3 ng/mL and about 200 ng/mL, between about 4 ng/mL and about 200 ng/mL, between about 5 ng/mL and about 200 ng/mL, between about 6 ng/mL and about 200 ng/mL, between about 7 ng/mL and about 200 ng/mL, between about 8 ng/mL and about 200 ng/mL, between about 9 ng/mL and about 200 ng/mL, between about 10 ng/mL and about 200 ng/mL, between about 15 ng/mL and about 200 ng/mL, between about 20 ng/mL and about 200 ng/mL, between about 25 ng/mL and about 200 ng/mL, between about 30 ng/mL and about 200 ng/mL, between about 35 ng/mL and about 200 ng/mL, between about 40 ng/mL and about 200 ng/mL, between about 45 ng/mL and about 200 ng/mL, between about 50 ng/mL and about 200 ng/mL, between about 55 ng/mL and about 200 ng/mL, between about 60 ng/mL and about 200 ng/mL, between about 65 ng/mL and about 200 ng/mL, between about 70 ng/mL and about 200 ng/mL, between about 75 ng/mL and about 200 ng/mL, between about 80 ng/mL and about 200 ng/mL, or any range or value therein.

In some embodiments, the first dose is effective to achieve a mean $C_{max}$ of COMPOUND A in the patient of between about 1 ng/mL and about 200 ng/mL, between about 2 ng/mL and about 175 ng/mL, between about 3 ng/mL and about 150 ng/mL, between about 4 ng/mL and about 125 ng/mL, between about 5 ng/mL and about 110 ng/mL, between about 6 ng/mL and about 100 ng/mL, between about 7 ng/mL and about 95 ng/mL, between about 8 ng/mL and about 90 ng/mL, between about 9 ng/mL and about 90 ng/mL, between about 10 ng/mL and about 85 ng/mL, between about 15 ng/mL and about 80 ng/mL, between about 20 ng/mL and bout 75 ng/mL, between about 25 ng/mL and about 70 ng/mL, between about 30 ng/mL and about 65 ng/mL, between about 35 ng/mL and about 60 ng/mL, between about 40 ng/mL and about 55 ng/mL, or between about 40 ng/mL and about 50 ng/mL, or between about 40 ng/mL and about 55 ng/mL, or between about 40 ng/mL and about 60 ng/mL, or about 40 ng/mL and about 65 ng/mL, or between about 40 ng/mL and about 70 ng/mL, or between about 40 ng/mL and about 75 ng/mL, or between about 40 ng/mL and about 80 ng/mL, or between about 40 ng/mL and about 90 ng/mL, between about 40 ng/mL and about 95 ng/mL, between about 40 ng/mL and about 100 ng/mL, about 40 ng/mL and about 110 ng/mL, about 40 ng/mL and about 120 ng/mL, or between about 40 ng/mL and about 130 ng/mL, or any range or value therein.

In some embodiments, the first dose is effective to achieve a mean $C_{max}$ of COMPOUND A in the patient of at least about 5% of 42 ng/mL, at least about 10% of 42 ng/mL, at least about 15% of 42 ng/mL, at least about 20% of 42 ng/mL, at least about 25% of 42 ng/mL, at least about 30% of 42 ng/mL, at least about 35% of 42 ng/mL, at least about 40% of 42 ng/mL, at least about 45% of 42 ng/mL, at least about 50% of 42 ng/mL, at least about 55% of 42 ng/mL, at least about 60% of 42 ng/mL, at least about 65% of 42 ng/mL, at least about 70% of 42 ng/mL, at least about 75% of 42 ng/mL, at least about 80% of 42 ng/mL, at least about 85% of 42 ng/mL, at least about 90% of 42 ng/mL, at least about 95% of 42 ng/mL, at least about 100% of 42 ng/mL, at least about 105% of 42 ng/mL, at least about 110% of 42 ng/mL, at least about 115% of 42 ng/mL, at least about 120% of 42 ng/mL, at least about 125% of 42 ng/mL, at least about 130% of 42 ng/mL, at least about 135% of 42 ng/mL, at least about 140% of 42 ng/mL, at least about 145% of 42 ng/mL, or at least about 150% of 42 ng/mL, or any range or value therein.

In some embodiments, the first dose is effective to achieve a mean $C_{max}$ of COMPOUND A in the patient of no greater than about 150% of 42 ng/mL, no greater than about 145% of 42 ng/mL, no greater than about 140% of 42 ng/mL, no greater than about 135% of 42 ng/mL, no greater than about 130% of 42 ng/mL, no greater than about 125% of 42 ng/mL, no greater than about 120% of 42 ng/mL, no greater than about 115% of 42 ng/mL, no greater than about 110% of 42 ng/mL, no greater than about 105% of 42 ng/mL, no greater than about 100% of 42 ng/mL, no greater than about 95% of 42 ng/mL, no greater than about 90% of 42 ng/mL, no greater than about 85% of 42 ng/mL, no greater than about 80% of 42 ng/mL, no greater than about 75% of 42 ng/mL, no greater than about 70% of 42 ng/mL, no greater than about 65% of 42 ng/mL, no greater than about 60% of 42 ng/mL, no greater than about 55% of 42 ng/mL, no greater than about 50% of 42 ng/mL, no greater than about 45% of 42 ng/mL, no greater than about 40% of 42 ng/mL, no greater than about 30% of 42 ng/mL, no greater than about 20% of 42 ng/mL, no greater than about 10% of 42 ng/mL, no greater than about 5% of 42 ng/mL, or less, or any range or value therein.

In some embodiments, the first dose is effective to achieve a mean $C_{max}$ of COMPOUND A in the patient of between about 40% and about 165% of 42 ng/mL, between about 45% and about 160% of 42 ng/mL, between about 50% and about 155% of 42 ng/mL, between about 55% and about 150% of 42 ng/mL, between about 60% and about 145% of 42 ng/mL, between about 65% and about 140% of 42 ng/mL, between about 70% and about 135% of 42 ng/mL, between about 75% and about 130% of 42 ng/mL, between about 80% and about 125% of 42 ng/mL, between about 85% and about 120% of 42 ng/mL, between about 90% and about 115% of 42 ng/mL, between about 95% and about 110% of 42 ng/mL, between about 100% and about 105% of 42 ng/mL, between about 50% and about 80% of 42 ng/mL, between about 50% and about 90% of 42 ng/mL, between about 50% and about 100% of 42 ng/mL, between about 50% and about 110% of 42 ng/mL, between about 50% and about 120% of 42 ng/mL, between about 50% and about 125% of 42 ng/mL, between about 50% and about 130% of 42 ng/mL, between about 40% and about 100% of 42 ng/mL, between about 40% and about 105% of 42 ng/mL, between about 40% and about 110% of 42 ng/mL, between about 40% and about 115% of 42 ng/mL, between about 40% and about 120% of 42 ng/mL, between about 40% and about 125% of 42 ng/mL, between about 40% and about 130% of 42 ng/mL, between about 40% and about 135% of 42 ng/mL, between about 40% and about 140% of 42 ng/mL, or any range or value therein between.

In some embodiments, the at first dose is effective to achieve a mean $C_{max}$ of COMPOUND A in the patient of about 35% of 42 ng/mL, about 40% of 42 ng/mL, about 45% of 42 ng/mL, about 50% of 42 ng/mL, about 55% of 42 ng/mL, about 60% of 42 ng/mL, about 65% of 42 ng/mL, about 70% of 42 ng/mL, about 75% of 42 ng/mL, about 80% of 42 ng/mL, about 85% of 42 ng/mL, about 90% of 42 ng/mL, about 95% of 42 ng/mL, about 100% of 42 ng/mL, about 105% of 42 ng/mL, about 110% of 42 ng/mL, about 115% of 42 ng/mL, about 120% of 42 ng/mL, about 125% of 42 ng/mL, about 130% of 42 ng/mL, about 135% of 42 ng/mL, about 140% of 42 ng/mL, about 145% of 42 ng/mL, or about 150% of 42 ng/mL, or any range or value therein between.

In some embodiments, the first dose is effective to achieve a mean $AUC_{\infty}$ of COMPOUND A in the patient of no greater than about 3500 ng·h/mL, no greater than about 3450 ng·h/mL, no greater than about 3400 ng·h/mL, no greater than about 3350 ng·h/mL, no greater than about 3300 ng·h/mL, no greater than about 3250 ng·h/mL, no greater than about 3150 ng·h/mL, no greater than about 3100 ng·h/mL, no greater than about 3050 ng·h/mL, no greater than about 3000 ng·h/mL, no greater than about 2950 ng·h/mL, no greater than about 2900 ng·h/mL, no greater than about 2850 ng·h/mL, no greater than about 2800 ng·h/mL, no greater than about 2750 ng·h/mL, no greater than about 2700 ng·h/mL, no greater than about 2650 ng·h/mL, no greater than about 2600 ng·h/mL, no greater than about 2550 ng·h/mL, no greater than about 2500 ng·h/mL, no greater than about 2450 ng·h/mL, no greater than about 2400 ng·h/mL, no greater than about 2350 ng·h/mL, no greater than about 2300 ng·h/mL, no greater than about 2250 ng·h/mL, no greater than about 2200 ng·h/mL, no greater than about 2150 ng·h/mL, no greater than about 2100 ng·h/mL, no greater than about 2050 ng·h/mL, no greater than about 2000 ng·h/mL, no greater than about 1950 ng·h/mL, no greater than about 1900 ng·h/mL, no greater than about 1850 ng·h/mL, no greater than about 1800 ng·h/mL, no greater than about 1750 ng·h/mL, no greater than about 1700 ng·h/mL, no greater than about 1650 ng·h/mL, no greater than about 1600 ng·h/mL, or any range or value therein.

In some embodiments, the first dose is effective to achieve a mean $AUC_{\infty}$ of COMPOUND A in the patient of at least about 300 ng·h/mL, at least about 350 ng·h/mL, at least about 400 ng·h/mL, at least about 450 ng·h/mL, at least about 500 ng·h/mL, at least about 550 ng·h/mL, at least about 600 ng·h/mL, at least about 650 ng·h/mL, at least about 700

ng·h/mL, at least about 750 ng·h/mL, at least about 800 ng·h/mL, at least about 850 ng·h/mL, at least about 900 ng·h/mL, at least about 950 ng·h/mL, at least about 1000 ng·h/mL, at least about 1050 ng·h/mL, at least about 1100 ng·h/mL, at least about 1150 ng·h/mL, at least about 1200 ng·h/mL, at least about 1250 ng·h/mL, at least about 1300 ng·h/mL, at least about 1350 ng·h/mL, at least about 1400 ng·h/mL, at least about 1450 ng·h/mL, at least about 1500 ng·h/mL, at least about 1550 ng·h/mL, at least about 1600 ng·h/mL, at least about 1650 ng·h/mL, at least about 1700 ng·h/mL, at least about 1750 ng·h/mL, at least about 1800 ng·h/mL, at least about 1850 ng·h/mL, at least about 1900 ng·h/mL, at least about 1950 ng·h/mL, at least about 2000 ng·h/mL, at least about 2050 ng·h/mL, at least about 2100 ng·h/mL, at least about 2150 ng·h/mL, at least about 2200 ng·h/mL, at least about 2250 ng·h/mL, at least about 2300 ng·h/mL, at least about 2350 ng·h/mL, at least about 2400 ng·h/mL, at least about 2450 ng·h/mL, at least about 2500 ng·h/mL, at least about 2550 ng·h/mL, at least about 2600 ng·h/mL, or any range or value therein.

In some embodiments, the first dose is effective to achieve a mean $AUC_\infty$ of COMPOUND A in the patient of between about 1000 ng·h/mL and about 3500 ng·h/mL, between about 1350 ng·h/mL and about 3450 ng·h/mL, between about 1400 ng·h/mL and about 3400 ng·h/mL, between about 1450 ng·h/mL and about 3350 ng·h/mL, between about 1500 ng·h/mL and about 3300 ng·h/mL, between about 1550 ng·h/mL and about 3250 ng·h/mL, between about 1600 ng·h/mL and about 3200 ng·h/mL, between about 1650 ng·h/mL and about 3150 ng·h/mL, between about 1700 ng·h/mL and about 3100 ng·h/mL, between about 1750 ng·h/mL and about 3050 ng·h/mL, between about 1800 ng·h/mL and about 3000 ng·h/mL, between about 1850 ng·h/mL and about 2950 ng·h/mL, between about 1900 ng·h/mL and about 2900 ng·h/mL, between about 1950 ng·h/mL and about 2850 ng·h/mL, between about 2000 ng·h/mL and about 2800 ng·h/mL, or any range or value therein between.

In some embodiments, the first dose is effective to achieve a mean $AUC_\infty$ in the patient of between about 40% and about 165% of 2328 ng·h/mL, between about 45% and about 160% of 2328 ng·h/mL, between about 50% and about 155% of 2328 ng·h/mL, between about 55% and about 150% of 2328 ng·h/mL, between about 60% and about 145% of 2328 ng·h/mL, between about 65% and about 140% of 2328 ng·h/mL, between about 70% and about 135% of 2328 ng·h/mL, between about 75% and about 130% of 2328 ng·h/mL, between about 80% and about 125% of 2328 ng·h/mL, between about 85% and about 120% of 2328 ng·h/mL, between about 90% and about 115% of 2328 ng·h/mL, between about 95% and about 110% of 2328 ng·h/mL, between about 100% and about 105% of 2328 ng·h/mL, between about 40% and about 60% of 2328 ng·h/mL, between about 40% and about 65% of 2328 ng·h/mL, between about 40% and about 70% of 2328 ng·h/mL, between about 40% and about 75% of 2328 ng·h/mL, between about 40% and about 80% of 2328 ng·h/mL, between about 40% and about 85% of 2328 ng·h/mL, between about 40% and about 90% of 2328 ng·h/mL, between about 40% and about 95% of 2328 ng·h/mL, between about 40% and about 100% of 2328 ng·h/mL, or any range or value therein between.

In some embodiments, the first dose comprises COMPOUND A in an amount of about 0.5 mg, about 1 mg, about 1.5 mg, about 2 mg, about 2.5 mg, about 3 mg, about 3.5 mg, about 4 mg, about 4.5 mg, about 5 mg, about 5.5 mg, about 6.0 mg, about 6.5 mg, about 7 mg, about 7.5 mg, about 8 mg, about 8.5 mg, about 9 mg, about 9.5 mg, about 10 mg, about 10.5 mg, about 11 mg, about 11.5 mg, about 12 mg, about 12.5 mg, about 13 mg, about 13.5 mg, about 14 mg, about 14.5 mg, about 15 mg, about 15.5 mg, about 16 mg, about 16.5 mg, about 17 mg, about 17.5 mg, about 18 mg, about, about 18.5 mg, about 19 mg, about 19.5 mg, or about 20 mg, or any range or value therein between.

In some embodiments, the first dose comprises COMPOUND A in an amount of between about 1 mg and about 15 mg, between about 1.5 mg and about 12.5 mg, between about 2 mg and about 12 mg, between about 2.5 mg and about 11.5 mg, between about 3 mg and about 11 mg, between about 3.5 mg and about 10.5 mg, between about 4 mg and about 10 mg, between about 4.5 mg and about 9.5 mg, between about 5 mg and about 9 mg, between about 5.5 mg and about 8.5 mg, or between about 6 mg and about 8 mg, or any range or value therein between.

The first dose may be administered by any route of introducing or delivering COMPOUND A or a pharmaceutically acceptable salt thereof to a subject to perform its intended function. In some embodiments, administration of the first dose may be carried out by any suitable route, including, but not limited to, orally, intravenously, intramuscularly, intraperitoneally, subcutaneously, and other suitable routes as described herein, or any combination of suitable routes. In some embodiments, the first dose is administered orally. In some embodiments, the first dose is administered orally in the form of a tablet or capsule.

*Maintenance Dose*

In some embodiments, the maintenance dose is administered not less than about 6 hours after the first dose, not less than about 8 hours after the first dose, not less than about 10 hours after the first dose, not less than about 12 hours after the first dose, not less than about 14 hours after the first dose, not less than 16 hours after the first dose, not less than about 18 hours after the first dose, not less than about 20 hours after the first dose, not less than about 22 hours after the first dose, not less than about 24 hours after the first dose, not less than about 30 hours after the first dose, not less than about 36 hours after the first dose, not less than about 42 hours after the first dose, not less than about 48 hours after the first dose, not less than about 54 hours after the first dose, not less than about 60 hours after the first dose, not less than

about 66 hours after the first dose, not less than about 72 hours after the first dose, not less than about 78 hours after the first dose, not less than about 84 hours after the first dose, not less than about 90 hours after the first dose, not less than about 96 hours after the first dose, not less than about 102 hours after the first dose, not less than about 108 hours after the first dose, not less than about 120 hours after the first dose, not less than about 132 hours after the first dose, not less than about 144 hours after the first dose, not less than about 156 hours after the first dose, not less than about 168 hours after the first dose, or not less than about 180 hours after the first dose, or any value therein between.

In some embodiments, the maintenance dose is effective to achieve a mean $C_{max}$ at steady-state of COMPOUND A in the patient of no greater than about 150 ng/mL, no greater than about 145 ng/mL, no greater than about 140 ng/mL, no greater than about 135 ng/mL, no greater than about 130 ng/mL, no greater than about 125 ng/mL, no greater than about 120 ng/mL, no greater than about 115 ng/mL, no greater than about 110 ng/mL, no greater than about, 105 ng/mL, no greater than about 100 ng/mL, no greater than about 95 ng/mL, no greater than about 90 ng/mL, no greater than about 89 ng/mL, no greater than about 88 ng/mL, no greater than about 87 ng/mL, no greater than about 86 ng/mL, no greater than about 85 ng/mL, no greater than about 84 ng/mL, no greater than about 83 ng/mL, no greater than about 82 ng/mL, no greater than about 81 ng/mL, no greater than about 80 ng/mL, no greater than about 79 ng/mL, no greater than about 78 ng/mL, no greater than about 77 ng/mL, no greater than about 76 ng/mL, no greater than about 75 ng/mL, no greater than about 74 ng/mL, no greater than about 73 ng/mL, no greater than about 72 ng/mL, no greater than about 71 ng/mL, no greater than about 70 ng/mL, or any range or value therein.

In some embodiments, the maintenance dose is effective to achieve a mean $C_{max}$ at steady-state of COMPOUND A in the patient of at least about 2 ng/mL, at least about 3 ng/mL, at least about 4 ng/mL, at least about 5 ng/mL, at least about 6 ng/mL, at least about 7 ng/mL, at least about 8 ng/mL, at least about 9 ng/mL, at least about 10 ng/mL, at least about 15 ng/mL, at least about 20 ng/mL, at least about 25 ng/mL, at least about 30 ng/mL, at least about 35 ng/mL, at least about 40 ng/mL, at least about 45 ng/mL, at least about 50 ng/mL, at least about 55 ng/mL, at least about 60 ng/mL, at least about 65 ng/mL, at least about 66 ng/mL, at least about 67 ng/mL, at least about 68 ng/mL, at least about 69 ng/mL, at least about 70 ng/mL, at least about 71 ng/mL, at least about 72 ng/mL, at least about 73 ng/mL, at least about 74 ng/mL, at least about 75 ng/mL, at least about 76 ng/mL, at least about 77 ng/mL, at least about 78 ng/mL, at least about 89 ng/mL, at least about 80 ng/mL, at least about 81 ng/mL, at least about 82 ng/mL, at least about 83 ng/mL, at least about 84 ng/mL, or at least about 85 ng/mL, at least about 90 ng/mL, at least about 95 ng/mL, at least about 100 ng/mL, at least about 105 ng/mL, at least about 110 ng/mL, at least about 115 ng/mL, at least about 120 ng/mL, at least about 125 ng/mL, at least about 130 ng/mL, at least about 135 ng/mL, at least about 140 ng/mL, at least about 145 ng/mL, or at least about 150 ng/mL, or greater, or any range or value therein.

In some embodiments, the at least one daily maintenance dose is effective to achieve a mean $C_{max}$ at steady-state of COMPOUND A in the patient of about 1 ng/mL, about 2 ng/mL, about 3 ng/mL, about 4 ng/mL, about 5 ng/mL, about 6 ng/mL, about 7 ng/mL, about 8 ng/mL, about 9 ng/mL, about 10 ng/mL, about 15 ng/mL, about 20 ng/mL, about 25 ng/mL, about 30 ng/mL, about 35 ng/mL, about 40 ng/mL, about 45 ng/mL, about 50 ng/mL, about 55 ng/mL, about 60 ng/mL, about 61 ng/mL, about 62 ng/mL, about 63 ng/mL, about 64 ng/mL, about 65 ng/mL, about 66 ng/mL, about 67 ng/mL, about 68 ng/mL, about 69 ng/mL, about 70 ng/mL, about 71 ng/mL, about 72 ng/mL, about 73 ng/mL, about 74 ng/mL, about 75 ng/mL, about 76 ng/mL, about 77 ng/mL, about 78 ng/mL, about 79 ng/mL, about 80 ng/mL, about 81 ng/mL, about 82 ng/mL, about 83 ng/mL, about 84 ng/mL, about 85 ng/mL, about 86 ng/mL, about 87 ng/mL, about 88 ng/mL, about 89 ng/mL, about 90 ng/mL, about 95 ng/mL, about 100 ng/mL, about 105 ng/mL, about 110 ng/mL, about 115 ng/mL, about 120 ng/mL, about 125 ng/mL, about 130 ng/mL, about 135 ng/mL, about 140 ng/mL, about 145 ng/mL, or about 150 ng/mL, or greater, or any range or value therein

In some embodiments, the at least one daily maintenance dose is effective to achieve a mean $C_{max}$ at steady-state of COMPOUND A in the patient of at least about 30% of 78 ng/mL, at least about 35% of 78 ng/mL, at least about 40% of 78 ng/mL, at least about 45% of 78 ng/mL, at least about 50% of 78 ng/mL, at least about 55% of 78 ng/mL, at least about 60% of 78 ng/mL, at least about 65% of 78 ng/mL, at least about 70% of 78 ng/mL, at least about 75% of 78 ng/mL, at least about 80% of 78 ng/mL, at least about 85% of 78 ng/mL, at least about 90% of 78 ng/mL, at least about 95% of 78 ng/mL, at least about 100% of 78 ng/mL, at least about 105% of 78 ng/mL, at least about 110% of 78 ng/mL, at least about 115% of 78 ng/mL, at least about 120% of 78 ng/mL, at least about 125% of 78 ng/mL, at least about 130% of 78 ng/mL, at least about 135% of 78 ng/mL, at least about 140% of 78 ng/mL, at least about 145% of 78 ng/mL, or at least about 150% of 78 ng/mL, or any range or value therein.

In some embodiments, the at least one daily maintenance dose is effective to achieve a mean $C_{max}$ at steady-state of COMPOUND A in the patient of no greater than about 150% of 78 ng/mL, no greater than about 145% of 78 ng/mL, no greater than about 140% of 78 ng/mL, no greater than about 135% of 78 ng/mL, no greater than about 130% of 78 ng/mL, no greater than about 125% of 78 ng/mL, no greater than about 120% of 78 ng/mL, no greater than about 115% of 78 ng/mL, no greater than about 110% of 78 ng/mL, no greater than about 105% of 78 ng/mL, no greater than about 100% of 78 ng/mL, no greater than about 95% of 78 ng/mL, no greater than about 90% of 78 ng/mL, no greater than about 85% of 78 ng/mL, no greater than about 80% of 78 ng/mL, no greater than about 75% of 78 ng/mL, no greater than about 70% of 78 ng/mL, no greater than about 65% of 78 ng/mL, no greater than about 60% of 78 ng/mL, no greater than about 55% of 78

ng/mL, no greater than about 50% of 78 ng/mL, no greater than about 45% of 78 ng/mL, no greater than about 40% of 78 ng/mL, or any range or value therein.

In some embodiments, the at least one daily maintenance dose is effective to achieve a mean $C_{max}$ at steady-state of COMPOUND A in the patient of between about 40% and about 165% of 78 ng/mL, between about 45% and about 160% of 78 ng/mL, between about 50% and about 155% of 78 ng/mL, between about 55% and about 150% of 78 ng/mL, between about 60% and about 145% of 78 ng/mL, between about 65% and about 140% of 78 ng/mL, between about 70% and about 135% of 78 ng/mL, between about 75% and about 130% of 78 ng/mL, between about 80% and about 125% of 78 ng/mL, between about 85% and about 120% of 78 ng/mL, between about 90% and about 115% of 78 ng/mL, between about 95% and about 110% of 78 ng/mL, between about 100% and about 105% of 78 ng/mL, between about 40% and about 60% of 78 ng/mL, between about 40% and about 65% of 78 ng/mL, between about 40% and about 70% of 78 ng/mL, between about 40% and about 75% of 78 ng/mL, between about 40% and about 80% of 78 ng/mL, between about 40% and about 85% of 78 ng/mL, between about 40% and about 90% of 78 ng/mL, between about 40% and about 95% of 78 ng/mL, between about 40% and about 100% of 78 ng/mL, between about 40% and about 105% of 78 ng/mL, between about 40% and about 110% of 78 ng/mL, between about 40% and about 115% of 78 ng/mL, between about 40% and about 120% of 78 ng/mL, between about 40% and about 125% of 78 ng/mL, between about 40% and about 130% of 78 ng/mL between about 40% and about 135% of 78 ng/mL, or between about 40% and about 140% of 78 ng/mL, or any range or value therein between.

In some embodiments, the at least one daily maintenance dose is effective to achieve a mean $C_{max}$ at steady-state of COMPOUND A in the patient of about 30% of 78 ng/mL, about 35% of 78 ng/mL, about 40% of 78 ng/mL, about 45% of 78 ng/mL, about 50% of 78 ng/mL, about 55% of 78 ng/mL, about 60% of 78 ng/mL, about 65% of 78 ng/mL, about 70% of 78 ng/mL, about 75% of 78 ng/mL, about 80% of 78 ng/mL, about 85% of 78 ng/mL, about 90% of 78 ng/mL, about 95% of 78 ng/mL, about 100% of 78 ng/mL, about 105% of 78 ng/mL, about 110% of 78 ng/mL, about 115% of 78 ng/mL, about 120% of 78 ng/mL, about 125% of 78 ng/mL, about 130% of 78 ng/mL, about 135% of 78 ng/mL, about 140% of 78 ng/mL, about 145% of 78 ng/mL, or about 150% of 78 ng/mL, or any range or value therein.

In some embodiments, the at least one daily maintenance dose is effective to achieve a mean $AUC_{\tau}$ at steady-state of COMPOUND A in the patient of no greater than about 3000 ng·h/mL, no greater than about 2950 ng·h/mL, no greater than about 2900 ng·h/mL, no greater than about 2850 ng·h/mL, no greater than about 2800 ng·h/mL, no greater than about 2750 ng·h/mL, no greater than about 2700 ng·h/mL, no greater than about 2650 ng·h/mL, no greater than about 2600 ng·h/mL, no greater than about 2550 ng·h/mL, no greater than about 2500 ng·h/mL, no greater than about 2450 ng·h/mL, no greater than about 2400 ng·h/mL, no greater than about 2350 ng·h/mL, no greater than about 2300 ng·h/mL, no greater than about 2250 ng·h/mL, no greater than about 2200 ng·h/mL, no greater than about 2150 ng·h/mL, no greater than about 2100 ng·h/mL, no greater than about 2050 ng·h/mL, no greater than about 2000 ng·h/mL, no greater than about 1950 ng·h/mL, no greater than about 1900 ng·h/mL, no greater than about 1850 ng·h/mL, no greater than about 1800 ng·h/mL, no greater than about 1750 ng·h/mL, no greater than about 1700 ng·h/mL, no greater than about 1650 ng·h/mL, no greater than about 1600 ng·h/mL, no greater than about 1550 ng·h/mL, no greater than about 1500 ng·h/mL, no greater than about 1450 ng·h/mL, no greater than about 1400 ng·h/mL, no greater than about 1350 ng·h/mL, no greater than about 1300 ng·h/mL, no greater than about 1250 ng·h/mL, no greater than about 1200 ng·h/mL, no greater than about 1150 ng·h/mL, no greater than about 1100 ng·h/mL, no greater than about 1050 ng·h/mL, no greater than about 1000 ng·h/mL, no greater than about 950 ng·h/mL, no greater than about 900 ng·h/mL, no greater than about 850 ng·h/mL, no greater than about 800 ng·h/mL, no greater than about 750 ng·h/mL, no greater than about 700 ng·h/mL, no greater than about 650 ng·h/mL, no greater than about 600 ng·h/mL, no greater than about 550 ng·h/mL, no greater than about 500 ng·h/mL, or any range or value therein.

In some embodiments, the at least one daily maintenance dose is effective to achieve a mean $AUC_{\tau}$ at steady-state of COMPOUND A in the patient of at least about 300 ng·h/mL, at least about 350 ng·h/mL, at least about 400 ng·h/mL, at least about 450 ng·h/mL, at least about 500 ng·h/mL, at least about 550 ng·h/mL, at least about 600 ng·h/mL, at least about 650 ng·h/mL, at least about 700 ng·h/mL, at least about 750 ng·h/mL, at least about 800 ng·h/mL, at least about 850 ng·h/mL, at least about 900 ng·h/mL, at least about 950 ng·h/mL, at least about 1000 ng·h/mL, at least about 1050 ng·h/mL, at least about 1100 ng·h/mL, at least about 1150 ng·h/mL, at least about 1200 ng·h/mL, at least about 1250 ng·h/mL, at least about 1300 ng·h/mL, at least about 1350 ng·h/mL, at least about 1400 ng·h/mL, at least about 1450 ng·h/mL, at least about 1500 ng·h/mL, at least about 1550 ng·h/mL, at least about 1600 ng·h/mL, at least about 1650 ng·h/mL, at least about 1700 ng·h/mL, at least about 1750 ng·h/mL, at least about 1800 ng·h/mL, at least about 1850 ng·h/mL, at least about 1900 ng·h/mL, at least about 1950 ng·h/mL, at least about 2000 ng·h/mL, at least about 2050 ng·h/mL, at least about 2100 ng·h/mL, at least about 2150 ng·h/mL, at least about 2200 ng·h/mL, at least about 2250 ng·h/mL, at least about 2300 ng·h/mL, at least about 2350 ng·h/mL, at least about 2400 ng·h/mL, at least about 2450 ng·h/mL, at least about 2500 ng·h/mL, at least about 2550 ng·h/mL, at least about 2600 ng·h/mL, or any range or value therein.

In some embodiments, the at least one daily maintenance dose is effective to achieve a mean $AUC_{\tau}$ at steady-state of COMPOUND A in the patient of between about 300 ng·h/mL and about 3000 ng·h/mL, between about 350 ng·h/mL

and about 2950 ng·h/mL, between about 400 ng·h/mL and about 2900 ng·h/mL, between about 450 ng·h/mL and about 2850 ng·h/mL, between about 500 ng·h/mL and about 2800 ng·h/mL, between about 550 ng·h/mL and about 2750 ng·h/mL, between about 600 ng·h/mL and about 2700 ng·h/mL, between about 650 ng·h/mL and about 2650 ng·h/mL, between about 700 ng·h/mL and about 2600 ng·h/mL, between about 750 ng·h/mL and about 2550 ng·h/mL, between about 800 ng·h/mL and about 2500 ng·h/mL, between about 850 ng·h/mL and about 2450 ng·h/mL, between about 900 ng·h/mL and about 2400 ng·h/mL, between about 950 ng·h/mL and about 2350 ng·h/mL, between about 1000 ng·h/mL and about 2300 ng·h/mL, between about 1050 ng·h/mL and about 2250 ng·h/mL, between about 1100 ng·h/mL and about 2200 ng·h/mL, between about 1150 ng·h/mL and about 2150 ng·h/mL, between about 1200 ng·h/mL and about 2100 ng·h/mL, between about 1250 ng·h/mL and about 2050 ng·h/mL, between about 1300 ng·h/mL and about 2000 ng·h/mL, between about 1350 ng·h/mL and about 1950 ng·h/mL, between about 1400 ng·h/mL and about 1900 ng·h/mL, between about 1450 ng·h/mL and about 1850 ng·h/mL, between about 1500 ng·h/mL and about 1800 ng·h/mL, or any range or value therein between.

In some embodiments, the at least one daily maintenance dose is effective to achieve a mean $AUC_{\tau}$ at steady-state in the patient of between about 40% and about 165% of 1487 ng·h/mL, between about 45% and about 160% of 1487 ng·h/mL, between about 50% and about 155% of 1487 ng·h/mL, between about 55% and about 150% of 1487 ng·h/mL, between about 60% and about 145% of 1487 ng·h/mL, between about 65% and about 140% of 1487 ng·h/mL, between about 70% and about 135% of 1487 ng·h/mL, between about 75% and about 130% of 1487 ng·h/mL, between about 80% and about 125% of 1487 ng·h/mL, between about 85% and about 120% of 1487 ng·h/mL, between about 90% and about 115% of 1487 ng·h/mL, between about 95% and about 110% of 1487 ng·h/mL, between about 100% and about 105% of 1487 ng·h/mL, between about 40% and about 60% of 1487 ng·h/mL, between about 40% and about 65% of 1487 ng·h/mL, between about 40% and about 70% of 1487 ng·h/mL, between about 40% and about 75% of 1487 ng·h/mL, between about 40% and about 80% of 1487 ng·h/mL, between about 40% and about 85% of 1487 ng·h/mL, between about 40% and about 90% of 1487 ng·h/mL, between about 40% and about 95% of 1487 ng·h/mL, between about 40% and about 100% of 1487 ng·h/mL, or any range or value therein between.

In some embodiments, the maintenance dose comprises COMPOUND A in an amount of about 0.1 mg, about 0.2 mg, about 0.3 mg, about 0.4 mg, about 0.5 mg, about 1 mg, about 1.5 mg, about 2 mg, about 2.5 mg, about 3 mg, about 3.5 mg, about 4 mg, about 4.5 mg, about 5 mg, about 5.5 mg, about 6.0 mg, about 6.5 mg, about 7 mg, about 7.5 mg, about 8 mg, about 8.5 mg, about 9 mg, about 9.5 mg, about 10 mg, about 10.5 mg, about 11 mg, about 11.5 mg, about 12 mg, about 12.5 mg, about 13 mg, about 13.5 mg, about 14 mg, about 14.5 mg, or about 15 mg, or any range or value therein between.

In some embodiments, the maintenance dose comprises COMPOUND A in an amount of between about 0.1 mg and about 6 mg, between about 0.2 mg and about 5.5 mg, between about 0.3 mg and about 5 mg, between about 0.4 mg and about 4.5 mg, between about 0.5 mg and about 4 mg, between about 0.6 mg and about 3.9 mg, between about 0.7 mg and about 3.8 mg, between about 0.8 mg and about 3.7 mg, between about 0.9 mg and about 3.6 mg, between about 1 mg and about 3.5 mg, between about 1.1 mg and about 3.4 mg, between about 1.2 mg and about 3.3 mg, between about 1.3 mg and about 3.2 mg, between about 1.4 mg and about 3.1 mg, or between about 1.5 mg and about 3.0 mg, or any range or value therein between.

In some embodiments, the maintenance dose may be administered once per day. In some embodiments, the maintenance dose may be administered twice per day. In some embodiments, the maintenance dose may be administered three times per day. In some embodiments, the maintenance dose may be administered multiple times per day, such as twice per day or three times per day. In some embodiments, the maintenance dose may be administered once per day, once every two days, once every three days, once every four days, once every five days, once every six days, once every seven days, once every eight days, once every nine days, or once every ten days, or in longer intervals. In some embodiments, the maintenance dose may be administered once per week, twice per week, three times per week, four times per week, five times per week, six times per week, seven times per week, eight times per week, nine times per week, ten times per week, or greater. In some embodiments, the maintenance dose may be administered once every 2 hours, once every 4 hours, once every 6 hours, once every 8 hours, once every 10 hours, once every 12 hours, once every 14 hours, once every 16 hours, once every 18 hours, once every 20 hours, once every 24 hours, once every 30 hours, once every 36 hours, once every 40 hours, once every 44 hours, once every 48 hours, once every 54 hours, once every 60 hours, once every 66 hours, once every 72 hours, once every 78 hours, once every 84 hours, once every 96 hours, once every 102 hours, once every 108 hours, once every 120 hours, once every 132 hours, or once every 144 hours, or in longer intervals, or any range or value therein between.

The maintenance dose may be administered by any route of introducing or delivering COMPOUND A or a pharmaceutically acceptable salt thereof to a subject to perform its intended function. In some embodiments, administration of the maintenance dose may be carried out by any suitable route, including, but not limited to, orally, intravenously, intramuscularly, intraperitoneally, subcutaneously, and other suitable routes as described herein, or any combination of suitable routes. In some embodiments, the maintenance dose is administered orally.

The present disclosure is not to be limited in terms of the particular embodiments described in this application, which are intended as single illustrations of individual aspects of the disclosure. All the various embodiments of the present

disclosure will not be described herein.

Unless otherwise defined, all terms (including technical and scientific terms) used herein have the same meaning as commonly understood by one of ordinary skill in the art to which this disclosure belongs. It will be further understood that terms, such as those defined in commonly used dictionaries, should be interpreted as having a meaning that is consistent with their meaning in the context of the present application and relevant art and should not be interpreted in an idealized or overly formal sense unless expressly so defined herein. While not explicitly defined below, such terms should be interpreted according to their common meaning.

As will be understood by one skilled in the art, for any and all purposes, particularly in terms of providing a written description, all ranges disclosed herein also encompass any and all possible subranges and combinations of subranges thereof. Any listed range can be easily recognized as sufficiently describing and enabling the same range being broken down into at least equal halves, thirds, quarters, fifths, tenths, etc. As a non-limiting example, each range discussed herein can be readily broken down into a lower third, middle third and upper third, etc. As will also be understood by one skilled in the art all language such as "up to," "at least," "greater than," "less than," and the like, include the number recited and refer to ranges which can be subsequently broken down into subranges as discussed above. Finally, as will be understood by one skilled in the art, a range includes each individual member. Thus, for example, a group having 1-3 cells refers to groups having 1, 2, or 3 cells. Similarly, a group having 1-5 cells refers to groups having 1, 2, 3, 4, or 5 cells, and so forth.

It is to be understood that the present disclosure is not limited to particular uses, methods, reagents, compounds, compositions or biological systems, which can, of course, vary. It is also to be understood that the terminology used herein is for the purpose of describing particular embodiments only, and is not intended to be limiting.

In addition, where features or aspects of the disclosure are described in terms of Markush groups, those skilled in the art will recognize that the disclosure is also thereby described in terms of any individual member or subgroup of members of the Markush group.

Unless the context indicates otherwise, it is specifically intended that the various features of the disclosure can be used in any combination. Moreover, the disclosure also contemplates that in some embodiments, any feature or combination of features set forth herein can be excluded or omitted. To illustrate, if the specification states that a complex comprises components A, B and C, it is specifically intended that any of A, B or C, or a combination thereof, can be omitted and disclaimed singularly or in any combination.

## EXAMPLES

### Example 1: A Randomized, Double-Blind, Placebo-Controlled, Safety, Tolerability and Pharmacokinetic Study of Escalating Single and Multiple Doses of COMPOUND A in Healthy Subjects

*1.0. Study Design and Plan*

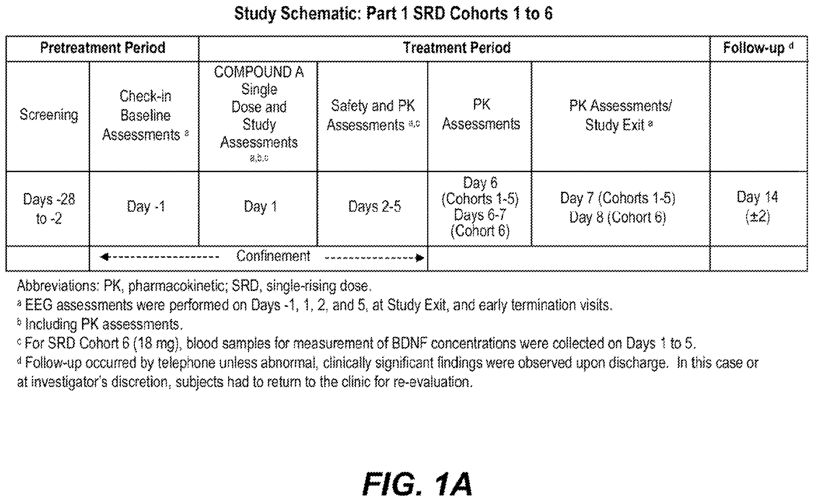

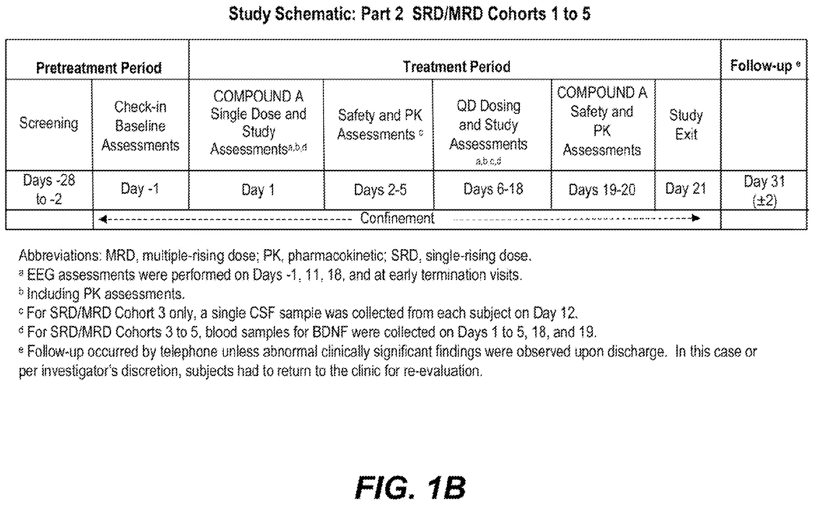

This was a randomized, double-blind, placebo-controlled, combined single-rising dose (SRD) and multiple-rising dose (MRD), Phase 1 clinical study in healthy adult male and female subjects, aged 18 to 55 years, inclusive. The study was designed to assess the safety, tolerability, PK, and PD of COMPOUND A. A total of 88 subjects were enrolled in 6 SRD and 5 SRD/MRD cohorts (n=8 per cohort). The study was composed of 2 parts. A schematic of the study design for Part 1 of the study is provided in FIG. 1A. A schematic of the study design for Part 2 of the study is provided in FIG. 1B.

In Part 1 of the study (SRD cohorts), a single oral tablet dose of COMPOUND A (0.3, 1, 3, 5, 9, or 18 mg) or matching placebo was administered on Day 1 followed by safety, tolerability, PK, and PD assessments. In each SRD cohort of 8 subjects (6 active: 2 placebo), sentinel dosing was used whereby 2 initial subjects (1 active: 1 placebo) were administered study drug to evaluate safety and tolerability before dosing in the remaining subjects (5 active: 1 placebo) after an interval of at least 24 hours. Dosing in the remaining 6 subjects from each cohort was staggered such that only 3 subjects were dosed in 1 day, with the last 3 subjects being dosed after an additional 24-hour interval. The data from each cohort were reviewed sequentially to ensure adequate safety and tolerability before administering the next dose level. Study drug was administered after an overnight fast of at least 10 hours. Subjects were confined in the study unit for 5 days, from Check-in on Day -1 until approximately 96 hours after dosing. Subjects in Cohorts 1 to 5 (0.3-9 mg) returned to the clinic on Days 6 and 7 and subjects in Cohort 6 (18 mg) returned on Days 6, 7 and 8 for additional PK blood sample collections. To allow for a preliminary assessment of the effect of food on the PK of COMPOUND A, subjects in SRD Cohort 3 (3 mg) returned to the clinic to receive study drug under fed conditions (standard high-fat meal, as defined by the Food and Drug Administration ("FDA")), after the safety and tolerability of the higher dose (5 mg) under fasted conditions had been established.

In Part 2 of the study (SRD/MRD cohorts), a single oral tablet dose of COMPOUND A (0.3, 1, 3, 6, or 9 mg) or matching placebo was administered on Day 1 followed by safety, tolerability, PK, and PD assessments. Study drug (at the same dose level) was then administered once daily (QD) for 13 days, on Days 6 through 18. Each cohort of 8 subjects (6 active: 2 placebo) were administered study drug sequentially to ensure adequate safety and tolerability before administering the next dose level. Neither sentinel nor staggered dosing was used for the SRD/MRD cohorts. Subjects were confined in the study unit for 21 days, from Check-in on Day -1 until 72 hours after the last dose of study drug.

For all cohorts in Parts 1 and 2, serial PK blood samples were collected pre- and post-dose on Day 1 to measure plasma concentrations of COMPOUND A and its metabolites. For all SRD/MRD cohorts in Part 2, urine samples were collected pre- and post-dose on Day 1 and post-dose on Day 18 to measure concentrations of COMPOUND A and its metabolites.

In both parts of the study, follow-up occurred approximately 14 days after the last dose of study drug to monitor adverse events (AEs) and concomitant medication use. End of study (study completion date) was based on the final data collection date for the entire study, which was the follow-up phone call/visit.

*2.0. Evaluations*

2.0.1. Data Sets Analyzed

For Part 1 of the study, all 36 subjects who received COMPOUND A were included in the PK set. For Part 2 of the study, all 30 subjects who received COMPOUND A were included in the PK set.

2.0.2. Part 1 (SRD Cohorts): COMPOUND A Plasma PK

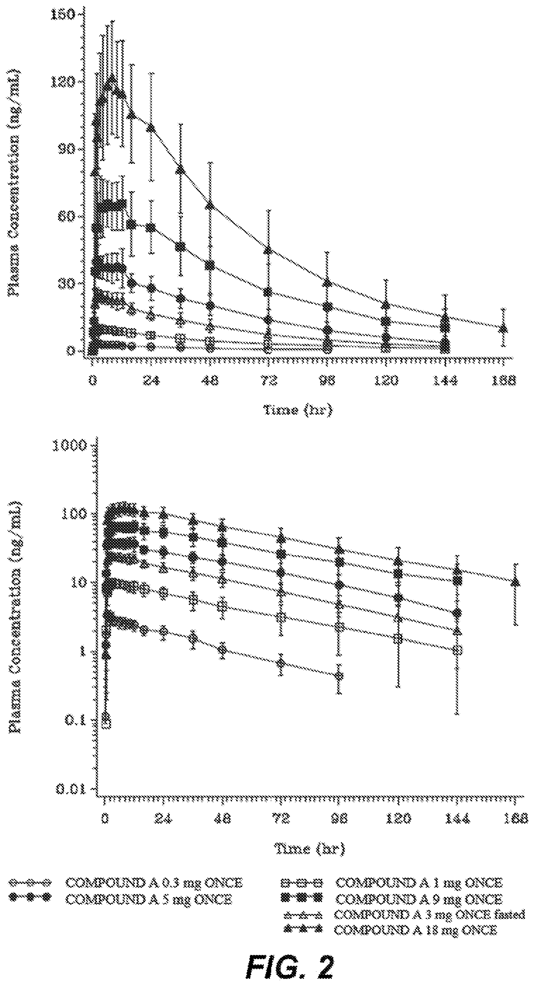

Referring now to FIG. 2, mean plasma concentration-time profiles (linear and semilog scales) of COMPOUND A were determined following single oral administration of COMPOUND A.

Referring to Table 1, after single oral administration of COMPOUND A (0.3, 1, 3, 5, 9, and 18 mg) to healthy subjects, mean plasma COMPOUND A concentrations rapidly increased, with median $t_{max}$ values ranging between 1.25 and 5.5 hours. Thereafter, mean concentrations decreased in a monoexponential manner. The shapes of the concentration-time profiles were similar for each dose level, and the mean COMPOUND A concentration increased in a dose-dependent manner.

Descriptive statistics for plasma PK parameter estimates of COMPOUND A following single oral administration of COMPOUND A are shown in Table 1.

After single oral administration of COMPOUND A, mean plasma COMPOUND A $C_{max}$ values increased in a dose-dependent manner, from 3.63 to 126 ng/mL across the 0.3 to 18 mg dose range. Similarly, mean COMPOUND A $AUC_{\infty}$ values increased in a dose-dependent manner from 148 to 8882 ng·h/mL.

Intersubject variability (%CV) was low for COMPOUND A exposure parameters, ranging from 10% to 20% for $C_{max}$ and from 24% to 45% for $AUC_{\infty}$. Mean terminal $t_{1/2z}$ values were similar for each dose level, ranging from 33.1 to 47.8 hours. In addition, mean CL/F and $V_z$/F values of COMPOUND A were similar across all dose levels, ranging from 1.79 to 2.29 L/h for CL/F and from 95.1 to 133 L for $V_z$/F.

2.0.3. Part 2 (SRD/MRD Cohorts): COMPOUND A Plasma PK

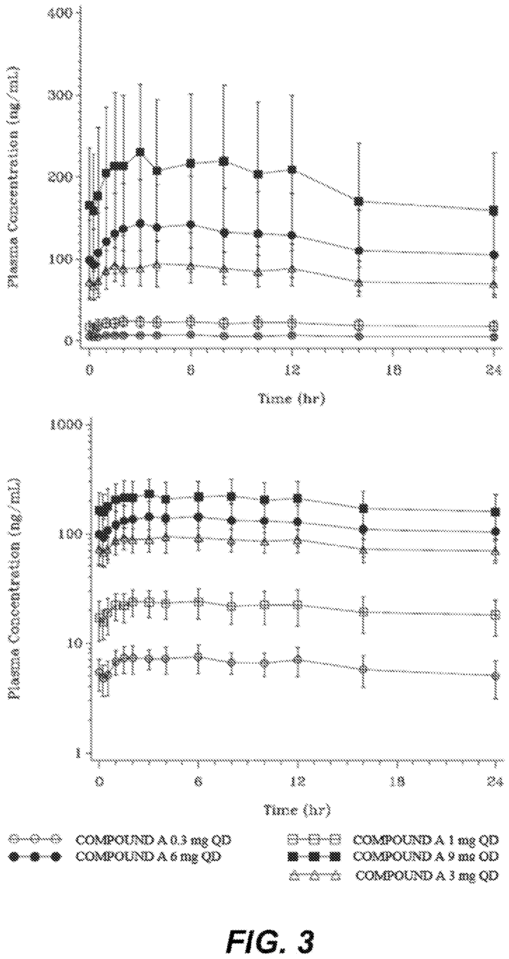

Referring now to FIG. 3, mean plasma concentration-time profiles (linear and semilog scales) of COMPOUND A were determined on Day 18 following oral administration of COMPOUND A QD for 13 days.

After oral administration of COMPOUND A QD (0.3, 1, 3, 6, and 9 mg) to healthy subjects, the COMPOUND A concentration at pre-dose approached the maximum on Day 14 for each dose level, and COMPOUND A exposure appeared to reach steady-state on Day 18. Mean COMPOUND A concentrations increased in a dose-dependent manner. Median $t_{max}$ for COMPOUND A was between 2.5 to 4 hours over the dose range.

Descriptive statistics for plasma PK parameter estimates of COMPOUND A following single and QD oral administration of COMPOUND A are shown in Table 2.

As shown in Table 2, after multiple oral administration of COMPOUND A, mean plasma COMPOUND A $C_{max,ss}$ values on Day 18 increased in a dose-dependent manner, from 7.86 to 243 ng/mL over the 0.3 to 9 mg dose range. Similarly, mean COMPOUND A $AUC_{\tau}$ values on Day 18 increased in a dose-dependent manner from 151 to 4598 ng·h/mL. Intersubject variability (%CV) was low for COMPOUND A exposure parameters, ranging from 27% to 37% for $C_{max,ss}$ and from 23% to 41% for $AUC_{\tau}$. Mean COMPOUND A $C_{av,ss}$ values on Day 18 also increased in a dose-dependent manner from 6.27 to 192 ng/mL.

Table 1. Plasma PK Parameters of COMPOUND A Following Single Oral Administration of COMPOUND A.

| Parameter (unit) | COMPOUND A 0.3 mg (N=6) | COMPOUND A 1 mg (N=6) | COMPOUND A 3 mg (N=6) | COMPOUND A 5 mg (N=6) | COMPOUND A 9 mg (N=6) | COMPOUND A 18 mg (N=6) |
|---|---|---|---|---|---|---|
| | Mean (%CV) | | | | | |
| $t_{max}$ (h) [a] | 1.25 (1.00-1.50) | 2.50 (1.00-3.00) | 1.75 (1.00-10.07) | 2.75 (1.00-12.10) | 5.50 (1.50-12.00) | 5.05 (1.00-10.03) |
| $C_{max}$ (ng/mL) | 3.63 (13) | 10.3 (14) | 27.1 (10) | 45.3 (13) | 69.5 (16) | 126 (20) |
| $AUC_{last}$ (h·ng/mL) | 126 (22) | 565 (35) | 1339 (22) | 2316 (25) | 4493 (34) | 8075 (27) |
| $AUC_{\infty}$ (h·ng/mL) | 148 (26) | 642 (45) | 1458 (24) | 2646 (28) | 5375 (44) | 8882 (33) |
| $t_{1/2z}$ (h) | 33.1 (23) | 41.3 (37) | 37.9 (24) | 42.5 (30) | 47.8 (37) | 44.1 (35) |
| CL/F (L/h) | 2.17 (32) | 1.79 (36) | 2.18 (28) | 2.08 (39) | 1.98 (43) | 2.29 (45) |
| $V_z$/F (L) | 98.8 (18) | 95.1 (12) | 114 (19) | 115 (11) | 120 (15) | 133 (22) |

Abbreviations: %CV, percent coefficient of variation; $AUC_{\infty\backslash}$, area under the plasma concentration-time curve from time 0 to infinity; $AUC_{last}$, area under the plasma concentration-time curve from time 0 to time of the last quantifiable concentration; CL/F, apparent clearance after extravascular administration; $C_{max}$, maximum observed plasma concentration; $t_{1/2z}$, terminal disposition phase half-life; $t_{max}$, time of first occurrence of $C_{max}$; $V_z$/F, apparent volume of distribution during the terminal disposition phase after extravascular administration.
[a] Median (min-max).

**Table 2. Plasma PK Parameters of COMPOUND A Following Single and Multiple QD Oral Administration of COMPOUND A**

| Parameter (unit) | Study Day | COMPOUND A 0.3 mg QD (N=6) | COMPOUND A 1 mg QD (N=6) | COMPOUND A 3 mg QD (N=6) | COMPOUND A 6 mg QD (N=6) | COMPOUND A 9 mg QD (N=6) |
|---|---|---|---|---|---|---|
| | | Mean (%CV) | | | | |
| $t_{max}$ (h) [a] | 1 | 1.25 (1.00-8.13) | 4.00 (1.00-6.00) | 1.50 (1.00-6.00) | 5.00 (3.00-6.08) | 4.51 (1.00-12.12) |
| | 6 | 1.50 (1.00-4.00) | 1.00 (1.00-6.00) | 1.54 (1.00-4.00) | 5.00 (1.00-8.02) | 4.00 (1.00-8.00) |
| | 18 | 2.50 (1.50-6.00) | 4.50 (2.00-12.00) | 4.00 (1.00-6.17) | 3,05 (1.00-10.00) [b] | 3.00 (1.50-8.00) |
| $C_{max}$ (ng/mL) | 1 | 2.93 (22) | 7.82 (12) | 28.7 (24) | 42.0 (24) | 78.4 (27) |
| | 6 | 3.07 (12) | 10.0(17) | 34.7 (28) | 50.2 (22) | 82.0 (30) |
| $C_{max,ss}$ (ng/mL) | 18 | 7.87 (27) | 24.9 (28) | 100 (28) | 148 (37) [b] | 243 (37) |
| $C_{av,ss}$ (ng/mL) | 18 | 6.27 (29) | 20.9 (33) | 81.1 (23) | 122 (41) [b] | 192 (41) |
| $AUC_{last}$ (h*ng/mL) | 1 | 118 (20) | 394 (25) | 1466 (26) | 1955 (49) | 4433 (37) |
| $AUC_{24}$ (h*ng/mL) | 1 | 48 (13) | 138 (11) | 480 (19) | 767 (25) | 1412 (24) |
| | 6 | 51.8 (17) | 162 (15) | 594 (24) | 784 (14) [b] | 1567 (33) |
| $AUC_{\tau}$ (h*ng/mL) | 18 | 151 (29) | 503 (33) | 1947 (23) | 2933 (41) [b] | 4598 (41) |

(continued)

| Parameter (unit) | Study Day | COMPOUND A 0.3 mg QD (N=6) | COMPOUND A 1 mg QD (N=6) | COMPOUND A 3 mg QD (N=6) | COMPOUND A 6 mg QD (N=6) | COMPOUND A 9 mg QD (N=6) |
|---|---|---|---|---|---|---|
| | | Mean (%CV) | | | | |
| $AUC_{\infty}$ (h*ng/mL) | 1 | 133 (24) | 470 (31) | 1874 (33) | 2328 (69) | 5760 (45) |
| $t_{1/2Z}$ (h) | 1 | 37.1 (24) | 42.4 (24) | 52.6 (29) | 36.0 (46) | 49.9 (43) |
| CL/F (L/h) | 1 | 2.38 (26) | 2.35 (37) | 1.81 (44) | 3.25 (39) | 2.17 (84) |
| $V_z$/F (L) | 1 | 121 (11) | 133 (12) | 126 (21) | 145 (16) | 119 (24) |

Abbreviations: %CV, percent coefficient of variation; AUC, area under the concentration-time curve; $AUC_{24}$, area under the plasma concentration-time curve from time 0 to 24 hours; $AUC_{\infty}$, area under the plasma concentration-time curve from time 0 to infinity; $AUC_{last}$, area under the plasma concentration-time curve from time 0 to time of the last quantifiable concentration; $AUC_{\tau}$ area under the plasma concentration-time curve during a dosing interval; CL/F, apparent clearance after extravascular administration; $C_{av,ss}$, average plasma concentration at steady state; $C_{max}$, maximum observed plasma concentration; $C_{max,ss}$, maximum observed plasma concentration during a dosing interval, at steady state; NC, not calculated; PK, pharmacokinetic; QD, once daily; $t_{1/2,}$, terminal disposition phase half-life; $t_{max}$, time of first occurrence of $C_{max}$; $V_z$/F, apparent volume of distribution during the terminal disposition phase after extravascular administration.

[a] Median (min-max).

[b] n=5.

**Example 2: 14-Day Rat Neurotoxicology Study**

*2.1. Materials and Methods*

2.1.1. Formulation

An appropriate amount of methylcellulose (MC) was weighed and mixed with a small amount of warm water for injection. The mixture was then diluted with water for injection to make a 0.5% (w/v) MC solution. The vehicle was used for dosing in the control group. To make a 40 mg/mL suspension of COMPOUND A, an appropriate amount of the test article was weighed and mixed with the vehicle using a planetary centrifugal mixer and the mixture was then diluted with additional vehicle. The 40 mg/mL suspension was used to make 20 and 10 mg/mL suspensions by stepwise dilutions with the vehicle. The dosing suspensions were stored in a refrigerator (actual values: 3.7°C to 6.1°C, acceptable range: 2°C to 8°C) under light-resistant conditions (tight container) and used within 8 days after preparation.

2.1.2. Animals

Sprague-Dawley rats were used because this is a standard rodent strain which has been frequently used in toxicity studies. Additionally, Sprague-Dawley rats were used in previous repeated dose toxicity studies with COMPOUND A.

Males and females in good condition were selected for this study based on examinations within 4 days before the first dosing. Based on their body weights, animals were stratified and allocated randomly to 3 main study groups, each composed of 18 males and 18 females, and 2 satellite groups each composed of 9 males and 9 females for determination of plasma drug and metabolite concentrations. Surplus animals after animal selection were excluded from the study on the day after the animal selection or the day dosing commenced (Day 1).

2.1.3. Animal Husbandry

The animals were individually housed in stainless steel mesh cages. They were allocated in the order of the animal identification numbers to a position on a rack in an animal room. The conditions of the room were as follows: temperature 21.8°C to 23.8°C (acceptable range: 19.0°C to 25.0°C); relative humidity 49.0% to 78.9% (acceptable range: 35.0% to 75.0%); air exchange 6 to 20 times/hour with fresh air; and a 12-hour light/dark cycle (lights on from 7:00 a.m. to 7:00 p.m.). The animals were allowed free access to a pelleted laboratory animal diet (CR-LPF, Oriental Yeast Co., Ltd., γ-irradiated, Lot 170630) and tap water (passed through a 5-μm filter and irradiated by UV-light) except for approximately 16 to 23 hours before scheduled-necropsy, when animals were deprived of food. Contaminants were confirmed not to be present in the diet or water at levels that might have interfered with this study.

2.1.4. Experimental Design

The experimental design is summarized below in Tables 3A and 3B.

2.1.5. Examinations and Methods

The day before the initiation of dosing was designated as Day -1 and the first day of dosing was designated as Day 1. The period from Day 1 to 7 was designated as Week 1.

*2.1.5.1. Toxicokinetics*

Referring to Tables 4A and 4B, the plasma concentrations of COMPOUND A on Days 2, 7, and 14 were determined in each satellite group. Blood samples (approximately 0.25 mL) were drawn without anesthesia from the subclavian vein of 3 animals of each sex in the satellite groups at each sampling point using sodium-heparinized syringes. Sampling points were as follows: pre-dose and at 1, 2, 4, 8, and 24 hours post-dose. In addition, the plasma concentrations of COMPOUND A before scheduled necropsy were determined in each main study group. The plasma samples (≥0.08 mL) in polypropylene tubes were frozen and stored in a freezer (actual values: -87.0°C to -83.5°C) until shipment with parallel quality control (QC) samples prepared by test site 2 and stored under the same conditions as study plasma samples in the test facility. The samples, including the parallel QC samples, were packed with dry ice and shipped to test site 2. The samples were stored frozen at -85.8°C to - 75.5°C until assay. The plasma concentrations of COMPOUND A and its metabolites, were determined by liquid chromatography/tandem mass spectrometry (LC/MS/MS) and the TK parameters ($t_{max}$, $C_{max}$, and $AUC_{24}$ value) on Days 2, 7, and 14 were calculated for the satellite groups at test site 2.

*2.1.5.2. Clinical Signs*

All animals were observed for survival and clinical signs 4 times (at pre-dose and approximately 1, 2, and 4 hours post-dose) on Day 1 and 3 times daily (at pre-dose and approximately 1 and 4 hours post-dose) from Day 2 to 14 during the dosing period and once on the day of necropsy.

The behavior of the high-dose main study and TK satellite groups was video-recorded continuously throughout the dosing period (until the morning on the day of necropsy) under non-GLP conditions. The video-recorded images were kept on a hard drive and were archived as study data. The videos from 3 females at 200 mg/kg/day with test article-related histopathological changes were examined for the presence of convulsions during the dosing period.

*2.1.5.3. Body Weights*

Each animal was weighed pre-dose using electronic balances (PB3002-S; Mettler-Toledo K.K. and UX4200H; Shimadzu Corporation) on Days 1, 2, 4, 7, 11, and 14 during the dosing period. Body weights were also measured for all animals on the day of necropsy on Days 3, 8, and 15, and these terminal body weight values were also used for calculation of the amount of anesthesia.

*2.1.5.4. Necropsy*

The animals were fasted for approximately 16 to 23 hours and weighed for calculation of the amount of anesthesia. The animals were anesthetized by intraperitoneal injection of sodium thiopental (Ravonal®, Mitsubishi Tanabe Pharma Corporation) approximately 0.5 to 2 hours after analgesic treatment by intramuscular injection of buprenorphine (0.01 mg/kg). Buprenorphine (Lepetan injection® 0.2 mg, Otsuka Pharmaceutical Factory, Co., Ltd.) was used after 5-fold dilution (0.04 mg/mL) with physiological saline (Japanese pharmacopoeia, Otsuka Pharmaceutical Factory, Inc.).

A cannula was inserted from the left ventricle toward the aortic root, and the whole body was fixed by perfusion with 4% paraformaldehyde fixative after flushing blood with 0.1 mol/L phosphate buffer using a peristaltic pump (perfusion fixation). For animals in the TK satellite groups, necropsy was conducted for all animals after blood sampling on Days 3, 8, and 15.

The animals were weighed for calculation of the amount of anesthesia before they were euthanized by exsanguination under sodium thiopental anesthesia (Ravonal®, Mitsubishi Tanabe Pharma Corporation). The brain

was examined macroscopically on Days 3, 8, and 15 at the time of necropsy.

*2.1.5.5. Histopathological Examination*

After perfusion fixation, the brain except for olfactory bulbs was collected and fixed by immersion in 10 vol% phosphate-buffer formalin. The olfactory bulb was fixed by immersion in 10 vol% phosphate-buffer formalin with the cranium and was removed after fixation without decalcification. Brains obtained from all animals in the control group, males at 100 mg/kg/day and females at 200 mg/kg/day on Days 3, 8, and 15, and females at 100 mg/kg/day on Day 15 were trimmed coronally at 7 levels in accordance with the STP position paper (Bolon B, Garman RH, Pardo ID, Jensen K, Sills RC, Roulois A, et al. STP position paper: Recommended practices for sampling and processing the nervous system (brain, spinal cord, nerve, and eye) during nonclinical general toxicity studies. Toxicol Pathol. 2013; 41: 1028-48.). These trimmed tissues were embedded in paraffin, sectioned and processed by standard methods to prepare hematoxylin and eosin (HE)-stained sections and for evaluation using with Fluoro-Jade C (Fluoro-Jade C Ready-to-Dilute Staining Kit, Batch No. BA01-30-100FJTK261017, Biosensis; thickness: 4 to 5 μm). The HE-stained specimens were shipped to test site 1 and examined for histopathology. After the examination at test site 1, the specimens were returned to the test facility. For Fluoro-Jade staining, a negative control section without penetration by Fluoro-Jade C and a positive control section (kainic acid neurotoxicity model) were used. The Fluoro-Jade stained specimens were examined using a fluorescence microscope at the test facility. None of the brains from males at 50 mg/kg/day (Days 3, 8, and 15), or females at 100 mg/kg/day (Days 3 and 8) were examined histopathologically because no abnormalities were noted in any males at 100 mg/kg/day or in females at 200 mg/kg/day on Day 3 or 8.

For animals in the TK satellite group, the brain and olfactory bulb were collected at necropsy and fixed by immersion in 10 vol% phosphate-buffer formalin. The olfactory bulb was fixed by immersion in 10 vol% phosphate-buffer formalin with the cranium but was not examined microscopically

*2.1. Conclusion*

The purpose of the present study was to assess the potential neurotoxicity of COMPOUND A in rats treated orally with the compound for 2, 7, or 14 days and euthanized the days after the last dose administration. TK analysis also was conducted.

TK analysis indicated $C_{max}$ and $AUC_{24}$ values similar to those seen in previous rat toxicity studies at doses similar to those used in the current study.

All rats survived to their scheduled euthanasia and there were no test article-related abnormalities in clinical signs, body weights, or macroscopic parameters of the brain.

Test article-related changes were noted in the cerebellum of 3 females at 200 mg/kg/day on Day 15. The Purkinje cell necrosis in 1 female was visible by HE staining and the necrotic cells also were positive for Fluoro-Jade staining. Two other females also showed Fluoro-Jade staining of Purkinje cell dendrites that were not visible using HE. Fluoro-Jade staining is thought to be a marker of neural degeneration and the labelling of dendrites, axons and cell bodies in the present study suggest Purkinje cell necrosis or degeneration (Schmued LC, Albertson C, Slikker W Jr. Fluoro-Jade: a novel fluorochrome for the sensitive and reliable histochemical localization of neuronal degeneration. Brain Res. 1997; 751: 37-46.).

Glutamate is the primary excitatory neurotransmitter in the brain, including the cerebellum. Purkinje cells receive 2 types of excitatory input from outside the cerebellum, one directly from climbing fibers and the other indirectly from parallel fibers. Each Purkinje cell receives excitatory inputs tens of thousands parallel fibers and specialized glial cells, referred to as Bergmann cells, also provide excitatory input to Purkinje cells and modulate levels of glutamate in synapses with Purkinje cells (Dzubay JA, Jahr CE. The concentration of synaptically released glutamate outside of the climbing fiber-Purkinje cell synaptic cleft. J. Neurosci. 1999; 19; 5265-74.).

AMPA receptors are especially important for fast synaptic transmission of neurons in the cerebellar cortex, and Purkinje cell degeneration has been reported to occur as a consequence of excessive glutamate-induced AMPA receptor activation secondary to a number of insults (Mansouri B, Henne WM, Omman SK, Bliss R, Attridge J, Finckbone V, et al. Involvement of calpain in AMPA-induced toxicity to rat cerebellar Purkinje neurons. Eur. J. Pharmacol. 2007; 557: 106-14.; Barenberg P, Strahlendorf H, Strahlendorf J. Hypoxia induces an excitotoxic-type of dark cell degeneration in cerebellar Purkinje neurons. Neurosci. Res. 2001; 40: 245-54.; Garthwaite G, Garthwaite J. AMPA neurotoxicity in rat cerebellar and hippocampal slices: histological evidence for three mechanisms. Eur. J. Neurosci. 1991; 3: 715-28.). As COMPOUND A is an AMPA receptor potentiator, the changes seen in Purkinje cells in the current study may reflect excessive glutamate-induced excitation as a result of AMPA receptor potentiation by COMPOUND A.

No clinical signs were recorded for any animal at scheduled observation times. When the video recordings of the 3 females at 200 mg/kg/day with cerebellar findings were examined, however, 2 of them showed tonic convulsions. One animal had a single convulsion on Day 3 of dosing. The other female that convulsed had multiple tonic convulsions or

intermittent convulsions (mainly forelimbs) beginning immediately after treatment on Day 6 and continuing for 18 hours post-dose. These 2 animals had Fluoro-Jade positive staining of Purkinje cells which also was visible in HE stained sections in 1 animal. However, 1 other female with histopathologic findings in the brain was not observed to convulse. Evaluation of exposures to COMPOUND A at the time of sacrifice (Day 15) indicated a COMPOUND A plasma level of 935 ng/mL in the animal (animal with lesions visible with both HE and Fluoro-Jade), and exposures of 182 ng/mL and 138 ng/mL in the remaining 2 females with lesions visible with Fluoro-Jade only. Plasma COMPOUND A exposures at the time of sacrifice in the 3 remaining females at 200 mg/kg/day that were not observed to convulse and that showed no brain histopathology were 192, 968, and 114 ng/mL. Although it is possible that tonic convulsions were associated with Purkinje cell necrosis, the absence of a convulsion in 1 animal with Purkinje cell damage argues against this. It is notable, however, that the animal with the highest plasma drug level at the time of sacrifice, and with the highest frequency of convulsions also had the highest degree of neuropathology.

In conclusion, tonic convulsions in 2 rats and necrosis and/or Fluoro-Jade positive staining of Purkinje cells (dendrites, cell bodies, or axons) in 3 rats were observed in females dosed with COMPOUND A for 14 days at 200 mg/kg/day. Therefore, it was concluded that the no-observed-adverse-effect level (NOAEL) was 100 mg/kg/day for both males and females with mean $C_{max}$ values of 1290 and 1670 ng/mL for COMPOUND A, and mean $AUC_{24}$ values of 17,000 and 17,800 ng·hr/mL for COMPOUND A for males and females, respectively, on Day 14.

**Table 3A. Study Design for 14-Day Rat Neurotoxicology Study (Main Study Group)**

| Test article | Dose | Dose volume | Concentration | No. of animals (Animal No.) | |
|---|---|---|---|---|---|
| | (mg/kg/day) | (mL/kg/day) | (mg/mL) | Male | Female |
| Control | 0 | 5 | 0 | 6 (10101-06)*1<br>6 (10107-12)*2<br>6 (10113-18)*3 | 6 (50101-06)*1<br>6 (50107-12)*2<br>6 (50113-18)*3 |
| COMPOUND A | 50 | 5 | 10 | 6 (10201-06)*1<br>6 (10207-12)*2<br>6 (10213-18)*3 | NA |
| COMPOUND A | 100 | 5 | 20 | 6 (10301-06)*1<br>6 (10307-12)*2<br>6 (10313-18)*3 | 6 (50201-06)*1<br>6 (50207-12)*2<br>6 (50213-18)*3 |
| COMPOUND A | 200 | 5 | 40 | NA | 6 (50301-06)*1<br>6 (50307-12)*2<br>6 (50313-18)*3 |

Control: 0.5 (w/v) MC solution; NA: not available
*1: Scheduled-sacrifice on Day 3
*2: Scheduled-sacrifice on Day 8
*3: Scheduled-sacrifice on Day 15

**Table 3B. Study Design for 14-Day Rat Neurotoxicology Study (TK Satellite Group)**

| Test article | Dose | Dose volume | Concentration | No. of animals (Animal No.) | |
|---|---|---|---|---|---|
| | (mg/kg/day) | (mL/kg/day) | (mg/mL) | Male | Female |
| COMPOUND A | 50 | 5 | 10 | 9 (20101-09) | NA |
| COMPOUND A | 100 | 5 | 20 | 9 (20201-09) | 9 (60101-09) |
| COMPOUND A | 200 | 5 | 40 | NA | 9 (60201-09) |

NA: Not available

**Table 4A. Listing of Animal Numbers for Blood Collection (Main Study Group)**

| Dose (mg/kg/day) | No. of animals (Animal No.) | | Blood collection |
|---|---|---|---|
| | Male | Female | |
| 0 | 2 (10101-02) | 2 (50101-02) | Day 3 |
| | 2 (10107-08) | 2 (50107-08) | Day 8 |
| | 2 (10113-14) | 2 (50113-14) | Day 15 |
| 50 | 6 (10201-06) | NA | Day 3 |
| | 6 (10207-12) | NA | Day 8 |
| | 6 (10213-18) | NA | Day 15 |
| 100 | 6 (10301-06) | 6 (50201-06) | Day 3 |
| | 6 (10307-12) | 6 (50207-12) | Day 8 |
| | 6 (10313-18) | 6 (50213-18) | Day 15 |
| 200 | NA | 6 (50301-06) | Day 3 |
| | NA | 6 (50307-12) | Day 8 |
| | NA | 6 (50313-18) | Day 15 |
| NA: Not available | | | |

**Table 4B. Listing of Animal Numbers for Blood Collection (TK Satellite Group)**

| Dose (mg/kg/day) | No. of animals (Animal No.) | | Blood collection |
|---|---|---|---|
| | Male | Female | |
| 50 | 3 (20101-03) | NA | At the 2$^{nd}$ dosing |
| | 3 (20104-06) | NA | At the 7$^{th}$ dosing |
| | 3 (20107-09) | NA | At the 14$^{th}$ dosing |
| 100 | 3 (20201-03) | 3 (60101-03) | At the 2$^{nd}$ dosing |
| | 3 (20204-06) | 3 (60104-06) | At the 7$^{th}$ dosing |
| | 3 (20207-09) | 3 (60107-09) | At the 14$^{th}$ dosing |
| 200 | NA | 3 (60201-03) | At the 2$^{nd}$ dosing |
| | NA | 3 (60204-06) | At the 7$^{th}$ dosing |
| | NA | 3 (60207-09) | At the 14$^{th}$ dosing |
| NA: Not available | | | |

**Table 5. Summary of Toxicokinetic Parameters**

| | $C_{max}$ (ng/mL) | | | $AUC_{24}$ (ng hr/mL) | | |
|---|---|---|---|---|---|---|
| Dose (mg/kg/day) | 50 | 100 | 200 | 50 | 100 | 200 |
| **COMPOUND A** | | | | | | |
| Day 2 | 836/NA | 1200/1640 | NA/1630 | 9480/NA | 15,000/20,500 | NA/18,000 |
| Day 7 | 841/NA | 917/1270 | NA/2340 | 10,700/NA | 13,000/14,900 | NA/31,800 |
| Day 14 | 728/NA | 1290/1670 | NA/2210 | 8750/NA | 17,000/17,800 | NA/27,600 |

**Example 3: 13-Week NHP GLP Toxicity Study**

*3.1. Materials and Methods*

The appropriate volume of reverse osmosis water was added into a suitable sized container and heated between 70°C and 90°C. An appropriate amount of MC (METOLOSE SM-100, Shin-Etsu Chemical Co Ltd) was weighed and transferred (while stirring) to the container with heated reverse osmosis water. The solution was cooled down until a clear solution was obtained (an ice bath was used). The solution was stored refrigerated (set to 2-8°C) until used. Unused reference item/vehicle was discarded when not needed anymore. To make a 0.2 w/v% suspension of COMPOUND A, an

appropriate amount of the test item was weighed. A small volume of vehicle was added to slowly wet the test item. The appropriate volume of vehicle was added to reach the final volume and the formulation was homogenized using a homogenizer until a uniform suspension was achieved and stirred for 30 to 59 minutes. The 0.2 w/v% suspension was then diluted with the 0.5 w/v% MC in reverse osmosis water solution to make 0.12, 0.06 and 0.006 w/v% suspensions. However, on October 1, 2015, the 1.2 mg/mL formulation was prepared by dissolving the appropriate amount of COMPOUND A in the vehicle, instead of diluting the 2 mg/mL formulation to reach the appropriate concentration. This deviation had no impact on the study since the appropriate concentration was prepared.

3.1.1. Test System

The test system for NHP GLP toxicity studies is summarized in Table 6.

**Table 6. Test system for NHP GLP Toxicity Studies**

| | |
|---|---|
| Species: | Monkey (*Macaca fascicularis)* |
| Strain: | Cynomolgus |
| Original source: | Guangxi Guidong Quadrumana Development Laboratory Co., Ltd., China<br>Guangxi Cangwu County Two Crest (Region Wood Section an Inside) |
| Total number of animals on study: | 19 males and 19 females (including 3 spare animals/sex) |
| Age: | 3.6 years and 5.2 years at the onset of dosing |
| Body weight: | 3.3 to 6.6 kg for males and 2.9 to 4.4 kg for females at the onset of dosing |

3.1.2. Housing

Due to the nature of the study, which did not permit group housing (telemetry data acquisition), animals were housed individually in stainless steel monkey cages equipped with an automatic watering system. Primary enclosures were as specified in the current guide to the Care and Use of Experimental Animals published by the Canadian Council on Animal Care and the Guide for the Care and Use of Laboratory Animals published by the US National Institutes of Health (National Research Council, 2011). The room in which the animals were kept during the treatment period was documented in the study records. Following assignment to dose group, all cages were clearly labeled with a color-coded cage card indicating at least the following: study number and animal number (including sex and group number). For telemetry data acquisition, one receiver for telemetry signals was installed in the cage for each animal. Cages were also equipped with day/night vision cameras for continuous behavioral video monitoring.

3.1.3. Room Environment

The animal room environment was set to maintain a temperature of 21°C ± 3°C (actual range was 17.30°C to 22.64°C); a relative humidity of 50% ± 20% (actual range was 26.84% to 93.76%); a light dark cycle of 12 hours light/12 hours dark (lights on at 7:00 a.m., lights off at 7:00 p.m.), except during Study Plan-designated procedures; and 10-15 air changes per hour. Temperature and relative humidity were monitored continuously. No cage exchange was performed during telemetry data acquisition. A cage exchange was conducted once approximately every 2 weeks before and after telemetry data acquisition. Animal enrichment during this time included a tuned radio and the provision of toys, treats and/or fresh or frozen fruits/vegetables.

3.1.4. Diet/Water

A standard certified commercial chow (Harlan Teklad Certified Hi-Fiber Primate Diet #7195C) was provided to the animals twice daily, except during designated procedures. Treats (fruity gems, supreme mini-treats, prima-treats, yogurt drops, fruity crunchies or fruity bites) or fresh or frozen fruits/vegetables (pear, banana, cucumber, honey melon, cantaloupe, watermelon or celery) were provided as part of the animal enrichment program. The provision of the treats or fresh fruits/vegetables was approved by the Study Director prior to being given.

3.1.5. Acclimation

An acclimation period of 2 to 11 days was allowed between transfer of the animals to the study and the surgery to accustom the animals to the laboratory environment. A recovery period of 20 to 35 days was allowed between surgery and

Phase 1. As per study plan, the recovery period between surgery and dosing was to be at least 3 weeks. This deviation had no impact on the study since all animals recovered well after the surgery and were in good health at the start of dosing.

Animals were acclimated to the gavage procedure on 3 occasions prior to Phase 1.

3.1.6. Preparation of the Test System

Thirty-seven animals (18 males and 19 females including three spare animals/sex) were surgically cannulated with a catheter (LABPC-01 or LABPFC-01, SAI Infusion Technologies) inserted in the femoral vein for blood collection and veterinary care in case of seizures and instrumented for EMG and EEG monitoring (EMG, C4-O2 and Cz-Oz) via the implantation of telemetry transmitters (Data Science International, Model: D70-EEE).

The animals were fasted overnight prior to the surgical procedures. The animals were sedated using a mixture of Ketamine hydrochloride (9.09 mg/kg intramuscularly ("IM")) and Acepromazine (0.09 mg/kg IM) followed by intubation. Lidocaine spray (10% w/w) was administered onto the glottis prior to intubation. An ophthalmic ointment was applied to both eyes to prevent drying of the cornea prior to and after anesthesia. If needed, the animal was anesthetized with isoflurane using a mask prior to intubation. Anesthesia was maintained with isoflurane by inhalation with an oxygen flow at approximately 200 mL/kg/min or as needed. The surgical sites were shaved and were aseptically prepared using chlorhexidine gluconate 4% and 70% isopropyl alcohol.

Animals were placed on a heating pad set to maintain the animal's body temperature at approximately 37°C. The animals were mechanically ventilated at a rate of 8-20 breaths/minute with inspiratory pressure of 18-25 $cmH_2O$. Hemoglobin oxygen saturation ($SpO_2$) and heart rate were regularly monitored to ensure proper ventilation of the animals but are not reported. Intravenous fluid therapy was given throughout the anesthesia using Sterile Lactated Ringer's solution at a rate of 10 mL/kg/hr or as required.

Prophylactic antibiotics (Pencillin G Procaine 300,000 IU/mL) were given IM, as per appropriate SOP, twice daily starting at least 30 minutes before surgery and then twice a day for 2 days post-surgery. Enrofloxacin (Baytril 50 mg/mL) was also administered intramuscularly, as per appropriate SOP, once daily as a prophylactic antibiotic starting at least 15 minutes before surgery and for 5 days following surgery. Analgesia was attained via a transdermal Fentanyl patch (approximately 12.5 μg/hr for animal bodyweights less than 5 kg, or 25 μg/hr for animal bodyweights from 5 to 10 kg) placed on the animal the day prior to surgery and removed 2 days post-surgery. A local anesthetic (50:50, Marcaine® 0.25% and Lidocaine 20 mg/ml) was injected subcutaneously in 6-10 sites (maximum of 0.1 mL/site, maximum total volume of 0.8 mL) distributed over the cranial, neck and abdominal surgical sites during the surgery.

The implantation of the transmitter and subcutaneous leads was performed as follows. A sterile transmitter (Data Science International, Model: D70-EEE was inserted between the internal abdominal oblique muscle and the aponeurosis of the transverses abdominis (location was recorded in the raw data). An incision in the nape of the neck was made. The EEG leads were tunneled from the abdominal cavity (where the transmitter was positioned) to the neck incision. The EEG leads were placed directly on the cranium to monitor two standard bipolar derivations (C4-O2 and Cz-Oz). A linear groove was made in cranial cortical bone to secure the metal part of the electrodes. The EEG leads were secured to the cranium with surgical glue (Vetbond) close to the implantation site at the level of the distal part of the silicone insulate and the metal portion of the leads was secured with acrylic. A loop was prepared with the distal segment of the electrodes, and the loop was secured with non-absorbable sutures in neck muscle tissues. The EMG electrodes were positioned parallel to the longitudinal axis of the neck muscles with a distance of at least 20 mm between the two electrodes.

A small incision was made to the left groin region, and the femoral vein was isolated. A small phlebotomy was made in the vein and a medical grade catheter was inserted. The tip of the catheter was fed through the vein until placed in the inferior vena cava at approximately the level of the kidneys. The catheter was secured in place with appropriate suture material and exteriorized at the nape of the neck through a subcutaneous tunnel from the inguinal area to the dorso-cervical area.

All surgical sites were rinsed with warm sterile saline and closed with absorbable sutures in anatomical planes. Cefazolin was administered locally in the surgical sites on the head (approximately 0.4 mL, concentration of 80 mg/mL). The animals were dressed in clean jackets.

The catheter patency was maintained by a continuous infusion of 0.9% Sodium Chloride for Injection USP (saline) at a rate of 4.0 mL/hour. A catheter patency check was performed at least daily. Approximately 0.5 mL of liquid was withdrawn from the catheter and the catheter was flushed with approximately 4 mL of 0.9% Sodium Chloride for Injection USP (saline). As the infusion system was only used for blood collection and bolus administration of diazepam in case of seizures, no infusion dosing accountability test was required.

3.1.7. Experimental Design

After suitable acclimation to the dosing procedure, the reference item (0.5% methylcellulose (MC) in reverse osmosis water) was administered once by oral gavage during Phase 1 of the study. The objective of Phase 1 was to confirm

the EEG morphologies in all animals before and after dosing procedures. The traces generated during Phase 1 were used for interpretation of Phase 2 data. Beginning 1 day later, the reference item or COMPOUND A was administered once daily by oral gavage for 90 days during Phase 2 as shown in Table 7.

3.1.8. Examinations and Methods

*3.1.8.1. Mortality*

Mortality checks were recorded concomitantly with the cage-side observations (see below) during all phases of the study.

*3.1.8.2. Clinical Observations*

Clinical signs (ill health, behavioral changes, etc.) were recorded on surviving animals twice daily during all phases of study and monitored continuously for at least 16 hours after the 1st, 3rd, 7th, 14th and 28th dose administration in Phase 2, using digital cameras (to minimize stress for the animals) or using direct observation when needed or during dose administration. Additional clinical observations were performed when deemed necessary.

A detailed clinical examination was performed on each animal prior to treatment group assignment and weekly thereafter, including up to one day prior to initiation of treatment and on the day of necropsy (prior to necropsy).

*3.1.8.3. Medical Treatments*

Animals were examined by the veterinary staff as warranted by clinical signs or other changes. All veterinary examinations and recommended therapeutic treatments were documented in the study records and are kept in the study file. Initiation of medical treatments, as recommended by a Veterinarian, began only following authorization (with the exception of pre-approved treatments) by the Study Director (in consultation with the Sponsor, when possible). Whenever possible, the Sponsor was consulted prior to administration of systemic therapeutic drugs *(i.e.,* other than topical or emergency diazepam administration). Administration of these medical treatments had no impact on the study since they were given for Group 1 animals (control), were administered topically or were not considered to have any interaction with the test item.

*3.1.8.4. Body Weights*

Body weights were recorded for all animals prior to treatment group assignment, at least weekly prior to initiation of dosing, and twice weekly thereafter. On the day of necropsy, an overnight fasted body weight was recorded for the calculation of relative organ weight.

*3.1.8.5. Food Appetence*

Beginning at least one week prior to the start of treatment and continuing throughout the study, a qualitative food appetence evaluation was performed once daily (except on days of food deprivation) as part of the cage-side clinical observations and recorded. Food appetence was evaluated based on the number of cookies left in the cage following the daily feeding period.

*3.1.8.6. Clinical Pathology*

Clinical pathology evaluations (hematology, clinical chemistry, coagulation and urinalysis parameters) were performed on all animals twice prior to Phase 1 and on all animals during the 4th week and the 13th week of dosing in Phase 2. Blood samples were collected from the femoral vein catheter or from the femoral vein by venipuncture and urine was collected overnight (approximately 14.5 to 17 hours) at room temperature. Food was removed overnight prior to blood collections (required for clinical chemistry evaluations), while food and water were removed overnight prior to urine collection. Various additional clinical pathology parameters, not mandated by the Study Plan, were automatically measured and recorded by the instrumentation, but are not reported. Repeat clinical pathology collections (for hematology and/or coagulation) when needed, were performed with or without food and/or water deprivation.

*3.1.8.7. Ophthalmology*

Direct, indirect (funduscopic) and biomicroscopic (slit lamp) examinations were performed on all animals once

prior to Phase 1 and on all animals once in Phase 2 towards the end of the dosing period (13th week of dosing). All examinations were conducted by a Board-certified veterinary ophthalmologist. Animals were anesthetized with an intramuscular injection of a combination of ketamine hydrochloride and acepromazine and mydriatic solution (Tropicamide 1%) was applied to both eyes as per standard operating procedures.

*3.1.8.8. Electrocardiogram*

Electrocardiograms (ECGs; limb leads I, II and III, and augmented leads aVR, aVL and aVF) were obtained from all surviving animals once prior to Phase 1, and from all surviving animals once in Phase 2 towards the end of the dosing period (13th week of dosing). ECGs were recorded 3-5 hours following the dosing of each animal.

Qualitative and quantitative assessments of the ECG tracings were performed, and the presence of gross changes indicative of cardiac electrical dysfunction and the potential presence of abnormalities involving heart rate (lead II), sinus and atrioventricular rhythm, or conductivity was determined. Heart rate, PR interval, QRS duration, QT, and QTc (QTcB) interval measurements were tabulated and are incorporated into the study report. Electrocardiograms were evaluated by a board-certified veterinary cardiologist.

*3.1.8.9. Electroencephalogram and Electromyogram Monitoring by Telemetry*

EEGs and EMGs were obtained from the animals using telemetry transmitter leads inserted subcutaneously using bipolar derivations (EMG, C4-O2 and Cz-Oz).

To ensure suitability of the animals, individual animal EEGs and EMGs were recorded at least once prior to Phase 1 during the pre-treatment for a period of approximately 24 hours. These recordings were examined for abnormalities and are retained in the study file but are not included in the study report. Video monitoring was recorded concomitantly but is not reported.

EEG and EMG monitoring was recorded continuously (except during study designated procedures or other procedures such as data backup) from at least 72 hours prior to the first dose of Phase 1 to at least 24 hours after the 28th dose of Phase 2. EEG and EMG monitoring was also recorded continuously (except during study designated procedures or other procedures such as data backup) for a period of at least 36 hours (from at least 12 hours prior to dosing until at least 24 hours post-dosing) once in the 8th week of dosing and once towards the end of the dosing period (13th week of dosing) in Phase 2.

Using the NeuroScore software (Data Science International), EEGs were analyzed from at least 24 hours prior to the first dose of Phase 1 to at least 24 hours after the 28th dose in Phase 2, and for a period of at least 36 hours (from at least 12 hours prior to dosing until at least 24 hours post-dosing) once in the 8th week of dosing and once towards the end of the dosing period (13$^{th}$ week of dosing) in Phase 2. EEGs were evaluated by the Neurologist when abnormal clinical signs (e.g., tremors, ataxia, emesis, muscle twitches, yawning and/or convulsions) were observed in sequences of EEG identified by the seizure detection module of the NeuroScore Software. EMGs were used to aid in the interpretation of the EEG data.

EEGs were reviewed by the Neurologist at the following timepoints: 1-1.5, 2.5-5.5, and 7-7.5 hours following the 1st, 3rd, 7th, 14th and 28th dose administration in Phase 2. The observance of abnormal behaviors/convulsions in individual animals by laboratory personnel also triggered an EEG review for approximately the same time period during which the abnormal behavior was witnessed.

The duration of each seizure was recorded and reported. Prodromal EEG and clinical signs were identified and are discussed in relation to seizure onset. The onset of paroxysmal activity, onset of generalized seizure and end of spike train (or end of generalized seizure) were also recorded and reported. EMGs were used as a complement for interpretation of EEGs. A signed contributing report was provided by the veterinary neurologist and is included with the study report.

For all animals in Groups 1, 2, and 3, absolute powers of frequency bands delta [0.5-4 Hz], theta [4-8 Hz], alpha [8-12 Hz], sigma [12-16 Hz], beta 1 [16-20 Hz], beta 2 [20-30 Hz], gamma 1 [30-55 Hz], gamma 2 [55-1000 Hz] and total power [0.5-1000 Hz] were calculated using NeuroScore software in 30 minute bins for two periods of approximately 24 hours prior to the first dose (reference item) of Phase 1, and a period of 24 hours after the 2nd and the 27th dose administration of reference item in Phase 2 (Group 1) or after the 2nd and the 27th dose administration of test item (Groups 2 and 3). For each frequency band, results were reported as percent change from a time matched 30 min baseline period in each animal.

In addition, percent change from time matched baseline of frequency bands listed above were calculated after the 2nd and the 27th dose administration of reference item in Phase 2 (Group 1) or after the 2nd and the 27th dose administration of test item (Groups 2 and 3) using the following bins.

- Set A: 2 to 8 hours, 8 to 14 hours, 14 to 20 hours and 20 to 24 hours post-dosing

· Set B: 2 hour bins from 0 to 24 hours post-dosing.

These 2 sets of data were used for statistical analysis (refer to section Data Analysis). Individual results, group means and standard deviations for sets A and B were not reported, but the statistical report was provided to the Study Director for inclusion in the final report. Frequency bands were calculated for Cz-Oz or C4-O2 derivation.

*3.1.8.10. Behavioral Video Monitoring*

Animals were continuously monitored (except during study designated procedures or other procedures such as data backup) using video cameras (concomitantly with the EEGs by telemetry monitoring) from at least 72 hours prior to the first dose of Phase 1 to at least 24 hours after the 28th dose in Phase 2 and also for a period of at least 36 hours (from at least 12 hours prior to dosing until at least 24 hours post-dosing) once in the 8th week of dosing and once towards the end of the dosing period (13th week of dosing) in Phase 2. Additionally, behavioral assessment of all animals was conducted by a technician in real time for 16 hours after the 1st, 3rd, 7th, 14th and 28th dose administration in Phase 2. Video monitoring was used to complement EEG interpretation.

*3.1.8.11. Procedures When Convulsions Occurred*

When convulsions were seen to occur after treatment, every attempt was made to acquire a blood sample for TK analysis from the animal immediately (within 5 min) after resolution of seizures, either spontaneously or after administration of an anticonvulsant. On some occasions where several episodes of convulsions occurred in a short period of time, blood samples could not be collected or were not taken within 5 minutes after resolution of seizure.

Convulsions occurring during the treatment period that had not resolved within 3 minutes of onset were controlled by administration of diazepam (1.0 mg/kg, IV).

Administration of phenytoin or propofol was not required as diazepam was sufficient to treat the convulsions that were observed in two females with COMPOUND A at 10.0 mg/kg.

*3.1.8.12. Toxicokinetics*

Blood samples (a target of 0.5 mL each, Na-heparin tubes) were collected from all animals at the following timepoints:

Phase 1: at 4 hours after dosing.

Phase 2: Plasma concentrations of COMPOUND A and its metabolites were determined for all dose groups after the 1st, the 29th dosing and the 90th dosing. Blood collection occurred in each animal in all groups before dosing (at the 29th and 90th dose only) and 1, 2, 4, 8, and 24 hours after dosing. Blood samples were collected into Na-heparin tubes via the implanted catheter or from the jugular, cephalic or femoral vein by venipuncture; tubes were kept on wet ice pending centrifugation. The blood samples were centrifuged at 5400 x g for 5 minutes under cooling conditions (set to 4°C) to obtain the plasma. The plasma was divided into duplicate samples (at least 100 μL each) and was kept on dry ice and stored in a freezer set to -20°C until shipment. Back-up samples (set 2 of 2) were stored separately from set 1 of 2.

Non-compartmental toxicokinetic analysis was performed by the subcontractor on the plasma concentration data for COMPOUND A, and its metabolites using validated WinNonlin software. The TK parameters ($t_{max}$, $C_{max}$, $t_{1/2}$, $MRT_{24}$, $MRT_{inf}$, $AUC_{24}$ and $AUC_{inf}$) were calculated for all animals except for the control animals.

Attempts were made to collect a blood sample (a target of 0.5 mL) from animals presenting with convulsions within 5 min after resolution of seizures. For cases in which animals were convulsing and the implanted catheter was used for injection of diazepam prior to blood collection, blood samples were collected from the femoral vein by venipuncture.

*3.1.8.13. Cerebro-Spinal Fluid Collection*

Prior to necropsy, CSF (a target of 2 mL) was collected into tubes from anesthetized animals via a puncture of the cerebellomedullary cistern at the foramen magnum using a needle and drip or aspiration method. Animals were anesthetized with an intramuscular injection of a combination of ketamine hydrochloride and acepromazine followed by maintenance with isoflurane by inhalation. A volume of rat plasma was added to each CSF sample to a ratio of 1 part of rat plasma for every 9 parts of CSF. For example, if 2 mL of CSF was retained then 0.222 mL of rat plasma was added. The volumes were adjusted but the ratio of plasma to CSF was maintained at 1:9.

The rat blood was previously collected from any strain of rats into tubes containing K2 EDTA or sodium heparin as anti-coagulant. The tubes of rat blood were kept on wet ice pending centrifugation and centrifuged under refrigeration (set to +4°C and 1500 g RCF) for a targeted 10 minutes to obtain plasma. Plasma was kept on dry ice until storage in a freezer (set to -70°C) until used. The rat plasma was thawed and kept on wet ice on the day of use, or collected on the same day as CSF collection and kept on wet ice.

Following the addition of the rat plasma, the sample was mixed well (using a vortex) and was transferred into 2 separate tubes. The CSF samples were labeled with at a minimum the animal number, species, dose group, day of collection, date, nominal collection time, sample type *(i.e.,* CSF), storage condition and study number. Samples were snap frozen using liquid nitrogen and placed on dry ice pending storage in a freezer set to -70°C. Back-up samples (set 2 of 2) were stored separately from set 1 of 2, except from 22 to 24 December 2015 and from 29 December 2015 to 04 January 2016 when both sets of some samples were stored in the same freezer. This deviation had no impact on the study since the temperature of the freezer during these periods remained within the acceptable range.

*3.1.8.14. Macroscopic Examination*

Animals surviving to scheduled termination were euthanized following an overnight period without food and subjected to a macroscopic examination. The animals were sedated with an intramuscular injection of a combination of ketamine hydrochloride and acepromazine, and then euthanized by an intravenous overdose of sodium pentobarbital, followed by exsanguination.

For all animals, necropsy consisted of an external macroscopic examination, including identification of all clinically-recorded lesions, as well as a detailed internal examination. Necropsies performed during regular working hours were conducted under the supervision of a veterinary pathologist.

A similar proportion of animals from each group and sex, as appropriate, was euthanized on any one day. Where possible, the order of necropsy for each prosecution group began with a control (Group 1).

In order to avoid autolytic changes, the macroscopic examination of the carcass was conducted, as soon as possible, on all animals which died prematurely or at scheduled termination.

The implanted transmitter was retrieved at macroscopic examination.

3.1.9. Brain Tissues for COMPOUND A and Metabolites Concentration Analysis

Two samples/animal of fresh brain tissue were weighed and retained from the frontal cortex and two samples from the striatum (100 mg minimum when possible, ideally, 150 mg each sample). To each brain sample, a volume of rat plasma was added in a ratio of 1:4. For example, if 150 mg of fresh brain tissue was retained, then 600 μL of rat plasma was added. If a different amount of tissue was collected, the volume of rat plasma was adjusted so as to maintain a 1:4 ratio.

The rat blood was previously collected from any strain of rats into tubes containing Na-heparin as anti-coagulant. Tubes were kept on wet ice pending centrifugation and centrifuged under refrigeration (set to +4°C and 1500 g RCF) for targeted 10 minutes to obtain plasma. The rat plasma was stored frozen (freezer set to -70°C) until being thawed and kept on wet ice on the day of use.

Following the addition of the rat plasma, the sample was then homogenized. The 2 frontal cortex samples from each animal were combined after homogenization and split into two equal aliquots. The two striatum samples from each animal were combined after homogenization and split into two equal aliquots. Samples were kept on wet ice pending storage in a freezer set to -70°C until shipment. The analytical methods used for brain tissues were not according to GLPs. Concentration of COMPOUND A and its metabolites in brain tissue samples were determined using a LC-MS/MS method and Analyst software.

*3.2. Discussion and Conclusion*

Oral administration of COMPOUND A to male and female cynomolgus monkeys once a day at dose levels ≤ 10.0 mg/kg/day for 13 weeks was not associated with any effects on mortality, bodyweights, clinical pathology, electrocardiography, ophthalmology, organ weight, macroscopic or microscopic parameters.

There were no adverse clinical signs or abnormal EEG changes with COMPOUND A at 0.3 or 3.0mg/kg/day in males or females. In males, decreased activity, tremors and circling were observed at 10 mg/kg/day but were not considered adverse as they were mild and transient and were not associated with abnormal EEG activity. In females, myoclonic jerks, decreased activity, weakness, and uncoordination were observed at 6.0 mg/kg/day and salivation, excessive licking, decreased activity, weakness, lying on cage floor, nystagmus, uncoordination, muscle twitches and myoclonic jerks were observed at 10.0 mg/kg/day. In females, these clinical signs at 6.0 and 10.0 mg/kg/day were observed with higher incidence and/or severity compared to control animals and were considered adverse. An episode suggestive of a self-limiting convulsion in a female monkey was witnessed by the technical staff on the second day of

dosing at 6.0 mg/kg/day. However, analysis of the EEG and EMG occurring at the time of the observation indicated that typical ictal or post-ictal changes did not occur after the clinical sign, suggesting that the episode was not a generalized seizure but may have been catalepsy or a partial seizure. In humans, catalepsy has been reported after administration of drugs such as ketamine (Heitz CR, Bence JR. Ketamine-induced catalepsy during adult sedation in the Emergency Department. J. Emerg. Med. 2013 Feb;44(2):e243-5.). Additionally, partial seizures in humans may not be identified by EEG if the focal ictal discharge is distant or deep, or involves too small a neuronal aggregate for synchronized depolarization activity to be detected on the EEG (Smith, 2005). The technical staff also reported a convulsion in the same animal after administration of COMPOUND A at 6.0 mg/kg/day on Day 79 but no EEG trace or video was available to characterize that episode.

Self-limiting generalized seizures, confirmed by EEG, were observed in two female monkeys treated with COMPOUND A at 10.0 mg/kg/day (1 and 10 generalized seizure episodes for animals 5502 on Day 2 and 5504 on Days 3, 7 and 8, respectively). Although tremors were present across all dose groups, the incidence and/or severity was highest in females at 10.0 or 6.0 mg/kg/day including the 2 female monkeys that did not demonstrate seizures. Thus, it is likely that the increased incidence and/or severity of tremors and seizures at 6.0/10.0 mg/kg/day reflects the exaggerated pharmacological action of COMPOUND A at higher dose levels, although the incidence of tremors and seizures was dissociable. In the animals that did show seizures, tremors were noted at least 2 hours before seizure onset; therefore, an increased incidence of tremors may be useful for monitoring early signs of potential toxicity associated with COMPOUND A treatment in humans, although the presence of tremors were not invariably linked to convulsions.

Overall, COMPOUND A was associated with an increase in total EEG power. The effects appeared to be greater in males although females still presented increased power mostly after the 2nd dose. Often, the magnitude of the stimulatory effects did not present a dose dependent pattern suggesting that the low dose of 0.3 mg/kg/day had activity comparable to that of 3 mg/kg/day. In some cases, the increases in power bands were followed by a compensatory decrease which might be expected to occur following activation. The most consistent qEEG effects seen were an increase in low frequencies (*i.e.,* Delta, Theta) and a decrease in higher frequencies (*i.e.,* Gamma 1) as well as an overall increase in total power in males after the 27th dose.

Pharmacokinetic analysis revealed higher COMPOUND A exposure in both females that presented generalized seizure compared to the other animals. Following administration of 6.0 and 10.0 mg/kg/day, the highest concentrations of COMPOUND A in plasma, CSF and brain were noted for the two females that presented convulsions. Episodes of convulsions were noted to occur around $t_{max}$. Additionally, TK analysis of plasma collected from these two animals shortly after the occurrence of what appeared to be convulsive episodes indicates that plasma levels of COMPOUND A were highest at these times, suggesting a relation between $C_{max}$ and convulsive episodes.

Across all TK sampling days, the mean $t_{max}$ values of COMPOUND A ranged from 2.0 to 15.0 hours. The mean $C_{max}$ and $AUC_{24}$ values increased dose-dependently after the 1st, the 29th and 90th dosing except for COMPOUND A between 3.0 and 10.0 mg/kg/day in males. With repeat dosing, the mean $C_{max}$ and $AUC_{24}$ values increased or tended to increase. There were no obvious sex differences in exposure except at the highest doses (10.0/6.0 mg/kg/day), at which females had exposures greater than males.

On the basis of the above, adverse clinical signs (salivation, excessive licking, decreased activity, weakness, tremors, muscle twitches, myoclonic jerks, uncoordination, nystagmus and lying on cage floor) and abnormal EEG changes suggestive of catalepsy or seizure were observed at > 3.0 mg/kg/day for females; therefore, it was concluded that the no-observed-adverse-effect level (NOAEL) was 10.0 mg/kg/day for males and 3.0 mg/kg/day for females.

**Table 7. Experimental Design for 13-Week NHP GLP Toxicity Study**

**Phase 1 (reference item)**

| Group | Treatment | Dose Level (mg/kg/day) | Dose Conc. (mg/mL) | Dose Conc. (w/v %) | Dose Volume (mL/kg) | Number of Animals | |
|---|---|---|---|---|---|---|---|
| | | | | | | Males | Females |
| 1 | Control * | 0 | 0 | 0 | 0 | 4 | 4 |
| 2 | | | | | | 4 | 4 |
| 3 | | | | | | 4 | 4 |
| 4 | | | | | | 4 | 4 |

*Reference item=0.5 w/v% MC in reverse osmosis water.

**Phase 2 (90-day repeat administration)**

| Group | Treatment[c] | | Dose Level (mg/kg/day) | Dose Conc. (mg/mL) | Dose Conc. (w/v %) | Animal ID | Number of Animals | |
|---|---|---|---|---|---|---|---|---|
| | | | | | | | M | F |
| 1 | Control[b] | 1st to 90th dose | 0 | 0 | 0% | 1001, 1102, 1003, 1104, 1601, 1502, 1503, 1604 | 4 | 4 |
| 2 | COMPOUND A Low Dose | 1st to 90th dose | 0.3 | 0.06 | 0.01% | 2001, 2002, 2003, 2004, 2501, 2502, 2503, 2504 | 4 | 4 |
| 3 | COMPOUND A Mid Dose | 1st to 90th dose | 3 | 0.6 | 0.06% | 3101, 3002, 3003, 3204, 3601, 3502, 3503, 3504 | 4 | 4 |
| 4 | COMPOUND A High Dose | 1st to 90th dose | 10 | 2 | 0.20% | 4001, 4002, 4003, 4004 | 4 | 0 |
| 5 | COMPOUND A High Dose | 1st to 9th dose | 10 | 2 | 0.20% | 5501 | 0 | 1 |
| | COMPOUND A Mid-high Dose | 10th to 90th dose | 6 | 1.2 | 0.12% | | | |
| | COMPOUND A High Dose | 1st to 8th dose | 10 | 2 | 0.20% | 5503, 5504[e] | 0 | 2 |
| | COMPOUND A Mid-high Dose | 9th to 90th dose | 6 | 1.2 | 0.12% | | | |
| | COMPOUND A High Dose | 1st and 2nd dose | 10 | 2 | 0.20% | 5502[f] | 0 | 1 |
| | COMPOUND A Mid-high Dose | 3rd to 90th dose | 6 | 1.2 | 0.12% | | | |

M: Male, F: Female

[a] The same animals were utilized in Phases 1 and 2 since no irregularities in EEG, behavior or other events occurred in any animal during Phase 1.

[b] Reference item=0.5 w/v% MC in reverse osmosis water.

[c] The dose volume was 5 mL/kg for all animals.

[d] Due to clinical observations of convulsions in two females at 10.0 mg/kg/day, the dose of COMPOUND A was reduced from 10.0 to 6.0 mg/kg/day for all females beginning at the dose number indicated.

[e] Animal 5504 had a dosing holiday from the 2nd to 4th doses and from the 8th to 12th doses inclusively.

[f] Animal 5502 had a dosing holiday from the 2nd to 4th doses inclusively.

**Example 4: Population PK Modeling and Simulation of COMPOUND A to Support Dose Selection for Planned Clinical Study**

*4.1. Methods*

4.1.1. Data Processing

Data processing and graphical visualization was performed using Matlab (version R2019a; The MathWorks, Inc., Natick, Massachusetts, United States). While no outliers were eliminated from the available data, one missing data point was excluded from the analysis, and concertation-time points below the LOQ were handled using the M3 Beal's method built in Monolix (2019-R1 suite; Lixoft SAS, Antony, France).

4.1.2. Model Building and Evaluation

The population PK model describing the PK of COMPOUND A following oral administration in active subjects enrolled in the COMPOUND A study (Example 1) was developed using nonlinear mixed effects modeling performed in Monolix. The Stochastic Approximation of Expectation Maximization (SAEM) algorithm, a stochastic algorithm for calculating the maximum likelihood estimator, was selected as model-estimation method for its ability to accurately estimate population parameters.

Model building and selection was based on the following criteria/procedures:

- Evaluation of individual and population parameter estimates and their precision (precision was quantified using the percentage of relative standard error (%RSE));
- Graphical examination of diagnostic and goodness-of-fit plots;
- Analysis of correlation matrix of the estimates and condition number;
- Reduction in both residual variability and inter-individual variability; and
- Reduction in loglikelihood (-2LL), Akaike Information Criterion (AIC) and Bayesian Information Criterion (BIC) for all nested models.

The base model was built by using these criteria to compare different structural models (*e.g.*, one-compartment, two-compartment, three-compartment) with different absorption (*e.g.,* zero order, first order) and elimination (*e.g.,* linear, nonlinear) processes. Development of the base model was performed by adding inter-individual variability (IIV) on one structural parameter at a time and applying the set criteria to select the best model. IIV was generally modeled as an exponential term as follows:

$$\log(\mathrm{Par}) = \log(\mathrm{TV_{Par}}) + \mathrm{ETA_{Par}} \quad (1)$$

or equivalently:

$$\mathrm{Par} = \mathrm{TV_{Par}} * \exp(\mathrm{ETA_{Par}}) \quad (2)$$

In equations (1) and (2), ETA's were assumed to be normally distributed. Several error models were also tested, including additive error, proportional error and combined (additive + proportional) error. Functional form a combined additive and proportional error terms is the following:

$$\mathrm{Y} = \mathrm{Y} + (a + b\mathrm{Y}) * \mathrm{eps} \quad (3)$$

In equation (3), eps was assumed to be normally distributed. Correlation between random effects was also included to account for potential collinearity between individual parameters.

A covariate model was then built to further reduce the unexplained inter-individual variability. Development of the covariate model was performed using the forward selection and backward elimination method. In the forward selection step, covariates were tested one at the time. Those that significantly (p-value<0.05) decreased the -2LL (delta-2LL<-3.84) were retained, and the one that produced the most significant reduction was ultimately incorporated in the base model. This step was repeated until no covariates were found to significantly decrease the -2LL. In the backward elimination step, covariates were eliminated one at the time and changes in -2LL recorded. If elimination of a covariate significantly (p-value<0.001) increased the -2LL (delta-2LL>10.83), the covariate was eliminated from the model; otherwise, it was retained. This step was repeated until covariate elimination was found not to significantly increase the -2LL, and the corresponding model was considered as the final model.

All investigated continuous covariates were centered/normalized with respect to reference values and added to the base model using a power function as follows:

$$\log(Par) = \log(TV_{Par}) + THETA*\log(Cov/Cov_{Ref}) + ETA_{Par} \quad (4)$$

or equivalently:

$$Par = TV_{Par}*(Cov/Cov_{Ref})\text{^}THETA*\exp(ETA_{Par}) \quad (5)$$

All investigated categorical covariates were scaled with respect to a reference value (set equal to 0) and added to the base model as follows:

$$\log Par = \log Par_{\boldsymbol{pop}} + \sum_{\boldsymbol{k=1}}^{\boldsymbol{n}} k\ theta_k + eta_{par}$$

where n is the number of categories belonging to the given covariate.

The final model was evaluated using a visual predictive check, a simulation-based method to visually assess concordance of the model-based simulations and observed data. Bootstrap analysis was also performed to determine the precision of the parameter estimates, estimate confidence interval of identified parameters and assess the stability of the model.

4.1.3. Model-Based Simulations

The developed POPPK model was used to inform the design of the planned Phase 2 clinical study by simulating median and variability of COMPOUND A steady states exposure ($C_{max,ss}$ and AUC,ss) in 1000 healthy subjects receiving different dosing regimens. In performing this scenario analysis, the predicted AUC, ss of COMPOUND A was constrained to remain at all times below 10-fold of the NOAEL exposure cap obtained from the 14-day neurotoxicity study in rats (17400 ng·h/mL) to assure safety of the recommended dose for the upcoming phase 2 study. The R-based Monolix PK simulator, SimulX, was used to simulate the final model for various dose regimens. SimulX was implemented in R (Version 3.5.3; Vienna, Austria) via the RStudio console (Version 1.2.133).

*4.2. Results*

4.2.1. Final Model

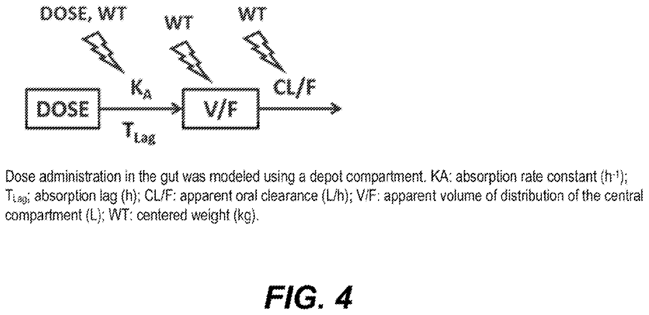

FIG. 4 shows the schematic structure of the final POPPK model. The parameter estimates of the final model, the precision of the parameter estimates reported as the percent relative standard error (RSE%), the parameter estimates of the final model and their confidence interval (CI) obtained from bootstrap analysis are listed in Table 8. All values of the population parameters were estimated with good precision (RSE<37%). IIV was <70% for TLag, V1/F and CL and <99% for $K_A$. Additive and proportional residual variability was approximately 35% SD and 38% CV, respectively.

4.2.2. Model-Based Simulations to Inform the Design of COMPOUND A Study

Since the final POPPK model adequately described the PK of COMPOUND A orally administered in SRD/MRD cohorts of healthy subjects, the model was used to guide the design of the planned COMPOUND A clinical study. A scenario analysis was performed to simulate various dose regimens and recommend the dose that would maximize COMPOUND A exposure (AUC,ss) while keeping exposure below 10-fold of the NOAEL exposure cap obtained from the 14-day neurotoxicity study in rats (17400 ng·h/mL). The following scenarios were investigated:

1. (Reference) Daily administration of 2.5 mg of COMPOUND A for 8 days;

2. Loading dose of 6 mg followed by daily administration of 2.5 mg of COMPOUND A for 7 days; and

3. Loading dose of 6 mg followed by daily administration of 3 mg of COMPOUND A for 7 days.

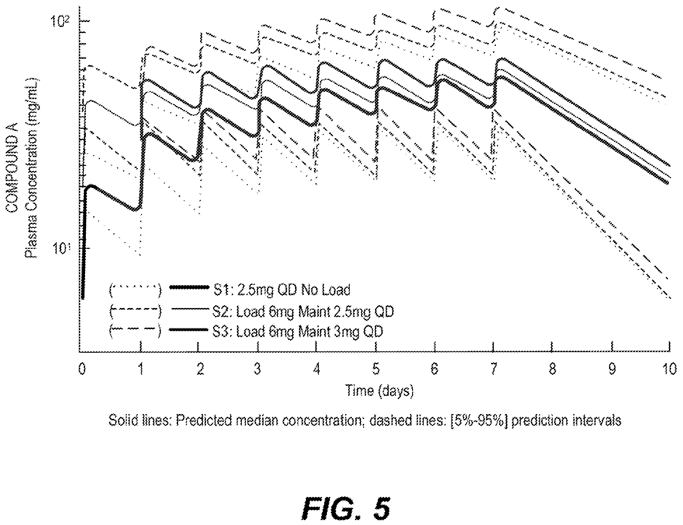

FIG. 5 summarizes the results of the simulated dose regimens considered for the planned COMPOUND A clinical

study.

Table 9 reports the exposure margins relative to exposure in rats at 1/10 of NOAEL from neurotoxicity studies. The simulation results indicate that the median exposure margins remain well below the NOAEL cap for all proposed scenarios of the COMPOUND A study.

Table 10 lists the percentage of $C_{max,ss}$ reached after repeated dosing. The use of a loading dose enables fine tuning the attainment of steady-state conditions within prespecified timelines.

**Table 8. Summary of Final Model Parameters and Their Precision**

| Parameter | Population Parameter Estimates | Precision (RSE %) | Median Bootstrap Parameter Estimates | Median Bootstrap Parameter CI [5%, 95%] |
|---|---|---|---|---|
| $T_{Lag}$_pop | 0.37 | 5.1 | 0.37 | [0.34, 0.40] |
| $K_A$_pop | 6.25 | 23.1 | 6.64 | [4.84, 9.11] |
| DOSE on $K_A$ | -0.36 | 23.4 | -0.39 | [-0.50, -0.27] |
| WT on $K_A$ | 2.19 | 36.3 | 2.13 | [1.06, 3.19] |
| V/F_pop | 119.63 | 1.5 | 119.47 | [116.58, 122.50] |
| WT on V/F | 1.13 | 9.8 | 1.13 | [0.97, 1.30] |
| CL/F_pop | 1.95 | 4.1 | 1.95 | [1.82, 2.09] |
| WT on CL/F | 1.3 | 23.6 | 1.25 | [0.76, 1.77] |
| $T_{Lag}$_IIV (%CV) | 69.17 | 9.5 | 67.78 | [61.97, 74.66] |
| $K_A$_IIV (%CV) | 98.99 | 11.4 | 97.18 | [85.83, 109.70] |
| V/F_IIV (%CV) | 33.77 | 10.4 | 33.27 | [29.42, 36.49] |
| CL/F_IIV (%CV) | 62.53 | 8.9 | 61.97 | [57.08, 66.05] |
| V/F-CL/F corr | 0.44 | 25.1 | 0.43 | [0.21, 0.59] |
| Additive Error (SD) | 0.35 | 18.7 | 0.34 | [0.24, 0.42] |
| Proportional Error (%CV) | 37.56 | 2.5 | 37.72 | [35.07, 40.23] |

$T_{Lag}$_pop: population value of $T_{Lag}$$K_A$_pop: population value of $K_A$; CL/F_pop: population value of CL/F; V/F_pop: population value of V/F; DOSE on $K_A$: effect of dose as covariate on $K_A$; WT on $K_A$: effect of weight as covariate on $K_A$; WT on V/F: effect of weight as covariate on $V_1$/F; WT on CL/F: effect of weight as covariate on CL/F; $T_{Lag}$_IIV: inter-individual variability for $T_{Lag}$; $K_A$_IIV: inter-

individual variability for $K_A$; V/F_IIV: inter-individual variability for V/F; CL/F_HV: inter-mdividual variability for CL/F; V/F-CL/F corr: correlation between V/F and CL/F

**Table 9. Exposure Margins Relative to Exposure in Rats at NOAEL**

| Clinical Dose | Predicted Clinical Exposure | Exposure margins relative to exposure in rats at NOAEL (17400 ng*h/mL) |
|---|---|---|
| | AUC,ss (ng*h/mL) Median [5% - 95%] | Fold $AUC_{,SS}$ Median [5% - 95%] |
| Load 2.5 mg Main 2.5 mg QD | 1158 [612 - 2094] | 15 [28.4 - 8.3] |
| Load 6 mg Main 2.5 mg QD | 1158 [612 - 2100] | 15 [28.4 - 8.3] |
| Load 6 mg Main 3 mg QD | 1389 [735 - 2518] | 12.5 [23.7 - 6.9] |

Steady-state was assumed to be attained after administration of 1 loading dose followed by 19 maintenance doses of COMPOUND A.

**Table 10. Percentage of $C_{max,ss}$ Attained After Repeated Daily Dosing**

| Dosing Time Day | Load 2.5 mg Main 2.5 mg QD Median [5%-95%] | Load 6 mg Main 2.5 mg QD Median [5%-95%] | Load 6 mg Main 3 mg QD Median [5%-95%] |
|---|---|---|---|
| 1 | 31.7 [24.8 - 40.3] | 75.9 [59.4 - 96.7] | 63.3 [49.6 - 80.6] |
| 2 | 53.3 [42.6 - 66.5] | 83.5 [68 - 102.5] | 74.8 [61 - 92.2] |
| 3 | 67.8 [56.1 - 81.6] | 88.3 [74.9 - 101.8] | 82.7 [69.4 - 95.7] |

(continued)

| Dosing Time Day | Load 2.5 mg Main 2.5 mg QD Median [5%-95%] | Load 6 mg Main 2.5 mg QD Median [5%-95%] | Load 6 mg Main 3 mg QD Median [5%-95%] |
|---|---|---|---|
| 4 | 77.8 [66.1 - 89.8] | 92.1 [80.1 - 100.1] | 88.2 [76 - 97.7] |
| 5 | 85 [73.6 - 94.7] | 94.5 [84.6 - 99.8] | 91.7 [81.4 - 98.1] |
| 6 | 89.5 [79.7 - 96.3] | 96.1 [87.9 - 99.8] | 94.2 [85.6 - 98.6] |
| 7 | 92.8 [84.3 - 97.6] | 97.4 [91.3 - 99.8] | 96 [89.3 - 99.1] |
| 8 | 95.1 [88.4 - 98.4] | 98.1 [93.4 - 99.9] | 97.4 [92.2 - 99.4] |

*4.3. Summary and Conclusion*

Data from COMPOUND A clinical study (Example 1) was used to develop a POPPK model aimed at recommending optimal dose regimens for the planned COMPOUND A clinical study using model-based simulations. A total of 2377 observed individual COMPOUND A plasma concentration-time points from 66 active subjects was used for modeling development. A total of 44 COMPOUND A plasma concentration-time points (1.85% of overall data) was found below the limit of quantification (LOQ) of 0.1 ng/mL. All the available data was used to for model building and evaluation.

A one-compartment model with a lagged, first-order absorption process and linear elimination was identified to best describe the PK of COMPOUND A following single (0.3-18 mg) and multiple (0.3-9 mg QD) oral dose administration in healthy subjects. Inter-individual variability (IIV) was included as an exponential term on absorption rate constant ($K_A$), lag time ($T_{Lag}$), apparent volume of distribution of the central compartment (V/F) and apparent clearance after extravascular administration (CL/F). To better capture the observed $C_{max}$ and $C_{trough}$ at all dose regimens, weight was added as a covariate on $K_A$, CL/F and V/F using a power function, and dose was added as a covariate on $K_A$ using a power function. Residual error was modeled as a combined additive and proportional error. Model qualification was conducted using visual predictive check and bootstrapping.

Since the developed PopPK model adequately described the PK of COMPOUND A, the model was used to select optimal dose regimens for the planned COMPOUND A clinical study. Based on the model simulations, subjects enrolled in the COMPOUND A study will receive a 6 mg loading dose of COMPOUND A on Day 1 followed by a 3 mg daily maintenance dose. The proposed daily maintenance dose after the loading dose will have a predicted area under the time-concentration curve, at steady-state (AUC,ss) that is on average at least 10-fold below the observed NOAEL exposure cap obtained from neurotoxicology studies in rats.

**Example 5: Randomized, Double-Blind, Placebo-Controlled, 3-Period Crossover Study Followed by 1 Open-Label Comparator Period to Evaluate the CNS PD Activity of COMPOUND A in Healthy Subjects Using TMS**

*5.1. Overall Study Design and Plan*

As shown in Table 11, this randomized, double-blind, placebo-controlled, 3-period crossover study followed by 1 open-label comparator period was designed to evaluate the CNS PD activity of COMPOUND A in healthy subjects using a transcranial magnetic stimulation (TMS). Twenty-four healthy male and female subjects aged 18 to 55 years, inclusive, were enrolled.

**Table 11. Summary of Study Population**

| | Total (N=24)[a] |
|---|---|
| **Age (years)** | |
| Mean (±SD) | 27.9 (9) |
| Minimum-maximum | 20-49 |
| **Gender, n (%)** | |
| Male | 23 (96) |
| Female | 1 (4) |
| **Race, n (%)** | |
| Caucasian | 22 (92) |
| Black* | 1 (4) |

(continued)

| | |
|---|---|
| **Race, n (%)** | |
| Asian | 1 (4) |
| Other | n/a |
| **BMI: Mean (±SD), kg/m²** | 23.9 (3) |

[a] All human analyses included 24 subjects except change of MEP 6.0 g dose, which only included 23 of the 24.
* Black and white biracial subject counted once as black.

The study included 3 treatments (placebo, COMPOUND A 0.5 mg, and COMPOUND A 6 mg) over 3 periods, each 1 day in duration, followed by a 2-day comparator (IV ketamine 0.5 mg/kg) period, alternating with 3 washout periods of at least 10 days (not to exceed 15 days). There were 5 clinic visits, including screening in which full medical, neurological, and psychiatric examinations were conducted; Day 1 of each of the first 3 treatment periods, during which TMS testing was conducted before and after subjects received placebo or COMPOUND A; and Day 1 of the fourth treatment period, during which TMS testing was conducted before and after subjects receive the NMDA antagonist ketamine, which was open-label.

Before the start of each treatment period, the investigator reviewed the safety assessments for the subject, including vital signs and ECG.

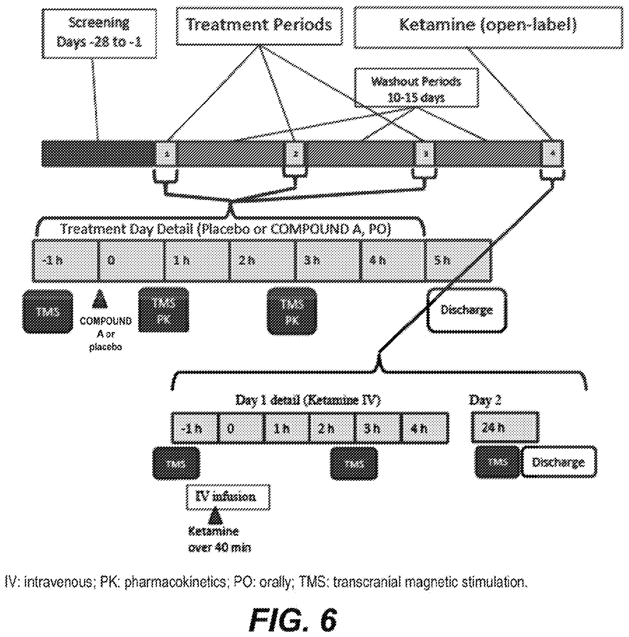

Referring now to FIG. 6, on the first day of each treatment period (Day 1), each subject underwent a TMS session assessing baseline TMS-elicited MEP amplitude and threshold, followed by administration of COMPOUND A or placebo (Treatment Periods 1-3) or ketamine (Treatment Period 4). For the first 3 treatment periods, TMS and MEP/EEG assessment sets were conducted along with blood sample collection for PK analyses at pre-dose and starting at 30 and 150 minutes (2.5 hours) after dosing. The 2.5-hour time point was chosen to capture $C_{max}$, and the 30-minute time point was to capture a lower exposure level in each subject. In Treatment Period 4, each subject underwent a TMS session assessing baseline TMS-elicited MEP amplitude and threshold, and blood sample collection for PK analyses at pre-dose, followed by administration of ketamine IV (0.5 mg/kg over 40 minutes). TMS assessments were conducted at 150 minutes (2.5 hours) after ketamine initiation (when ketamine acute dissociative effects had subsided), and blood samples were collected for PK analysis at selected time points. Subjects underwent a follow-up TMS session starting at 24 hours (Day 2 of Treatment Period 4) to capture ketamine modulation of TMS responses at a time in which antidepressant effects of ketamine are already present in a major depressive disorder (MDD) population.

Subjects remained in the clinic on treatment days until approximately 2 hours post-TMS procedures for safety observation. During the ketamine-testing period, subjects remained at the site overnight after the infusion, until the 24-hour follow-up TMS assessments were completed. Before discharge, a neurological and mental state examination was performed.

EEG and MEP signals evoked by TMS as PD endpoints for assessing the effects of COMPOUND A were selected on the basis of nonclinical data showing that COMPOUND A increases the amplitude of MEPs in rats. The goal of this study was to evaluate the PD effects of COMPOUND A after single-dose treatment as measured by responses to TMS.

Details of study drug and sequences are presented in Table 12.

**Table 12. Study Drug and Treatment Sequences**

| Sequence | Period 1 | Period 2 | Period 3 | Period 4 |
|---|---|---|---|---|
| 1 | COMPOUND A 0.5 mg | COMPOUND A 6 mg | Placebo | Ketamine 0.5 mg/kg |
| 2 | COMPOUND A 6 mg | COMPOUND A 0.5 mg | Placebo | Ketamine 0.5 mg/kg |
| 3 | COMPOUND A 0.5 mg | Placebo | COMPOUND A 6 mg | Ketamine 0.5 mg/kg |
| 4 | COMPOUND A 6 mg | Placebo | COMPOUND A 0.5 mg | Ketamine 0.5 mg/kg |
| 5 | Placebo | COMPOUND A 0.5 mg | COMPOUND A 6 mg | Ketamine 0.5 mg/kg |

(continued)

| Sequence | Period 1 | Period 2 | Period 3 | Period 4 |
|---|---|---|---|---|
| 6 | Placebo | COMPOUND A 6 mg | COMPOUND A 0.5 mg | Ketamine 0.5 mg/kg |

Subjects reported to the clinical site on Day 1 of all treatment periods and left after completion of all study-related procedures on each day on Treatment Periods 1 through 3. At the discretion of the investigator, subjects may have been requested to remain in the clinical site longer. On Day 1 of Treatment Period 4, during the ketamine-testing period, subjects remained at the site overnight after the infusion, until the 24-hour follow-up TMS assessment had been completed. Before discharge, a neurological and mental state examination was performed.

Subjects fasted from all food and drink except water for at least 8 hours before screening as well as before study drug dosing in all 4 treatment periods. Subjects received a single oral dose of study drug (COMPOUND A or placebo tablets in Treatment Periods 1-3), ketamine (Treatment Period 4), and TMS procedures, and a meal was delivered after the second TMS assessment on dosing days, along with approximately 240 mL of water. Water was restricted 1 hour before and 1 hour after study drug administration. A standard meal was provided to subjects present during meal times on scheduled study visit days listed in the schedule of study procedures. The caloric content and composition of meals was the same for all subjects in each treatment period. For Treatment Period 4, after the 24-hour post-dose procedures were completed, subsequent meals and snacks were unrestricted in caloric content, composition, and timing.

Safety was assessed by full physical examination, vital signs, 12-lead ECG, AE monitoring, and clinical laboratory tests (hematology, chemistry, and urinalysis).

PD assessments were performed to determine threshold and amplitude of MEP driven by single-pulse TMS, TMS stimulation paradigms, EEG signals, and CHDR Neurocart (body sway, smooth pursuit eye movements, saccadic eye movements, adaptive tracking, Stroop test, Bond and Lader visual analogue scale (VAS) and Bowdle VAS).

Plasma concentrations of COMPOUND A and ketamine were determined for all evaluable subjects.

For this trial, TMS-evoked MEP was the critical procedure.

TMS post-dose assessments were performed as close to the exact nominal time point/scheduled time as possible.

All other procedures were performed as close as possible (either before or after the nominal time of the critical procedure).

ECG and vital signs measurements were performed before the PK blood draw, if scheduled together.

The order of priority could be changed during the study with joint agreement of the investigator and the sponsor.

Any nonscheduled procedures required for urgent evaluation of safety concerns took precedence over all routine scheduled procedures.

*3.2. Discussion and Overall Conclusions*

5.2.1. Discussion

This study was designed to evaluate whether COMPOUND A, in comparison with placebo, increases CNS excitability in healthy subjects. The study provides a preliminary assessment of whether COMPOUND A modulates the responses evoked with paired TMS pulses that capture intracortical modulation, and whether ketamine increases CNS excitability assessed with TMS-evoked MEP.

Peak-to-peak amplitude of the MEP was obtained with single-pulse TMS (stimulation intensity 120% of baseline rMT). COMPOUND A 6 mg at 2.5 hours post-dose showed a statistically significant difference from baseline when compared with placebo. Changes from baseline in the rMT obtained with single-pulse TMS were not statistically significant. The change from baseline of LICI 300 ms was statistically significant for COMPOUND A 0.5 mg at 2.5 hours post-dose. The SICI measurements, as well as other secondary endpoints, were not statistically significant. COMPOUND A was generally well tolerated at single doses of COMPOUND A 0.5 mg, and COMPOUND A 6 mg as oral tablets, and 20 subjects received single doses of ketamine 0.5 mg/kg administered as an IV infusion. No deaths or SAEs were reported, and no subject in placebo or COMPOUND A treatments discontinued because of AEs or experienced AESIs (convulsions and seizures). One subject in the ketamine treatment was discontinued from study drug because of AEs.

5.2.2. Conclusions

COMPOUND A 6 mg demonstrated a statistically significant PD effect as measured by TMS measure of single-pulse peak-to-peak amplitude at 2.5 hours post-dose. Additionally, COMPOUND A 0.5 mg demonstrated a statistically

significant PD effect as measured by rMT, LICI 300 ms only, at 2.5 hours post-dose.

**Example 6: A Randomized, Double-Blind, Placebo-Controlled Study to Assess Safety and Pharmacokinetics of Adjunctive COMPOUND A in Adult Subjects with Major Depressive Disorder**

A Phase 1b, randomized, double-blind, placebo-controlled study will be conducted in up to 3 study centers in the United States in approximately 10 subjects (minimum of 6 active and 2 placebo completers).

The subjects will include male and female subjects, 18 to 65 years of age (inclusive), with a primary diagnosis of MDD who are currently on stable pharmacological treatment for depression, defined as ≤50% change in dose during the 6 weeks prior to randomization. Any other psychopharmacological treatment, if present, is also stable.

Subjects will be housed in an inpatient study center for 2 weeks to monitor the safety and tolerability of COMPOUND A. After providing informed consent, subjects will be screened for eligibility within 28 days before Day 1 (first day of dosing). Eligible subjects will be admitted to the study center on Day -1 (day before dosing), and safety assessments will be conducted.

On Day 1, subjects will be randomized to receive COMPOUND A or placebo, respectively, for 2 weeks. Doses will be formulated as tablets for oral administration, and subjects will be instructed to take their study drug with water. Subjects in the COMPOUND A group will be administered a single loading dose of 6 mg on Day 1 followed by 3 mg daily from Day 2 to Day 14; subjects in the placebo group will receive matching placebo tablets on an identical schedule as COMPOUND A. Study treatment will be administered orally each morning on Days 1 to 14 (study treatment may be administered with or without food).

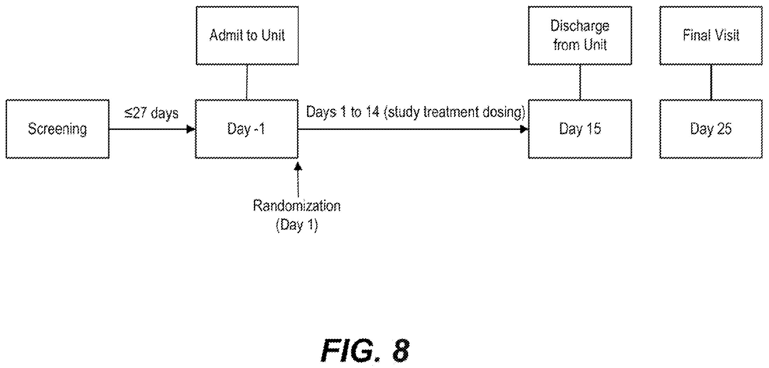

The expected duration of study participation for each subject is approximately 53 days, including up to 28 days of screening, 15 days of inpatient stay, and 10 days of follow-up. A schematic of the study design is provided in FIG. 8.

The primary objective of the study is to evaluate the safety and tolerability of COMPOUND A when administered to subjects with major depressive disorder (MDD) on antidepressant medication. The safety endpoints investigated will be treatment-emergent adverse events (TEAEs), electrocardiograms (ECGs), vital signs, clinical laboratory values, and Columbia-Suicide Severity Rating Scale (C-SSRS) results.

Exploratory endpoints will include: (1) assessing the PK of COMPOUND A in subjects with MDD on antidepressant medication(s); (2) exploring the efficacy of COMPOUND A on symptoms of depression, as measured by the Montgomery Åsberg Depression Rating Scale (MADRS); (3) exploring the overall effect of COMPOUND A on MDD severity, as measured by the Clinical Global Impression-Severity Scale (CGI-S); and (4) exploring the overall effect of COMPOUND A on MDD improvement, as measured by the Clinical Global Impression-Improvement Scale (CGI-I).

All safety and exploratory endpoints will be summarized descriptively by treatment group.

**Table 13. Study Treatments**

| Treatment Group | Active (COMPOUND A) | Placebo |
|---|---|---|
| Treatment administration | COMPOUND A will be administered as a single loading dose of 6 mg on Day 1 followed by 3 mg daily from Day 2 to Day 14 | Placebo will be administered in the same manner and on an identical schedule as COMPOUND A |
| Unit dose strength | 3 mg per tablet | Not applicable |
| Dose formulation | Tablet | Tablet |
| Route of administration | Oral | Oral |
| Sourcing | Provided centrally by Sponsor | Provided centrally by Sponsor |
| Packaging and labeling | Provided in HDPE bottles labeled in accordance with applicable regulatory requirements. | Provided in HDPE bottles labeled in accordance with applicable regulatory requirements. |
| HDPE=high density polyethylene | | |

**Table 14. List of Abbreviations**

| List of Abbreviations | |
|---|---|
| **Term** | **Definition** |
| %CV | percent of coefficient of variation |

(continued)

| List of Abbreviations | |
|---|---|
| **Term** | **Definition** |
| $\tau$ | dosing interval |
| AE | adverse event |
| AESI | adverse event of special interest |
| $Ae_t$ | amount of drug excreted in urine from time 0 to time t |
| $Ae_\tau$ | amount of drug excreted in urine during a dosing interval $\tau$ |
| ALT | alanine aminotransferase |
| AMPA | alpha-amino-3-hydroxy-5-methyl-4-isoxazolepropionic acid |
| AST | aspartate aminotransferase |
| AUC | area under the plasma concentration-time curve |
| $AUC_\infty$ | area under the concentration-time curve from time 0 to infinity |
| $AUC_{24}$ | area under the concentration-time curve from time 0 to 24 hours |
| $AUC_{last}$ | area under the plasma concentration-time curve from time 0 to the time of the last quantifiable concentration |
| AUC$\tau$ | area under the concentration-time curve during a dosing interval |
| AUEC | area under the effect curve |
| $AUEC_{24}$ | area under the effect curve from time 0 to 24 hours |
| $AUEC_{96}$ | area under the effect curve from time 0 to 96 hours |
| BMI | body mass index |
| $C_{av,ss}$ | average plasma concentration at steady state |
| CL/F | apparent clearance after extravascular administration |
| $CL_R$ | renal clearance |
| $C_{max}$ | maximum observed plasma concentration |
| $C_{max,ss}$ | maximum observed plasma concentration during a dosing interval at steady state |
| CNS | central nervous system |
| CS | clinically significant |
| CSF | cerebrospinal fluid |
| C-SSRS | Columbia-Suicide Severity Rating Scale |
| ECG | electrocardiogram |
| eCRF | electronic case report form |
| EEG | electroencephalogram |
| $E_{max}$ | maximum drug-induced effect |
| FDA | U.S. Food and Drug Administration |
| GCP | Good Clinical Practice |
| GGT | $\gamma$-glutamyl transferase |
| GLP | Good Laboratory Practice |
| HBsAg | hepatitis B virus surface antigen |
| HCV | hepatitis C virus |
| ICF | informed consent form |
| ICH | International Council for Harmonisation |

(continued)

| List of Abbreviations | |
|---|---|
| **Term** | **Definition** |
| IEC | independent ethics committee |
| INR | international normalized ratio |
| IRB | investigational review board |
| LFT | liver function test |
| MADRS | Montgomery-Åsberg Depression Rating Scale |
| MAV | markedly abnormal value |
| MDD | major depressive disorder |
| MedDRA | Medical Dictionary for Regulatory Activities |
| MRD | multiple-rising dose |
| NHP | non-human primate(s) |
| NOAEL | no-observed-adverse-effect level |
| OTC | over-the-counter |
| PD | pharmacodynamics(s) |
| PGx | pharmacogenomics |
| PK | pharmacokinetic(s) |
| PT | preferred term |
| PTE | pretreatment event |
| QD | once daily |
| qEEG | quantitative electroencephalogram |
| QTcF | QT interval with Fridericia correction method |
| POPPK | population pharmacokinetic |
| $R_{ac(AUC)}$ | accumulation ratio (based on AUC) |
| $R_{ac(Cmax)}$ | accumulation ratio (based on $C_{max}$) |
| SAE | serious adverse event |
| SAP | statistical analysis plan |
| SI | Systéme Internationale (units) |
| SOC | system organ class |
| SRD | single-rising dose |
| $t_{1/2z}$ | terminal disposition phase half-life |
| TEAE | treatment-emergent adverse event |
| TK | toxicokinetic(s) |
| $t_{max}$, | time of first occurrence of $C_{max}$ |
| TRD | treatment-resistant depression |
| ULN | upper limit of normal |
| $V_z/F$ | apparent volume of distribution during the terminal disposition phase after extravascular administration |

## Claims

1. A compound which is 9-[4-(cyclohexyloxy)phenyl]-7-methyl-3,4-dihydropyrazino[2,1-c][1,2,4]thiadiazine 2,2-dioxide ("COMPOUND A"), or a pharmaceutically acceptable salt thereof, for use in a method for treating depression in a patient, the method comprising:

   administering to the patient a first dose comprising COMPOUND A or a pharmaceutically acceptable salt thereof; and
   administering to the patient at least one daily maintenance dose comprising COMPOUND A or pharmaceutically acceptable salt thereof, wherein the at least one daily maintenance dose is less than the first dose.

2. The method according to claim 1, wherein:

   (1) the depression is major depressive disorder;
   (2) the depression is treatment-resistant depression; and/or
   (3) treating depression comprises treating cognitive impairment associated with the depression.

3. The compound or pharmaceutically acceptable salt for use according to claim 1 or 2, wherein:

   (1) the patient has failed to respond to at least one prior therapy for depression; and/or
   (2) the patient is a responder to at least one prior therapy for depression.

4. The compound or pharmaceutically acceptable salt for use according claim 3, wherein the at least one prior therapy for depression comprises an administration of:

   ketamine or a salt thereof; (S)-ketamine (esketamine) or a salt thereof; (R)-ketamine or a salt thereof; norketamine or a salt thereof; 2 diastereomeric hydroxyketamine or a salt thereof; 6 diastereomeric hydroxynorketamine (HNK) or a salt thereof; (2S,6S)-HNK or a salt thereof; (2R,6R)-HNK or a salt thereof; dehydronorketamine or a salt thereof; methoxetamine or a salt thereof; or combinations thereof;
   preferably wherein the at least one prior therapy comprises an administration of ketamine or a salt thereof; (S)-ketamine (esketamine) or a salt thereof; methoxetamine or a salt thereof; or combinations thereof.

5. The compound or pharmaceutically acceptable salt for use according to any one of claims 1-4, wherein: the at least one daily maintenance dose is administered not less than about 12 hours after the first dose.

6. The compound or pharmaceutically acceptable salt for use according to any one of claims 1-5, wherein:

   (1) the first dose comprises between about 5 mg and about 9 mg of COMPOUND A or a pharmaceutically acceptable salt thereof;
   (2) the first dose is effective to achieve a mean $C_{max}$ in the patient of at least about 6 ng/mL;
   (3) the first dose is effective to achieve a mean $C_{max}$ in the patient of not more than about 100 ng/mL;
   (4) the first dose is effective to achieve a mean $C_{max}$ in the patient of about 6 ng/mL to about 100 ng/mL; and/or
   (5) the first dose is effective to achieve a mean $C_{max}$ in the patient of about 75% to about 130% of 42 ng/mL.

7. The compound or pharmaceutically acceptable salt for use according to any one of claims 1-6, wherein:

   (1) the at least one daily maintenance dose comprises between about 0.5 mg and about 4 mg once per day of COMPOUND A or a pharmaceutically acceptable salt thereof;
   (2) the at least one daily maintenance dose is effective to achieve a mean $C_{max}$ at steady-state in the patient of no greater than about 130 ng/mL; and/or
   (3) the at least one daily maintenance dose is effective to achieve a mean $C_{max}$ at steady-state in the patient of about 75% to about 130% of 78 ng/mL.

8. The compound or pharmaceutically acceptable salt for use according to any one of claims 1-7, wherein:

   (1) the mean $AUC_T$ at steady-state in the patient is no greater than about 2600 ng·h/mL;
   (2) the mean $AUC_T$ at steady-state in the patient is about 700 ng·h/mL to about 2600 ng·h/mL;
   (3) the mean $AUC_T$ at steady-state in the patient is about 75% to about 130% of 1487 ng·h/mL; and/or

(4) the mean AUC- in the patient is about 75% to about 130% of 2328 ng·h/mL.

**9.** The compound or pharmaceutically acceptable salt for use according to any one of claims 1-8, wherein:

(1) the first dose is orally administered; and/or
(2) the at least one daily maintenance dose is orally administered.

**10.** The compound or pharmaceutically acceptable salt for use according to any one of claims 1-9, wherein:

(1) the first dose comprises about 6 mg of COMPOUND A or a pharmaceutically acceptable salt thereof;
(2) the maintenance dose comprises about 3 mg once per day of COMPOUND A or a pharmaceutically acceptable salt thereof;
(3) the first dose comprises about 6 mg of COMPOUND A or a pharmaceutically acceptable salt thereof, and the at least one daily maintenance dose comprises about 3 mg of COMPOUND A or a pharmaceutically acceptable salt thereof; and/or
(4) the first dose comprises about 6 mg of COMPOUND A, and the at least one daily maintenance dose comprises about 3 mg of COMPOUND A.

**11.** The compound or pharmaceutically acceptable salt for use according to any one of claims 1-10, wherein:

(1)

the first dose is effective to achieve a mean $C_{max}$ in the patient of about 40 to about 100 ng/mL; and
the at least one daily maintenance dose is effective to achieve a mean $C_{max}$ at steady-state in the patient of no greater than about 130 ng/mL, and a mean $AUC_T$ at steady-state in the patient of no greater than about 2600 ng·h/mL, after the at least one daily maintenance dose;

(2)

the first dose is effective to achieve a mean $C_{max}$ in the patient of about 75% to about 130% of about 42 ng/mL; and
the at least one daily maintenance dose is effective to achieve a mean $C_{max}$ at steady-state in the patient of about 75% to about 130% of 78 ng/mL, and a mean $AUC_T$ at steady-state in the patient of about 75% to about 130% of 1487 ng·h/mL, after the at least one daily maintenance dose;

or
(3)

the first dose is effective to achieve a mean $C_{max}$ in the patient of about 75% to about 130% of 42 ng/mL, and a mean AUC- in the patient of about 75% to about 130% of 2328 ng·h/mL; and
the at least one daily maintenance dose is effective to achieve a mean $C_{max}$ at steady-state in the patient of about 75% to about 130% of 78 ng/mL, and a mean $AUC_T$ at steady-state in the patient of about 75% to about 130% of 1487 ng·h/mL, after the at least one daily maintenance dose.

**12.** The compound or pharmaceutically acceptable salt for use according to any one of claims 1-11, wherein:

(1) the first dose comprises COMPOUND A; and/or
(2) the at least one daily maintenance dose comprises COMPOUND A.

**13.** The compound or pharmaceutically acceptable salt for use according to any one of claims 1-12, wherein:

the patient is 18 to 55 years old;
preferably wherein the patient is 20 to 49 years old.

**14.** The compound or pharmaceutically acceptable salt for use according to any one of claims 1-13, wherein:

(1) treating depression comprises improving symptoms of depression, as measured by the Montgomery Åsberg Depression Rating Scale (MADRS);

(2) treating depression comprises reducing MDD severity, as measured by the Clinical Global Impression-Severity Scale (CGI-S);
(3) treating depression comprises treating MDD, as measured by the Clinical Global Impression-Severity Scale (CGI-S); and/or
(4) treating depression comprises improving quality of life as measured by 5 Level EuroQol-5 Dimension.

**15.** The compound or pharmaceutically acceptable salt for use according to any one of claims 1-13, wherein: the treating depression is preventing relapse of depression.

**16.** The compound or pharmaceutically acceptable salt for use according to any one of claims 1-14, wherein:

the patient is on stable pharmacological treatment for depression;
optionally wherein the patient is on stable pharmacological treatment for depression, defined as ≤50% change in dose during the 6 weeks prior to randomization.

**Patentansprüche**

**1.** Verbindung, die 9-[4-(Cyclohexyloxy)phenyl]-7- methyl-3,4-dihydropyrazino[2,1-c]-[1,2,4]thiadiazin-2,2-dioxid ("VERBINDUNG A") ist, oder ein pharmazeutisch annehmbares Salz davon zur Verwendung in einem Verfahren zur Behandlung von Depression bei einem Patienten, wobei das Verfahren Folgendes umfasst:

das Verabreichen einer ersten Dosis, die VERBINDUNG A oder ein pharmazeutisch annehmbares Salz davon umfasst, an den Patienten; und
das Verabreichen zumindest einer täglichen Erhaltungsdosis, die VERBINDUNG A oder ein pharmazeutisch annehmbares Salz davon umfasst, an den Patienten, wobei die zumindest eine tägliche Erhaltungsdosis geringer ist als die erste Dosis.

**2.** Verfahren nach Anspruch 1, wobei:

(1) die Depression eine schwere depressive Episode ist;
(2) die Depression eine behandlungsresistente Depression ist; und/oder
(3) das Behandeln der Depression das Behandeln einer kognitiven Beeinträchtigung im Zusammenhang mit der Depression umfasst.

**3.** Verbindung oder pharmazeutisch annehmbares Salz zur Verwendung nach Anspruch 1 oder 2, wobei:

(1) der Patient nicht auf zumindest eine vorangegangene Therapie für Depression angesprochen hat; und/oder
(2) der Patient auf zumindest eine vorangegangene Therapie für Depression angesprochen hat.

**4.** Verbindung oder pharmazeutisch annehmbares Salz zur Verwendung nach Anspruch 3, wobei die zumindest eine vorangegangene Therapie für Depression eine Verabreichung vom Folgendem umfasst:

Ketamin oder einem Salz davon; (S)-Ketamin (Esketamin) oder einem Salz davon;
(R)-Ketamin oder einem Salz davon; Norketamin oder einem Salz davon; 2-diastereomerem Hydroxyketamin oder einem Salz davon; 6-diastereomerem Hydroxynorketamin (HNK) oder einem Salz davon; (2S,6S)-HNK oder einem Salz davon; (2R,6R)-HNK oder einem Salz davon; Dehydronorketamin oder einem Salz davon; Methoxyketamin oder einem Salz davon; oder Kombinationen davon;
wobei die zumindest eine vorangegangene Therapie vorzugsweise eine Verabreichung von Ketamin oder einem Salz davon; (S)-Ketamin (Esketamin) oder einem Salz davon; Methoxetamin oder einem Salz davon oder Kombinationen davon umfasst.

**5.** Verbindung oder pharmazeutisch annehmbares Salz zur Verwendung nach einem der Ansprüche 1 bis 4, wobei: die zumindest eine tägliche Erhaltungsdosis nicht weniger als etwa 12 h nach der ersten Dosis verabreicht wird.

**6.** Verbindung oder pharmazeutisch annehmbares Salz zur Verwendung nach einem der Ansprüche 1 bis 5, wobei:

(1) die erste Dosis zwischen etwa 5 mg und etwa 9 mg der VERBINDUNG A oder eines pharmazeutisch

annehmbaren Salzes davon umfasst;
(2) die erste Dosis wirksam ist, um in dem Patienten eine mittlere $C_{max}$ von zumindest etwa 6 ng/ml zu erzielen;
(3) die erste Dosis wirksam ist, um in dem Patienten eine mittlere $C_{max}$ von nicht mehr als etwa 100 ng/ml zu erzielen;
(4) die erste Dosis wirksam ist, um in dem Patienten eine mittlere $C_{max}$ von etwa 6 ng/ml bis etwa 100 ng/ml zu erzielen; und/oder
(5) die erste Dosis wirksam ist, um in dem Patienten eine mittlere $C_{max}$ von etwa 75 % bis etwa 130 % von 42 ng/ml zu erzielen.

**7.** Verbindung oder pharmazeutisch annehmbares Salz davon zur Verwendung nach einem der Ansprüche 1 bis 6, wobei:

(1) die zumindest eine tägliche Erhaltungsdosis zwischen etwa 0,5 mg und etwa 4 mg einmal täglich der VERBINDUNG A oder eines pharmazeutisch annehmbaren Salzes davon umfasst;
(2) die zumindest eine tägliche Erhaltungsdosis wirksam ist, um in dem Patienten in einem Steady State eine mittlere $C_{max}$ von nicht mehr als etwa 130 ng/ml zu erzielen; und/oder
(3) die zumindest eine tägliche Erhaltungsdosis wirksam ist, um in dem Patienten in einem Steady State eine mittlere $C_{max}$ von etwa 75 % bis etwa 130 % von 78 ng/ml zu erzielen.

**8.** Verbindung oder pharmazeutisch annehmbares Salz zur Verwendung nach einem der Ansprüche 1 bis 7, wobei:

(1) in dem Patienten im Steady State die mittlere $AUC_T$ nicht größer als etwa 2.600 ng·h/ml ist;
(2) in dem Patienten im Steady State die mittlere $AUC_T$ etwa 700 ng·h/ml bis etwa 2.600 ng·h/ml beträgt;
(3) in dem Patienten im Steady State die mittlere $AUC_T$ etwa 75 % bis etwa 130 % von 1.487 ng·h/ml beträgt; und/oder
(4) in dem Patienten die mittlere $AUC_\infty$ etwa 75 % bis etwa 130 % von 2.328 ng·h/ml beträgt.

**9.** Verbindung oder pharmazeutisch annehmbares Salz zur Verwendung nach einem der Ansprüche 1 bis 8, wobei:

(1) die erste Dosis oral verabreicht wird; und/oder
(2) die zumindest eine tägliche Erhaltungsdosis oral verabreicht wird.

**10.** Verbindung oder pharmazeutisch annehmbares Salz zur Verwendung nach einem der Ansprüche 1 bis 9, wobei:

(1) die erste Dosis etwa 6 mg der VERBINDUNG A oder eines pharmazeutisch annehmbaren Salzes davon umfasst;
(2) die Erhaltungsdosis etwa 3 mg einmal täglich der VERBINDUNG A oder eines pharmazeutisch annehmbaren Salzes davon umfasst;
(3) die erste Dosis etwa 6 mg der VERBINDUNG A oder eines pharmazeutisch annehmbaren Salzes davon umfasst und die zumindest eine tägliche Erhaltungsdosis etwa 3 mg der VERBINDUNG A oder eines pharmazeutisch annehmbaren Salzes davon umfasst; und/oder
(4) die erste Dosis etwa 6 mg der VERBINDUNG A umfasst und die zumindest eine tägliche Erhaltungsdosis etwa 3 mg der VERBINDUNG A umfasst.

**11.** Verbindung oder pharmazeutisch annehmbares Salz zur Verwendung nach einem der Ansprüche 1 bis 10, wobei:

(1) die erste Dosis wirksam ist, um in dem Patienten eine mittlere $C_{max}$ von etwa 40 bis etwa 100 ng/ml zu erzielen; und
die zumindest eine tägliche Erhaltungsdosis wirksam ist, um in dem Patienten im Steady State eine mittlere $C_{max}$ von nicht mehr als etwa 130 ng/ml und in dem Patienten im Steady State eine mittlere $AUC_T$ von nicht mehr als etwa 2.600 ng·h/ml nach der zumindest einen täglichen Erhaltungsdosis zu erzielen;
(2) die erste Dosis wirksam ist, um in dem Patienten eine mittlere $C_{max}$ von etwa 75 % bis etwa 130 % von etwa 42 ng/ml zu erzielen; und
die zumindest eine tägliche Erhaltungsdosis wirksam ist, um in dem Patienten im Steady State eine mittlere $C_{max}$ von etwa 75 % bis etwa 130 % von 78 ng/ml und in dem Patienten im Steady State eine mittlere $AUC_T$ von etwa 75 % bis etwa 130 % von 1.487 ng·h/ml nach der zumindest einen täglichen Erhaltungsdosis zu erzielen; oder
(3) die erste Dosis wirksam ist, um in dem Patienten eine mittlere $C_{max}$ von etwa 75 % bis etwa 130 % von 42 ng/ml und in dem Patienten eine mittlere $AUC_\infty$ von etwa 75 % bis etwa 130 % von 2.328 ng·h/ml zu erzielen; und

die zumindest eine tägliche Erhaltungsdosis wirksam ist, um in dem Patienten im Steady State eine mittlere $C_{max}$ von etwa 75 % bis etwa 130 % von 78 ng/ml und in dem Patienten im Steady State eine mittlere $AUC_T$ von etwa 75 % bis etwa 130 % von 1.487 ng·h/ml nach der zumindest einen täglichen Erhaltungsdosis zu erzielen.

**12.** Verbindung oder pharmazeutisch annehmbares Salz zur Verwendung nach einem der Ansprüche 1 bis 11, wobei:

(1) die erste Dosis VERBINDUNG A umfasst; und/oder
(2) die zumindest eine tägliche Erhaltungsdosis VERBINDUNG A umfasst.

**13.** Verbindung oder pharmazeutisch annehmbares Salz zur Verwendung nach einem der Ansprüche 1 bis 12, wobei:

der Patient 18 bis 55 Jahre alt ist;
wobei der Patient vorzugsweise 20 bis 49 Jahre alt ist.

**14.** Verbindung oder pharmazeutisch annehmbares Salz zur Verwendung nach einem der Ansprüche 1 bis 13, wobei:

(1) das Behandeln der Depression das Verbessern der Symptome der Depression, gemessen anhand der Montgomery-Åsberg-Depressionsbewertungsskala (MADRS), umfasst;
(2) das Behandeln der Depression das Reduzieren des MDD-Schweregrads, gemessen anhand der Clinical Global Impression-Schweregradskala (CGI-S), umfasst;
(3) das Behandeln der Depression das Behandeln von MDD, gemessen anhand der Clinical Global Impression-Schweregradskala (CGI-S), umfasst; und/oder
(4) das Behandeln der Depression das Verbessern der Lebensqualität, gemessen anhand der 5-stufigen EuroQol-5-Dimension, umfasst.

**15.** Verbindung oder pharmazeutisch annehmbares Salz davon zur Verwendung nach einem der Ansprüche 1 bis 13, wobei:
das Behandeln der Depression einen Rückfall der Depression verhindert.

**16.** Verbindung oder pharmazeutisch annehmbares Salz davon zur Verwendung nach einem der Ansprüche 1 bis 14, wobei:

der Patient eine stabile pharmakologische Behandlung für Depression erhält;
wobei der Patient gegebenenfalls eine stabile pharmakologische Behandlung für Depression erhält, die als eine Veränderung von ≤ 50 % der Dosis während 6 Wochen vor der Randomisierung definiert ist.

## Revendications

**1.** Composé qui est le 2,2-dioxyde de 9-[4-(cyclohexyloxy)phényl]-7-méthyl-3,4-dihydropyrazino[2,1-c][1,2,4]thiadiazine (« COMPOSÉ A »), ou un sel pharmaceutiquement acceptable de celui-ci, pour utilisation dans un procédé de traitement de la dépression chez un patient, le procédé comprenant les étapes consistant à :

administrer au patient une première dose comprenant le COMPOSÉ A ou un sel pharmaceutiquement acceptable de celui-ci ; et
administrer au patient au moins une dose d'entretien quotidienne comprenant le COMPOSÉ A ou un sel pharmaceutiquement acceptable de celui-ci, dans lequel la au moins une dose d'entretien quotidienne est inférieure à la première dose.

**2.** Procédé selon la revendication 1, dans lequel :

(1) la dépression est un trouble dépressif majeur ;
(2) la dépression est une dépression résistante au traitement ; et/ou
(3) le traitement de la dépression comprend un traitement d'une déficience cognitive associée à la dépression.

**3.** Composé ou sel pharmaceutiquement acceptable pour utilisation selon la revendication 1 ou 2, dans lequel :

(1) le patient n'a pas répondu à au moins une thérapie antérieure pour la dépression ; et/ou

(2) le patient répond à au moins une thérapie antérieure pour la dépression.

**4.** Composé ou sel pharmaceutiquement acceptable pour utilisation selon la revendication 3, dans lequel la au moins une thérapie antérieure pour la dépression comprend une administration :

de kétamine ou d'un sel de celle-ci ; de (S)-kétamine (eskétamine) ou d'un sel de celle-ci ; de (R)-kétamine ou d'un sel de celle-ci ; de norkétamine ou d'un sel de celle-ci ; de 2 hydroxykétamine diastéréomérique ou d'un sel de celle-ci ; de 6 hydroxynorkétamine diastéréomérique (HNK) ou d'un sel de celle-ci ; de (2S,6S)-HNK ou d'un sel de celle-ci ; de (2R,6R)-HNK ou d'un sel de celle-ci ; de déshydronorkétamine ou d'un sel de celle-ci ; de méthoxétamine ou d'un sel de celle-ci ; ou de combinaisons de ceux-ci ;
de préférence dans lequel la au moins une thérapie antérieure comprend une administration de kétamine ou d'un sel de celle-ci ; de (S)-kétamine (eskétamine) ou d'un sel de celle-ci ; de méthoxétamine ou d'un sel de celle-ci ; ou de combinaisons de ceux-ci.

**5.** Composé ou sel pharmaceutiquement acceptable pour utilisation selon l'une quelconque des revendications 1 à 4, dans lequel :
la au moins une dose d'entretien quotidienne est administrée au moins environ 12 heures après la première dose.

**6.** Composé ou sel pharmaceutiquement acceptable pour utilisation selon l'une quelconque des revendications 1 à 5, dans lequel :

(1) la première dose comprend entre environ 5 mg et environ 9 mg de COMPOSÉ A ou d'un sel pharmaceutiquement acceptable de celui-ci ;
(2) la première dose est efficace pour atteindre une $C_{max}$ moyenne chez le patient d'au moins environ 6 ng/ml ;
(3) la première dose est efficace pour atteindre une $C_{max}$ moyenne chez le patient ne dépassant pas environ 100 ng/ml ;
(4) la première dose est efficace pour atteindre une $C_{max}$ moyenne chez le patient d'environ 6 ng/ml à environ 100 ng/ml ; et/ou
(5) la première dose est efficace pour atteindre une $C_{max}$ moyenne chez le patient d'environ 75 % à environ 130 % de 42 ng/mL.

**7.** Composé ou sel pharmaceutiquement acceptable pour utilisation selon l'une quelconque des revendications 1 à 6, dans lequel :

(1) la au moins une dose d'entretien quotidienne comprend entre environ 0,5 mg et environ 4 mg une fois par jour de COMPOSÉ A ou d'un sel pharmaceutiquement acceptable de celui-ci ;
(2) la au moins une dose d'entretien quotidienne est efficace pour atteindre une $C_{max}$ moyenne à l'état d'équilibre chez le patient ne dépassant pas environ 130 ng/ml ; et/ou
(3) la au moins une dose d'entretien quotidienne est efficace pour atteindre une $C_{max}$ moyenne à l'état d'équilibre chez le patient d'environ 75 % à environ 130 % de 78 ng/ml.

**8.** Composé ou sel pharmaceutiquement acceptable pour utilisation selon l'une quelconque des revendications 1 à 7, dans lequel :

(1) l'$AUC_T$ moyenne à l'état d'équilibre chez le patient n'est pas supérieure à environ 2 600 ng·h/mL ;
(2) l'$AUC_T$ moyenne à l'état d'équilibre chez le patient est d'environ 700 ng·h/mL à environ 2 600 ng·h/mL ;
(3) l'$AUC_T$ moyenne à l'état d'équilibre chez le patient est d'environ 75 % à environ 130 % de 1 487 ng-h/mL ; et/ou
(4) l'$AUC_\infty$ moyenne chez le patient est d'environ 75 % à environ 130 % de 2 328 ng·h/mL.

**9.** Composé ou sel pharmaceutiquement acceptable pour utilisation selon l'une quelconque des revendications 1 à 8, dans lequel :

(1) la première dose est administrée par voie orale ; et/ou
(2) la au moins une dose d'entretien quotidienne est administrée par voie orale.

**10.** Composé ou sel pharmaceutiquement acceptable pour utilisation selon l'une quelconque des revendications 1 à 9, dans lequel :

(1) la première dose comprend environ 6 mg de COMPOSÉ A ou d'un sel pharmaceutiquement acceptable de celui-ci ;
(2) la dose d'entretien comprend environ 3 mg une fois par jour de COMPOSÉ A ou d'un sel pharmaceutiquement acceptable de celui-ci ;
(3) la première dose comprend environ 6 mg de COMPOSÉ A ou d'un sel pharmaceutiquement acceptable de celui-ci, et la au moins une dose d'entretien quotidienne comprend environ 3 mg de COMPOSÉ A ou d'un sel pharmaceutiquement acceptable de celui-ci ; et/ou
(4) la première dose comprend environ 6 mg de COMPOSÉ A, et la au moins une dose d'entretien quotidienne comprend environ 3 mg de COMPOSÉ A.

**11.** Composé ou sel pharmaceutiquement acceptable pour utilisation selon l'une quelconque des revendications 1 à 10, dans lequel :

(1)

la première dose est efficace pour atteindre une $C_{max}$ moyenne chez le patient d'environ 40 à environ 100 ng/ml ; et
la au moins une dose d'entretien quotidienne est efficace pour atteindre une $C_{max}$ moyenne à l'état d'équilibre chez le patient ne dépassant pas environ 130 ng/ml, et une $AUC_T$ moyenne à l'état d'équilibre chez le patient ne dépassant pas environ 2 600 ng·h/ml, après la au moins une dose d'entretien quotidienne ;

(2)

la première dose est efficace pour atteindre une $C_{max}$ moyenne chez le patient d'environ 75 % à environ 130 % d'environ 42 ng/ml ; et
la au moins une dose d'entretien quotidienne est efficace pour atteindre une $C_{max}$ moyenne à l'état d'équilibre chez le patient d'environ 75 % à environ 130 % de 78 ng/ml, et une $AUC_T$ moyenne à l'état d'équilibre chez le patient d'environ 75 % à environ 130 % de 1 487 ng-h/ml, après la au moins une dose d'entretien quotidienne ;

ou
(3)

la première dose est efficace pour atteindre une $C_{max}$ moyenne chez le patient d'environ 75 % à environ 130 % de 42 ng/ml, et une $AUC_{\infty}$ moyenne chez le patient d'environ 75 % à environ 130 % de 2 328 ng·h/ml ; et
la au moins une dose d'entretien quotidienne est efficace pour atteindre une $C_{max}$ moyenne à l'état d'équilibre chez le patient d'environ 75 % à environ 130 % de 78 ng/ml, et une $AUC_T$ moyenne à l'état d'équilibre chez le patient d'environ 75 % à environ 130 % de 1 487 ng·h/ml, après la au moins une dose d'entretien quotidienne.

**12.** Composé ou sel pharmaceutiquement acceptable pour utilisation selon l'une quelconque des revendications 1 à 11, dans lequel :

(1) la première dose comprend le COMPOSÉ A ; et/ou
(2) la au moins une dose d'entretien quotidienne comprend le COMPOSÉ A.

**13.** Composé ou sel pharmaceutiquement acceptable pour utilisation selon l'une quelconque des revendications 1 à 12, dans lequel :

le patient est âgé de 18 à 55 ans ;
de préférence dans lequel le patient est âgé de 20 à 49 ans.

**14.** Composé ou sel pharmaceutiquement acceptable pour utilisation selon l'une quelconque des revendications 1 à 13, dans lequel :

(1) le traitement de la dépression comprend l'amélioration des symptômes de la dépression, tels que mesurés par l'échelle d'évaluation de la dépression de Montgomery Åsberg (MADRS) ;
(2) le traitement de la dépression comprend la réduction de la gravité du TDM, telle que mesurée par l'échelle de

gravité d'impression clinique globale (CGI-S) ;
(3) le traitement de la dépression comprend le traitement du TDM, tel que mesuré par l'échelle de gravité d'impression clinique globale (CGI-S) ; et/ou
(4) le traitement de la dépression comprend l'amélioration de la qualité de vie telle que mesurée par la dimension EuroQol-5 à 5 niveaux.

**15.** Composé ou sel pharmaceutiquement acceptable pour utilisation selon l'une quelconque des revendications 1 à 13, dans lequel :
le traitement de la dépression prévient la rechute de la dépression.

**16.** Composé ou sel pharmaceutiquement acceptable pour utilisation selon l'une quelconque des revendications 1 à 14, dans lequel :

le patient est sous traitement pharmacologique stable pour la dépression ;
facultativement dans lequel le patient est sous traitement pharmacologique stable pour la dépression, défini comme un changement de dose ≤ 50 % au cours des 6 semaines précédant la randomisation.

**Study Schematic: Part 1 SRD Cohorts 1 to 6**

| Pretreatment Period | | Treatment Period | | | | Follow-up [d] |
|---|---|---|---|---|---|---|
| Screening | Check-in Baseline Assessments [a] | COMPOUND A Single Dose and Study Assessments [a,b,c] | Safety and PK Assessments [a,c] | PK Assessments | PK Assessments/ Study Exit [a] | |
| Days -28 to -2 | Day -1 | Day 1 | Days 2-5 | Day 6 (Cohorts 1-5) Days 6-7 (Cohort 6) | Day 7 (Cohorts 1-5) Day 8 (Cohort 6) | Day 14 (±2) |
| | ◄------ Confinement ------► | | | | | |

Abbreviations: PK, pharmacokinetic; SRD, single-rising dose.

[a] EEG assessments were performed on Days -1, 1, 2, and 5, at Study Exit, and early termination visits.

[b] Including PK assessments.

[c] For SRD Cohort 6 (18 mg), blood samples for measurement of BDNF concentrations were collected on Days 1 to 5.

[d] Follow-up occurred by telephone unless abnormal, clinically significant findings were observed upon discharge. In this case or at investigator's discretion, subjects had to return to the clinic for re-evaluation.

*FIG. 1A*

**Study Schematic: Part 2 SRD/MRD Cohorts 1 to 5**

| Pretreatment Period | | Treatment Period | | | | | Follow-up [e] |
|---|---|---|---|---|---|---|---|
| Screening | Check-in Baseline Assessments | COMPOUND A Single Dose and Study Assessments[a,b,d] | Safety and PK Assessments [c] | QD Dosing and Study Assessments [a,b,c,d] | COMPOUND A Safety and PK Assessments | Study Exit | |
| Days -28 to -2 | Day -1 | Day 1 | Days 2-5 | Days 6-18 | Days 19-20 | Day 21 | Day 31 (±2) |
| | ←------ Confinement ------→ | | | | | | |

Abbreviations: MRD, multiple-rising dose; PK, pharmacokinetic; SRD, single-rising dose.

[a] EEG assessments were performed on Days -1, 11, 18, and at early termination visits.

[b] Including PK assessments.

[c] For SRD/MRD Cohort 3 only, a single CSF sample was collected from each subject on Day 12.

[d] For SRD/MRD Cohorts 3 to 5, blood samples for BDNF were collected on Days 1 to 5, 18, and 19.

[e] Follow-up occurred by telephone unless abnormal clinically significant findings were observed upon discharge. In this case or per investigator's discretion, subjects had to return to the clinic for re-evaluation.

*FIG. 1B*

Plasma Concentration (ng/mL)
150
120
90
60
30
0
0
24
48
72
96
120
144
168
Time (hr)
Plasma Concentration (ng/mL)
1000
100
10
1
0.1
0.01
0
24
48
72
96
120
144
168
Time (hr)
COMPOUND A 0.3 mg ONCE
COMPOUND A 5 mg ONCE
COMPOUND A 1 mg ONCE
COMPOUND A 9 mg ONCE
COMPOUND A 3 mg ONCE fasted
COMPOUND A 18 mg ONCE

*FIG. 2*

400
300
200
100
0
Plasma Concentration (ng/mL)
0
6
12
18
24
Time (hr)
1000
100
10
1
Plasma Concentration (ng/mL)
0
6
12
18
24
Time (hr)
COMPOUND A 0.3 mg QD
COMPOUND A 1 mg QD
COMPOUND A 6 mg QD
COMPOUND A 9 mg QD
COMPOUND A 3 mg QD

*FIG. 3*

DOSE, WT
WT
WT
DOSE
K_A
T_Lag
V/F
CL/F

Dose administration in the gut was modeled using a depot compartment. KA: absorption rate constant ($h^{-1}$); $T_{Lag}$: absorption lag (h); CL/F: apparent oral clearance (L/h); V/F: apparent volume of distribution of the central compartment (L); WT: centered weight (kg).

*FIG. 4*

COMPOUND A
Plasma Concentration (mg/mL)
$10^2$
$10^1$
0
1
2
3
4
5
6
7
8
9
10
Time (days)
S1: 2.5mg QD No Load
S2: Load 6mg Maint 2.5mg QD
S3: Load 6mg Maint 3mg QD
Solid lines: Predicted median concentration; dashed lines: [5%-95%] prediction intervals

*FIG. 5*

Screening
Days -28 to -1
Treatment Periods
Ketamine (open-label)
Washout Periods
10-15 days
Treatment Day Detail (Placebo or COMPOUND A, PO)
-1 h
0
1 h
2 h
3 h
4 h
5 h
TMS
COMPOUND
A or
placebo
TMS
PK
TMS
PK
Discharge
Day 1 detail (Ketamine IV)
-1 h
0
1 h
2 h
3 h
4 h
Day 2
24 h
TMS
TMS
TMS
Discharge
IV infusion
Ketamine
over 40 min


IV: intravenous; PK: pharmacokinetics; PO: orally; TMS: transcranial magnetic stimulation.

*FIG. 6*

Change from baseline (uV)
500
400
300
200
100
0
-100
-200
-300
-400
0.0
0.5
1.0
1.5
2.0
2.5
Time (hour)
P=0.1053†
P=0.8475†
P=0.0497†
P=0.6418†
Treatment
Placebo
COMPOUND A 0.5 mg
COMPOUND A 6.0 mg

† *P* values of the test on the difference in least-squares means using Dunnett's multiple comparison method.

***FIG. 7***

Screening
≤27 days
Day -1
Admit to Unit
Randomization (Day 1)
Days 1 to 14 (study treatment dosing)
Day 15
Discharge from Unit
Day 25
Final Visit

FIG. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 8575154 B


### Non-patent literature cited in the description


- **SINGLETON et al.** *Dictionary of Microbiology and Molecular Biology*, 1994
- The Cambridge Dictionary of Science and Technology. 1988
- The Glossary of Genetics. Springer Verlag, 1991
- **HALE** ; **MARHAM**. The Harper Collins Dictionary of Biology. 1991
- **BOLON B** ; **GARMAN RH** ; **PARDO ID** ; **JENSEN K** ; **SILLS RC** ; **ROULOIS A et al.** STP position paper: Recommended practices for sampling and processing the nervous system (brain, spinal cord, nerve, and eye) during nonclinical general toxicity studies.. *Toxicol Pathol.*, 2013, vol. 41, 1028-48
- **SCHMUED LC** ; **ALBERTSON C** ; **SLIKKER W JR.** Fluoro-Jade: a novel fluorochrome for the sensitive and reliable histochemical localization of neuronal degeneration.. *Brain Res.*, 1997, vol. 751, 37-46
- **DZUBAY JA** ; **JAHR CE.** The concentration of synaptically released glutamate outside of the climbing fiber-Purkinje cell synaptic cleft.. *J. Neurosci.*, 1999, vol. 19, 5265-74
- **MANSOURI B** ; **HENNE WM** ; **OMMAN SK** ; **BLISS R** ; **ATTRIDGE J** ; **FINCKBONE V et al.** Involvement of calpain in AMPA-induced toxicity to rat cerebellar Purkinje neurons.. *Eur. J. Pharmacol.*, 2007, vol. 557, 106-14
- **BARENBERG P** ; **STRAHLENDORF H** ; **STRAHLENDORF J.** Hypoxia induces an excitotoxic-type of dark cell degeneration in cerebellar Purkinje neurons.. *Neurosci. Res.*, 2001, vol. 40, 245-54
- **GARTHWAITE G** ; **GARTHWAITE J.** AMPA neurotoxicity in rat cerebellar and hippocampal slices: histological evidence for three mechanisms.. *Eur. J. Neurosci.*, 1991, vol. 3, 715-28
- **HEITZ CR** ; **BENCE JR.** Ketamine-induced catalepsy during adult sedation in the Emergency Department.. *J. Emerg. Med.*, February 2013, vol. 44 (2), e243-5